# EUROPEAN PATENT SPECIFICATION

(11) **EP 4 117 660 B1**
(45) Date of publication and mention of the grant of the patent: **05.11.2025**
(21) Application number: 21768880.3
(22) Date of filing: 11.03.2021
(51) Int. Cl.: A61K 31/69, A61K 31/4439, A61K 31/519, A61P 35/00, A61P 9/04, A61P 25/28, A61P 37/06

(54) **METHODS OF MODULATING T-CELL ACTIVATION AND TREATING CHRONIC HEART FAILURE USING CARBORANES**
VERFAHREN ZUR MODULIERUNG DER T-ZELL-AKTIVIERUNG UND ZUR BEHANDLUNG VON CHRONISCHER HERZINSUFFIZIENZ MIT CARBORANEN
PROCÉDÉS DE MODULATION DE L'ACTIVATION DE LYMPHOCYTES T ET DE TRAITEMENT DE L'INSUFFISANCE CARDIAQUE CHRONIQUE À L'AIDE DE CARBORANES

(30) Priority: 11.03.2020 US 202062988239 P
(43) Date of publication of application: 18.01.2023
(73) Proprietor: Ohio State Innovation Foundation, Columbus, OH 43201 (US)
(72) Inventor: BANSAL, Shyam S., Columbus, Ohio 43201 (US); GUMINA, Richard Joseph, Columbus, Ohio 43201 (US)
(74) Representative: Berggren Oy
(86) International application number: PCT/US2021/021909
(87) International publication number: WO 2021/183764

(56) References cited:
- EP-A1- 1 145 718
- WO-A1-2017/049307
- WO-A1-2019/191301
- WO-A1-2020/117799
- WO-A2-00/40232
- HIRATA M ET AL: "A novel carborane analog, BE360, with a carbon-containing polyhedral boron-cluster is a new selective estrogen receptor modulator for bone", BIOCHEMICAL AND BIOPHYSICAL RESEARCH COMMUNICATIONS, ELSEVIER, AMSTERDAM NL, vol. 380, no. 2, 6 March 2009 (2009-03-06), pages 218 - 222, XP025958597, ISSN: 0006-291X, [retrieved on 20090119], DOI: 10.1016/J.BBRC.2009.01.033
- OHTA KIMINORI ET AL: "ER subtype selectivity of m-carborane-containing phenols: C-alkyl groups on the m-carborane cage enhance ER[alpha] selectivity", BIOORGANIC & MEDICINAL CHEMISTRY LETTERS, ELSEVIER, AMSTERDAM NL, vol. 29, no. 16, 19 June 2019 (2019-06-19), pages 2290 - 2293, XP085759053, ISSN: 0960-894X, [retrieved on 20190619], DOI: 10.1016/J.BMCL.2019.06.025
- BERNARDO BIANCA C., OOI JENNY Y.Y., MCMULLEN JULIE R.: "The yin and yang of adaptive and maladaptive processes in heart failure", DRUG DISCOVERY TODAY: THERAPEUTIC STRATEGIES, ELSEVIER, AMSTERDAM, NL, vol. 9, no. 4, 1 January 2012 (2012-01-01), AMSTERDAM, NL , pages e163 - e172, XP055857482, ISSN: 1740-6773, DOI: 10.1016/j.ddstr.2013.10.001

## Description

### CROSS-REFERENCE TO RELATED APPLICATIONS

This application claims the benefit of priority to U.S. Provisional Application No. 62/988,239 filed March 11, 2020.

### STATEMENT OF GOVERNMENT SUPPORT

This invention was made with government support under R00 HL132123 awarded by the National Institutes of Health. The government has certain rights in the invention.

### BACKGROUND

Activation of innate and adaptive immune cells underlies inflammatory responses in many chronic diseases. Monocytes, macrophages, and dendritic cells (DCs) mediate innate immune responses, whereas CD3⁺CD4⁺ helper and CD3⁺CD8⁺ cytotoxic T-cells arbitrate adaptive immunity. While innate immune cells comprise the first line of defense against acute injury, chronic inflammation often implies activation and clonal expansion of specialized effector T-cells following antigen presentation. In chronic heart failure, the importance of activated monocytes, macrophages, DCs and T-cells has been increasingly recognized. However, the molecular mechanisms that are involved in pathological immune cell activation, and the specific roles of such alterations in the progression of adverse remodeling and inflammation in chronic heart failure, are unknown.

### SUMMARY

Note that the references to the methods of treatment by therapy in this description are to be interpreted as references to compounds, pharmaceutical compositions and medicaments of the present invention for use in those methods.

Disclosed herein are methods of modulating immune response in a subject using carboranes. The carborane can selectively inhibit the activation and/or proliferation of T-cells, reducing circulating T-cell levels in a subject without significantly affecting circulating levels of neutrophils, monocytes, or B-cells. As a result, the carboranes can be used in therapeutic and/or prophylactic applications, to treat or prevent chronic heart failure (CHF) in a subject post-myocardial infarction (MI).

The details of one or more embodiments of the invention are set forth in the accompanying drawings and the description below. Other features, objects, and advantages of the invention will be apparent from the description and drawings, and from the claims.

### DESCRIPTION OF DRAWINGS

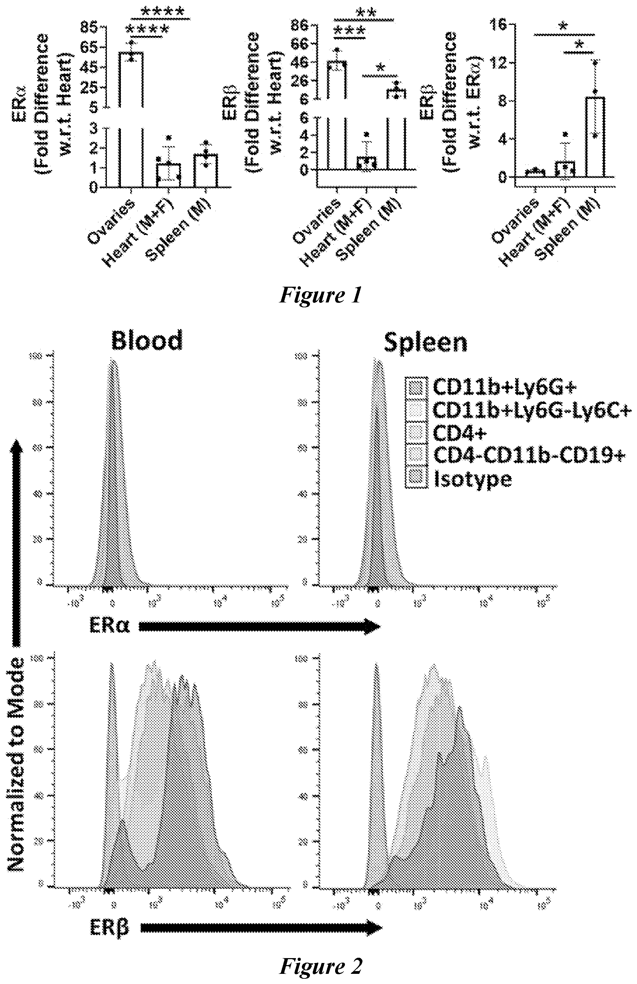

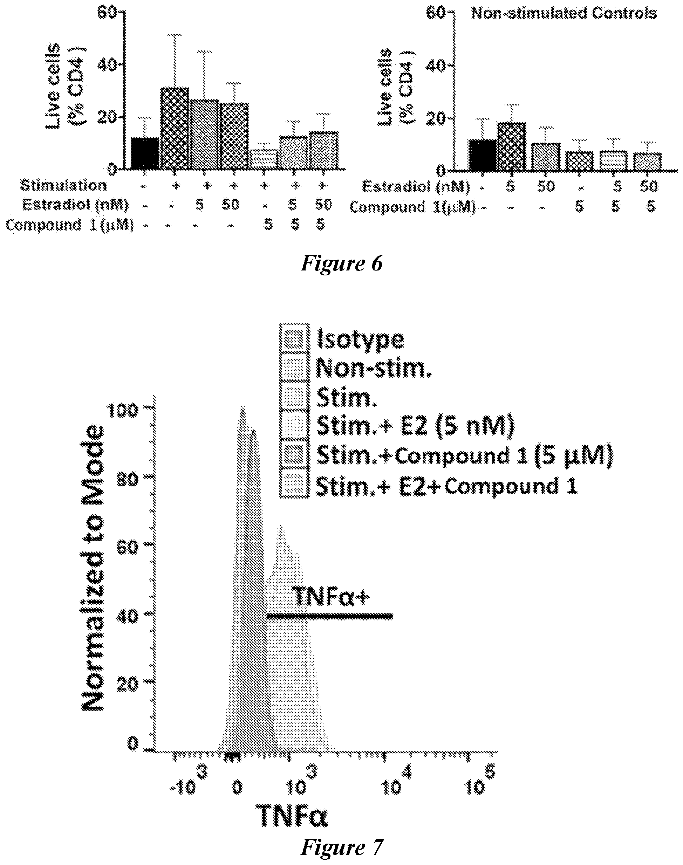

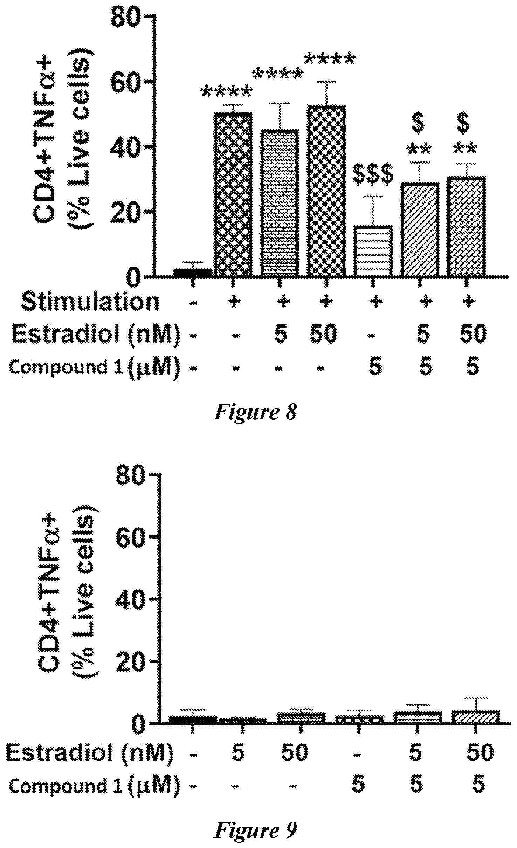

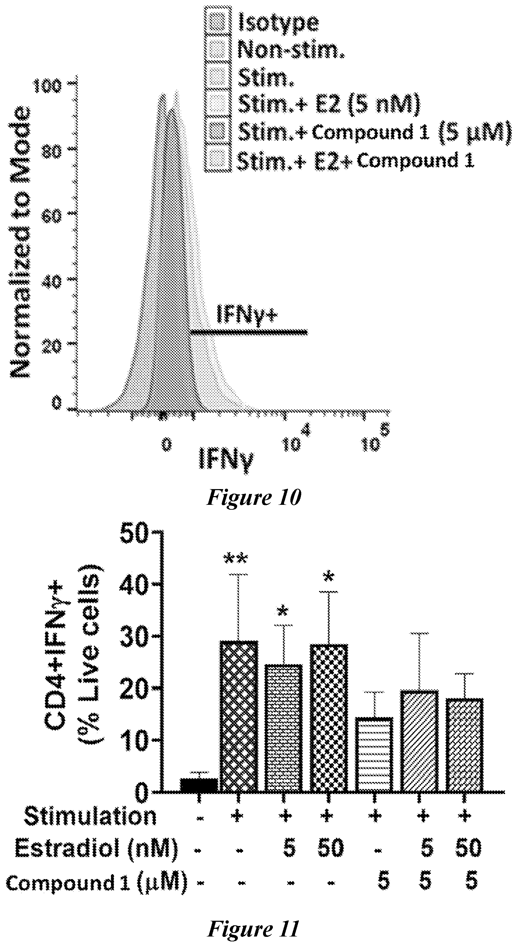

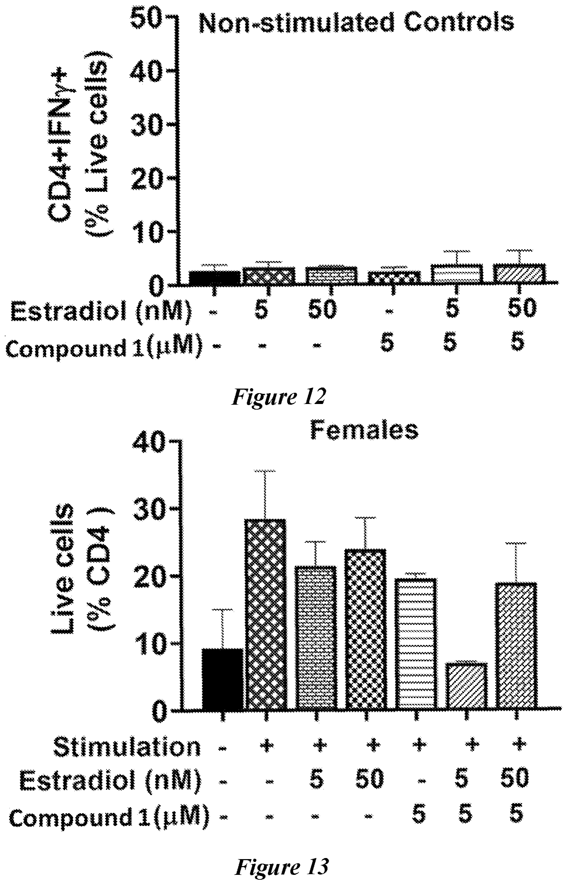

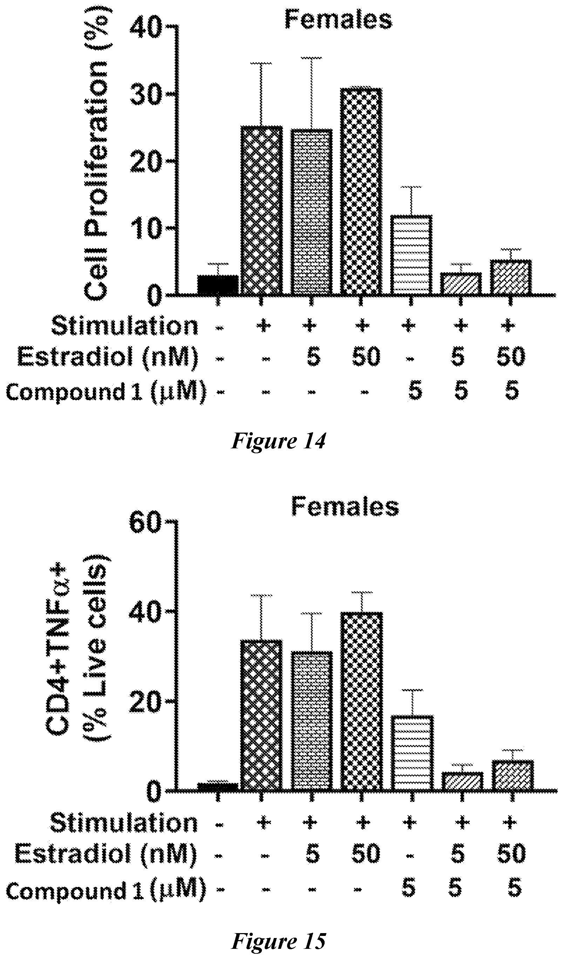

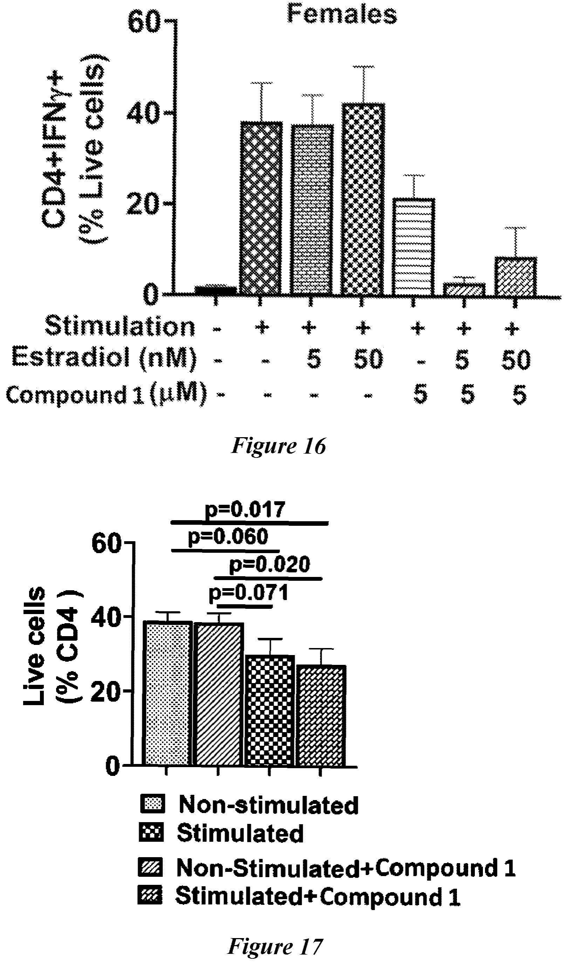

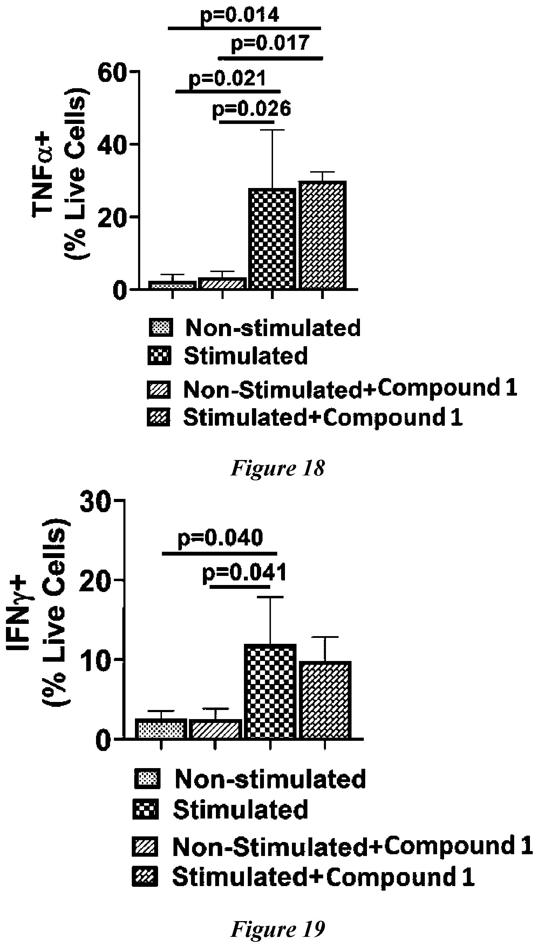

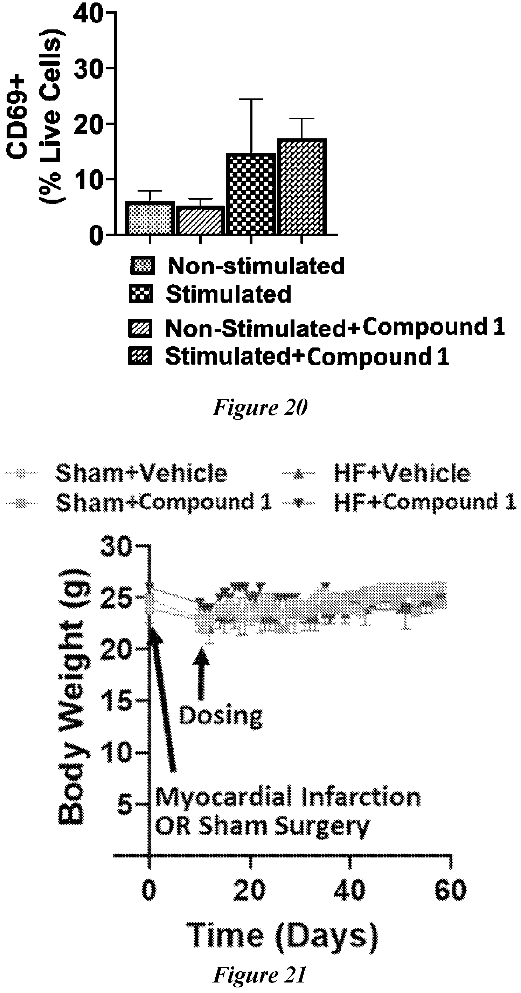

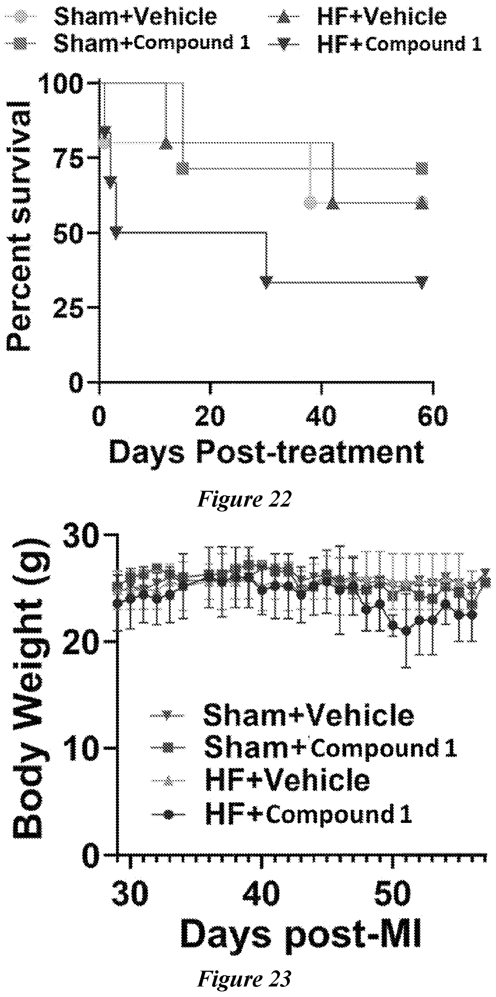

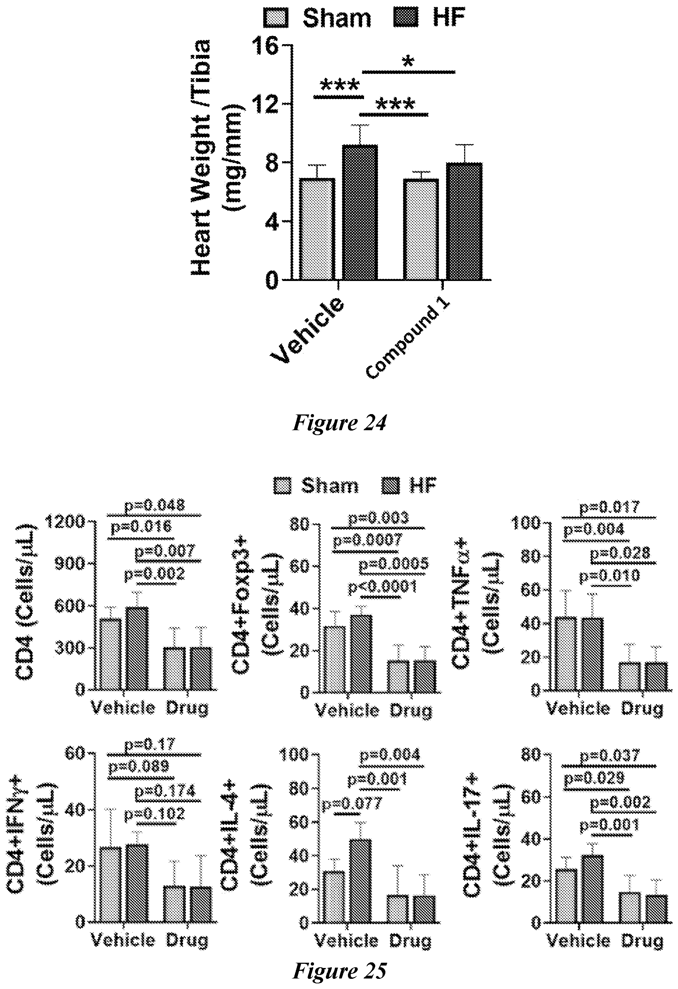

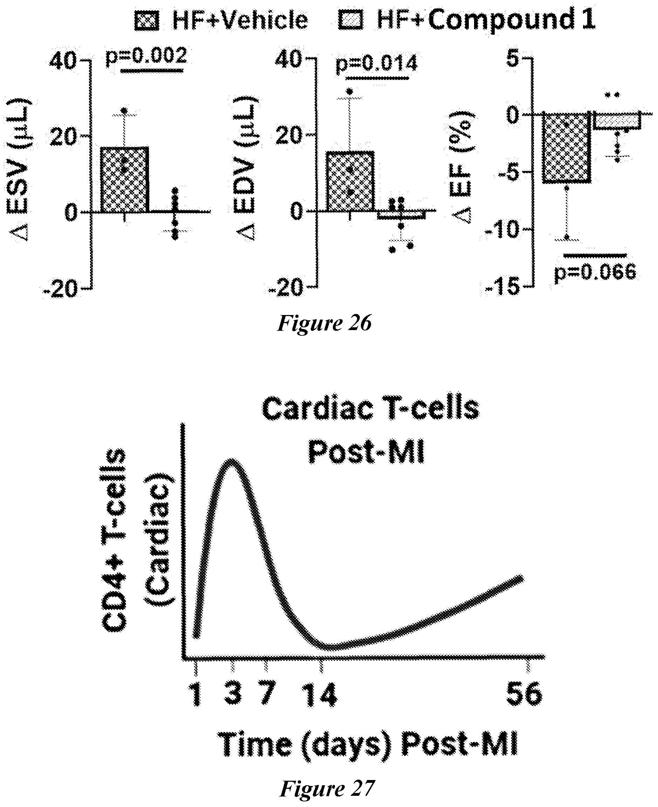

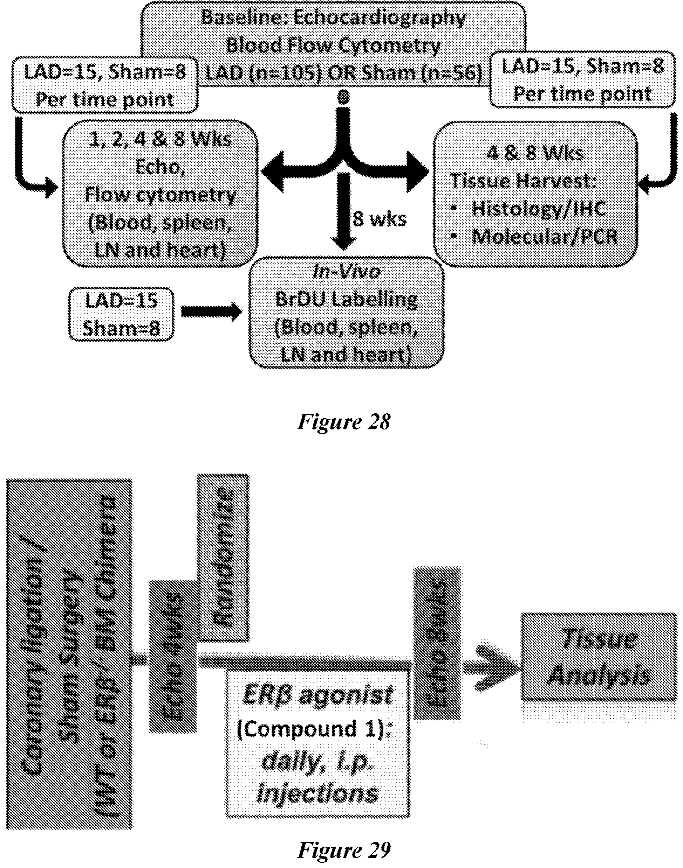

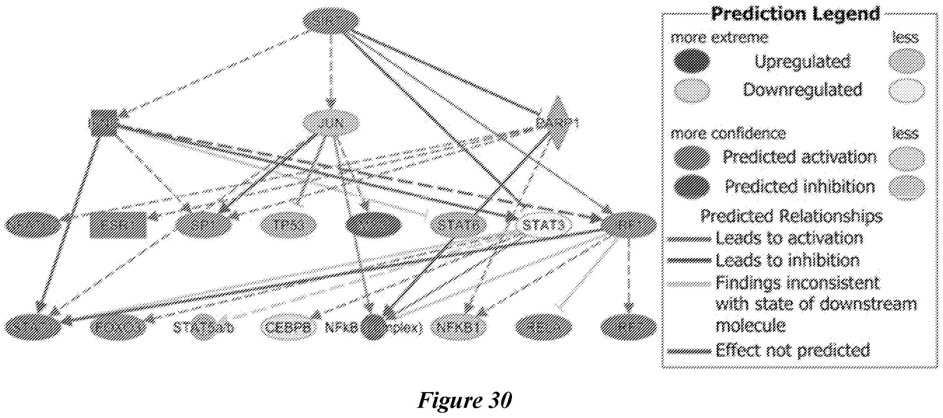

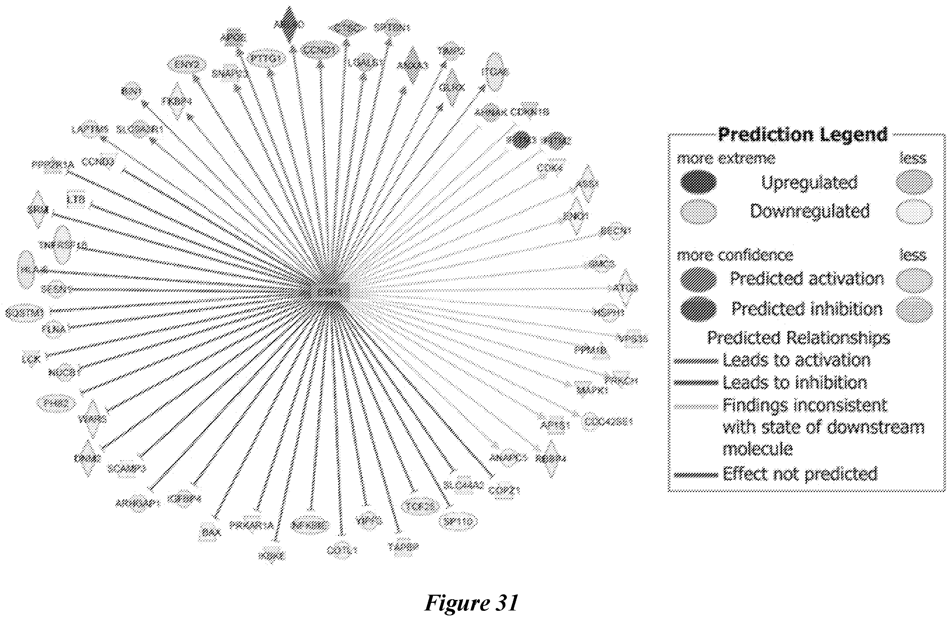

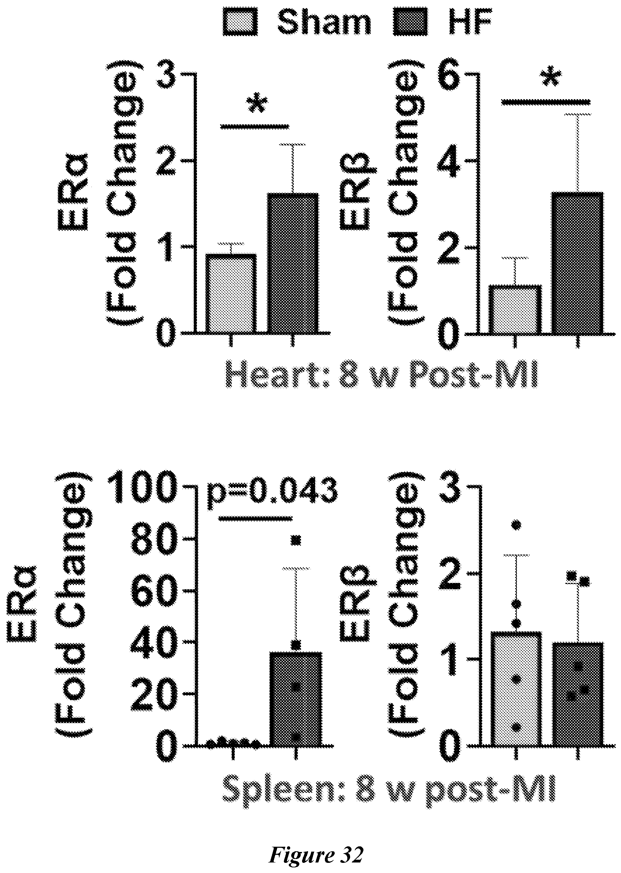

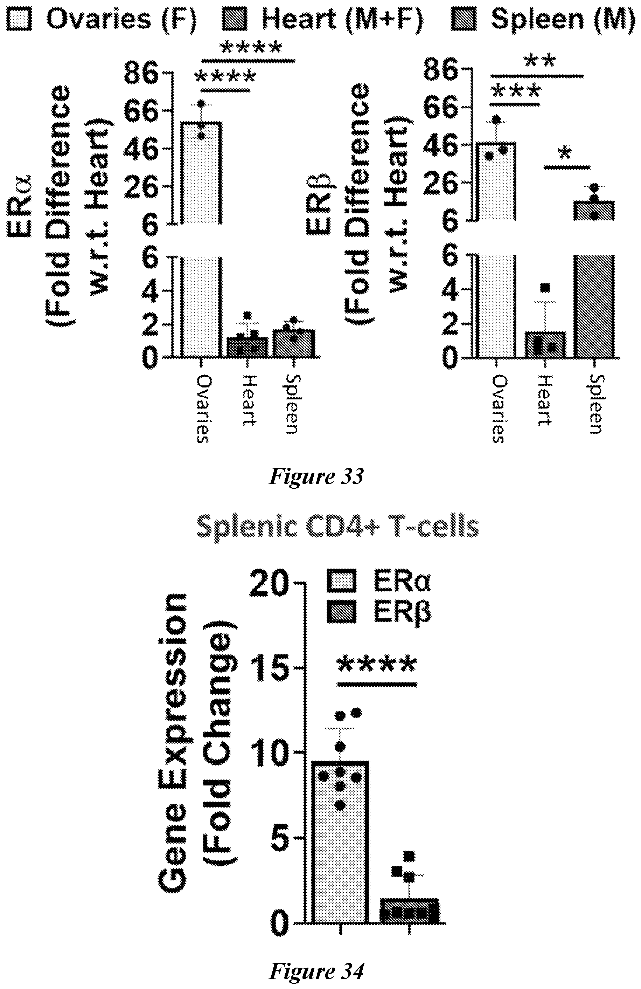

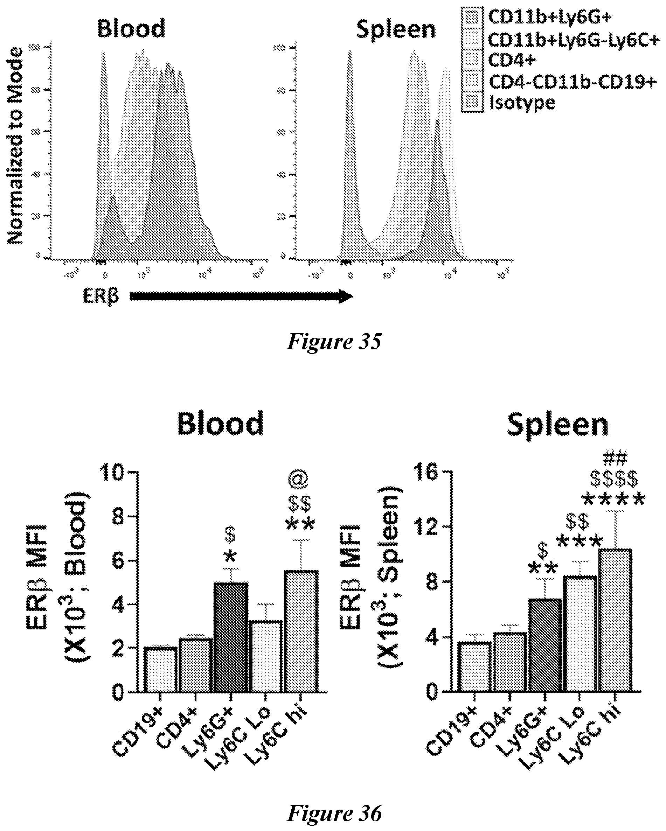

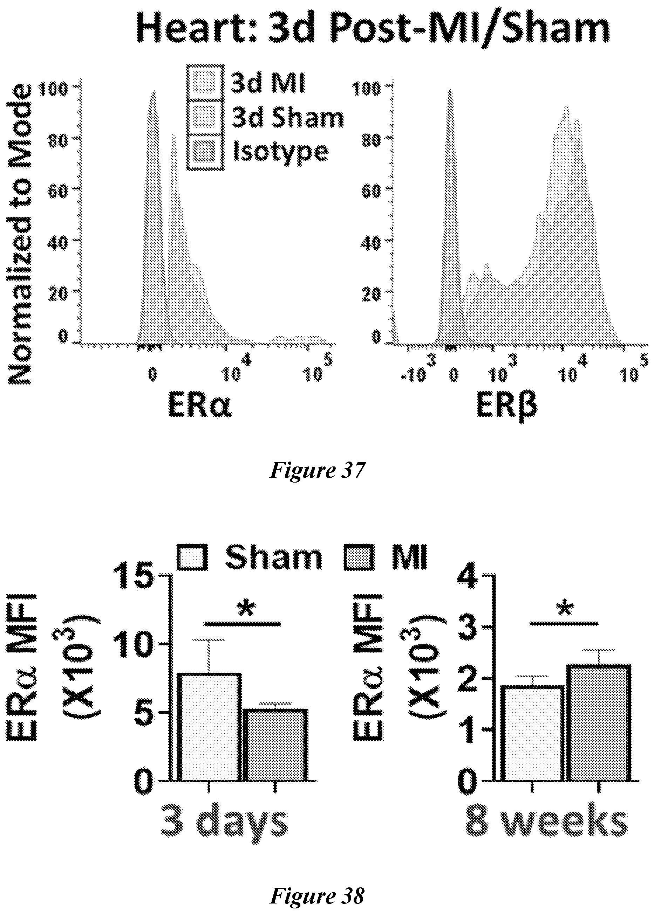

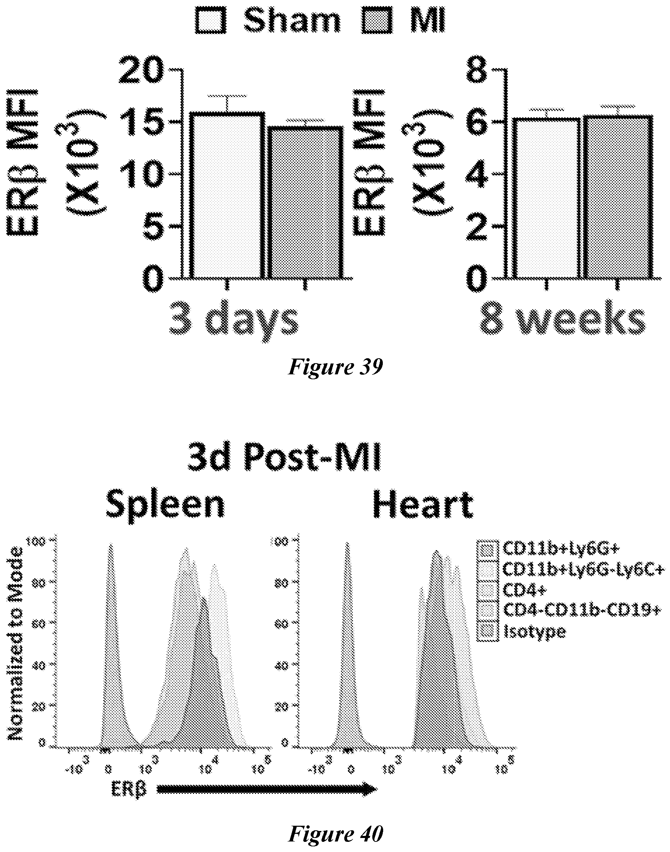

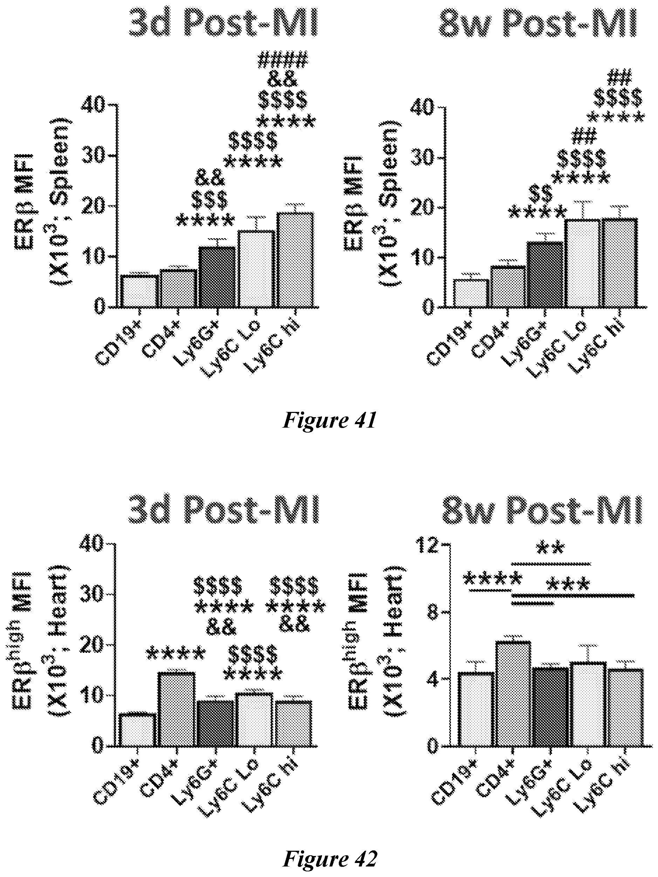

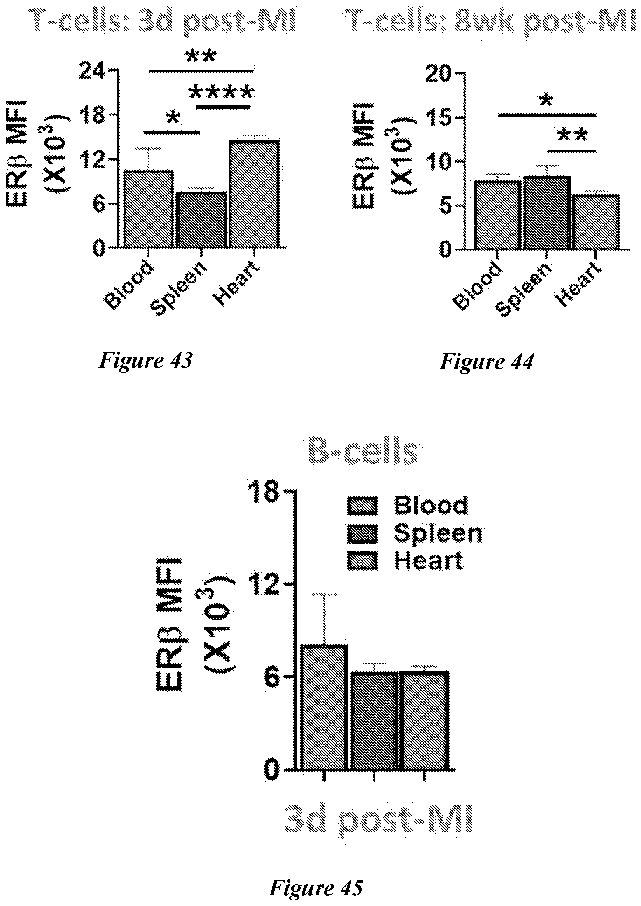

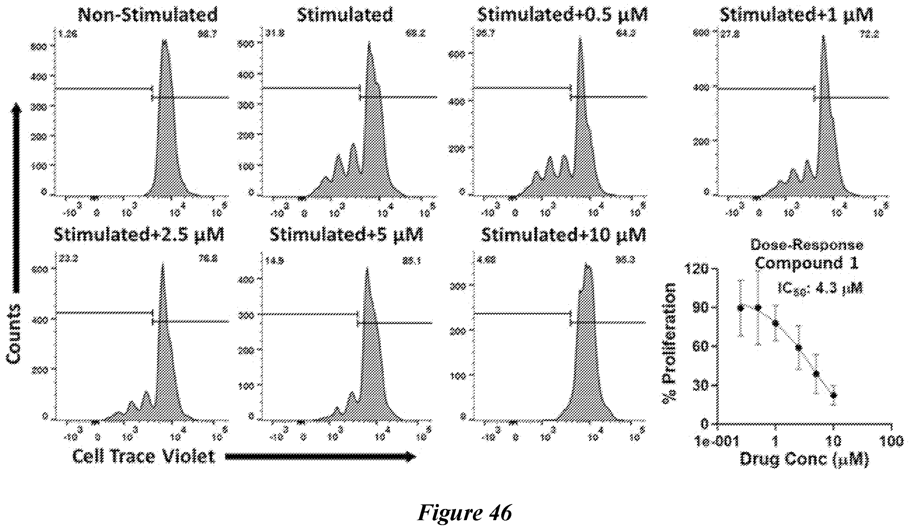

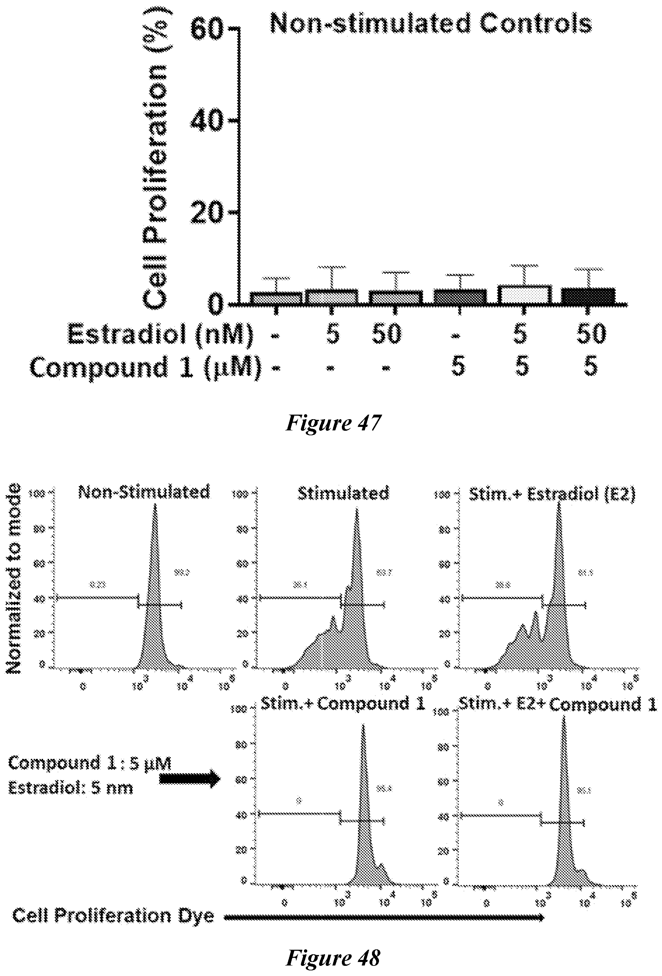

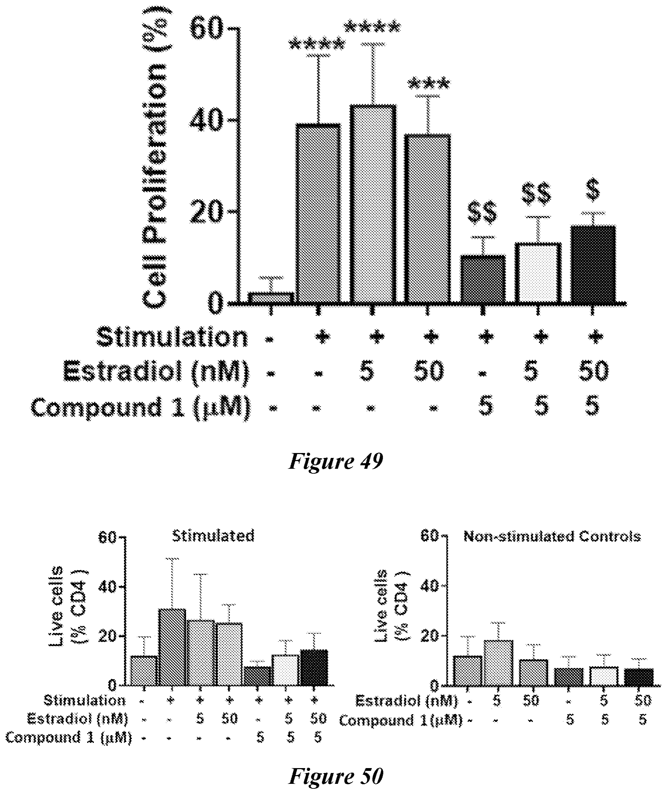

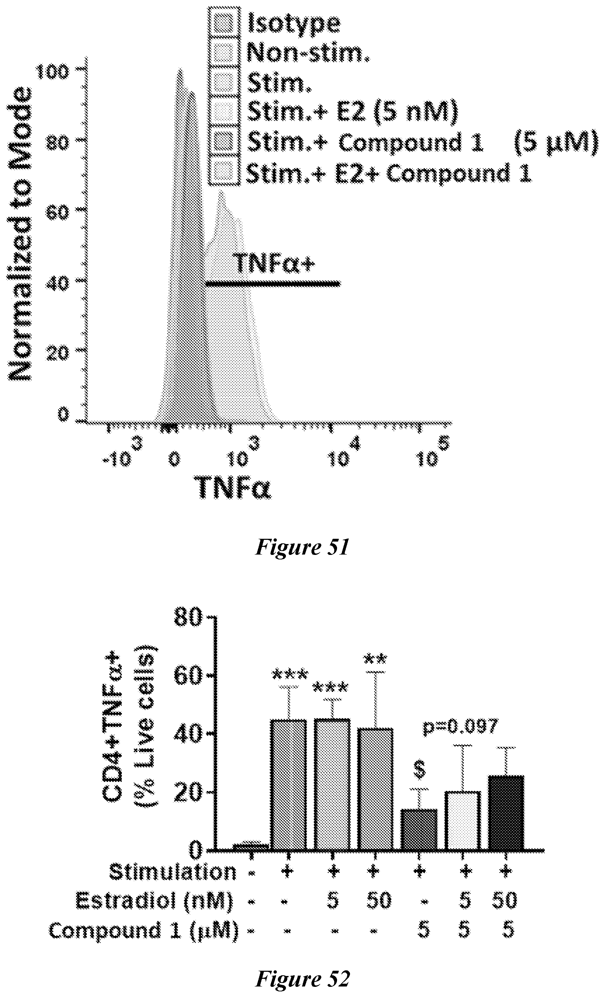

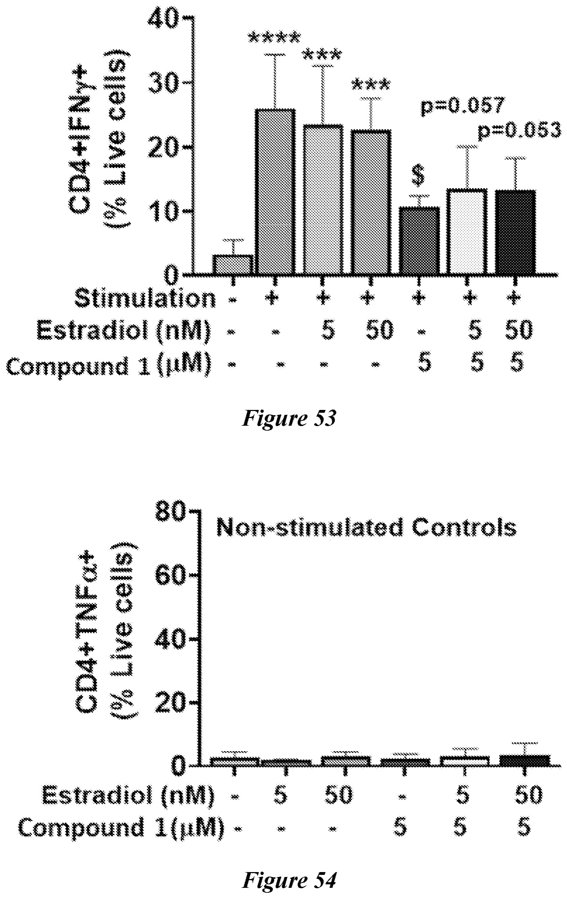

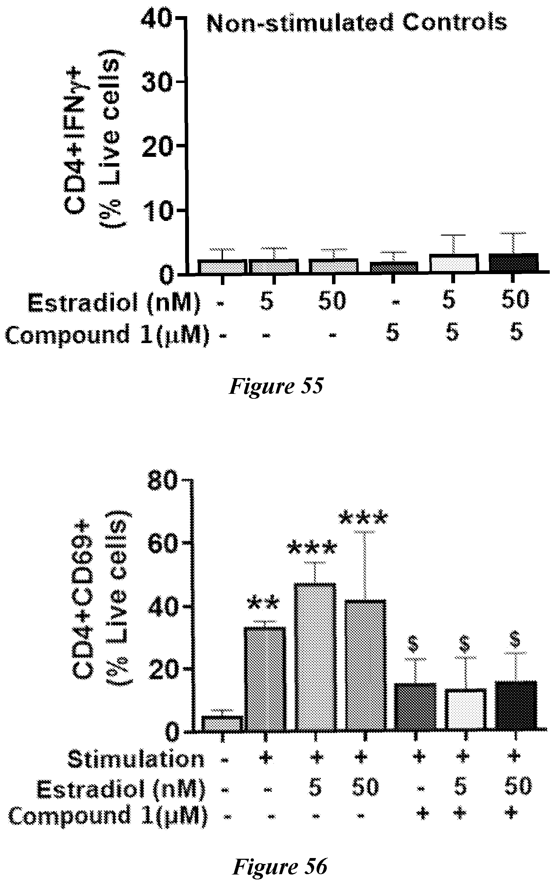

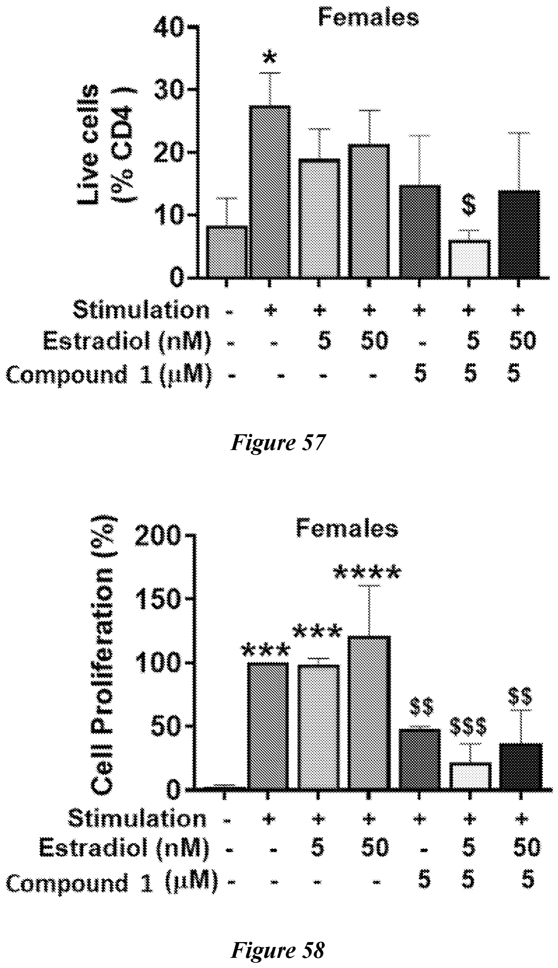

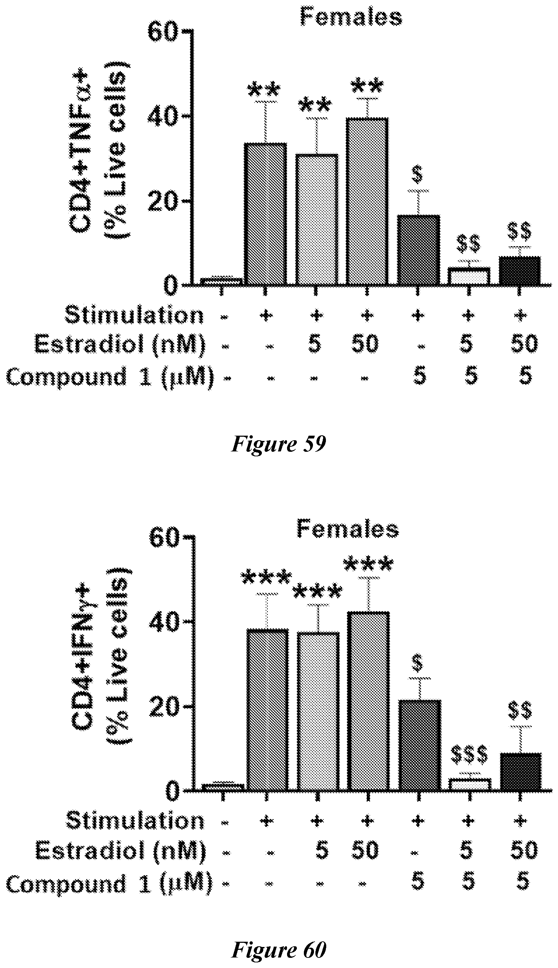

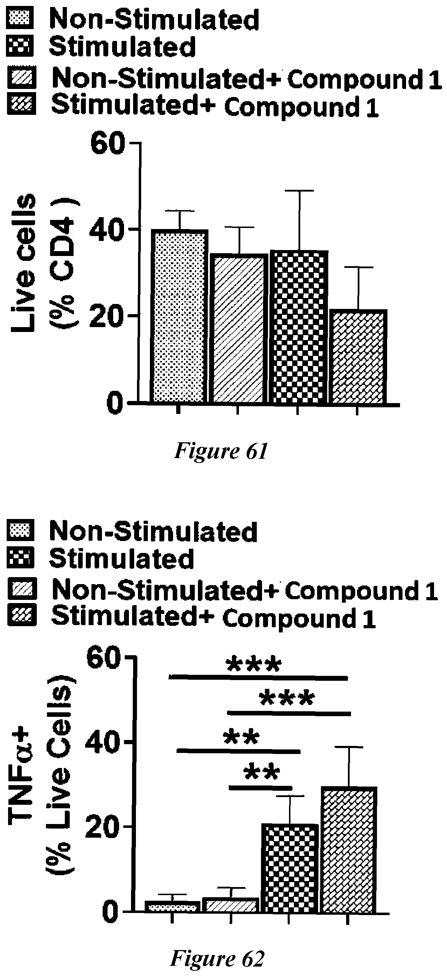

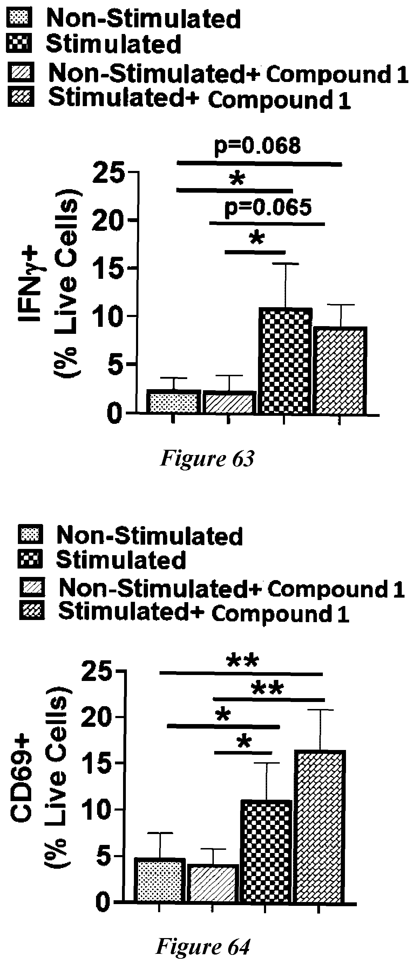

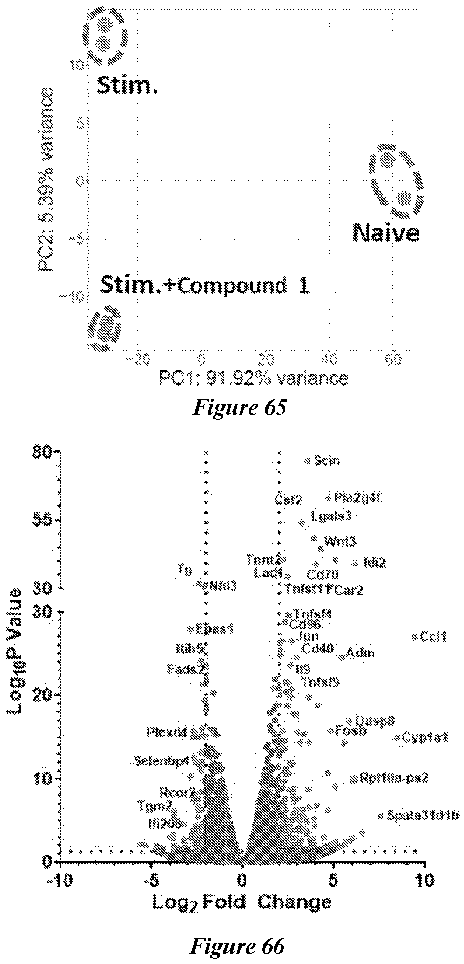

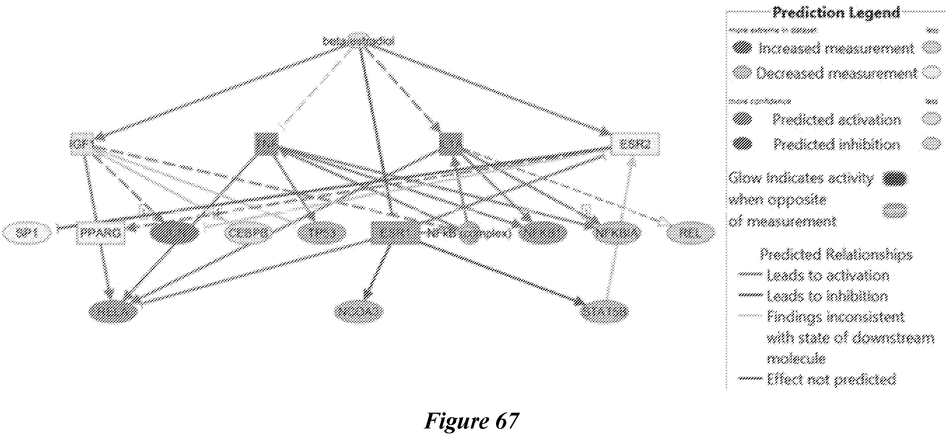

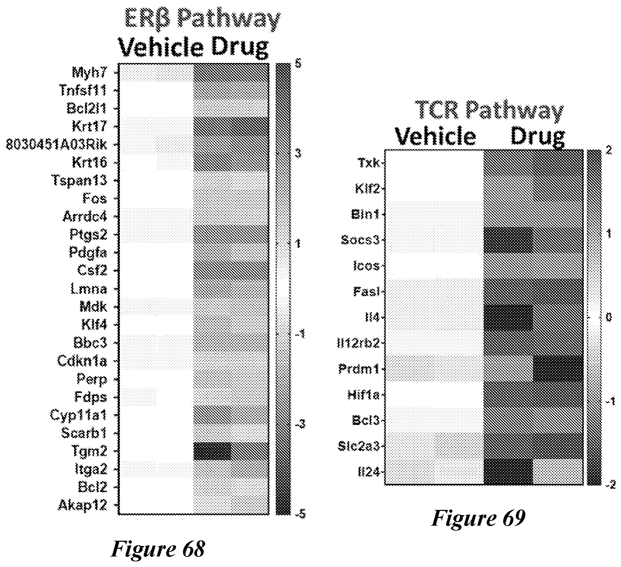

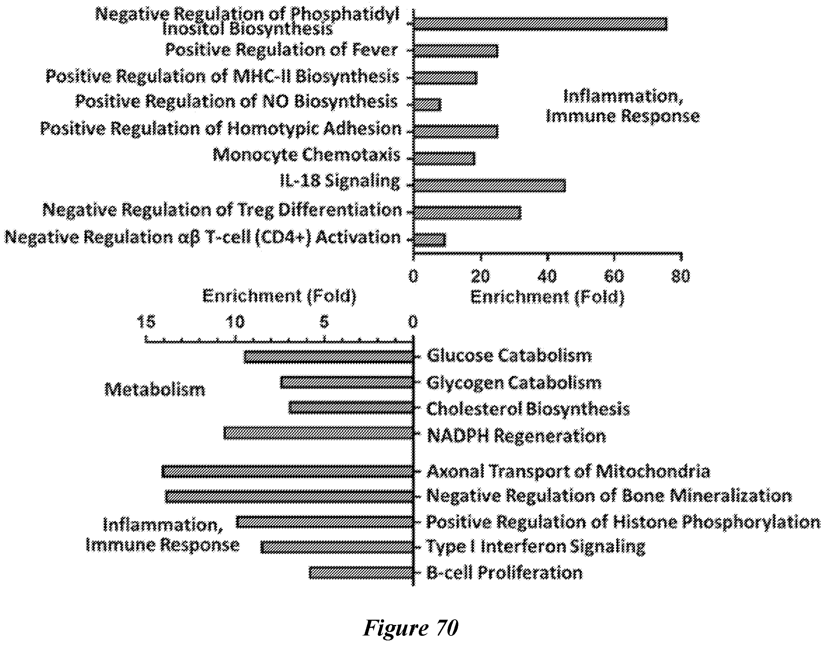

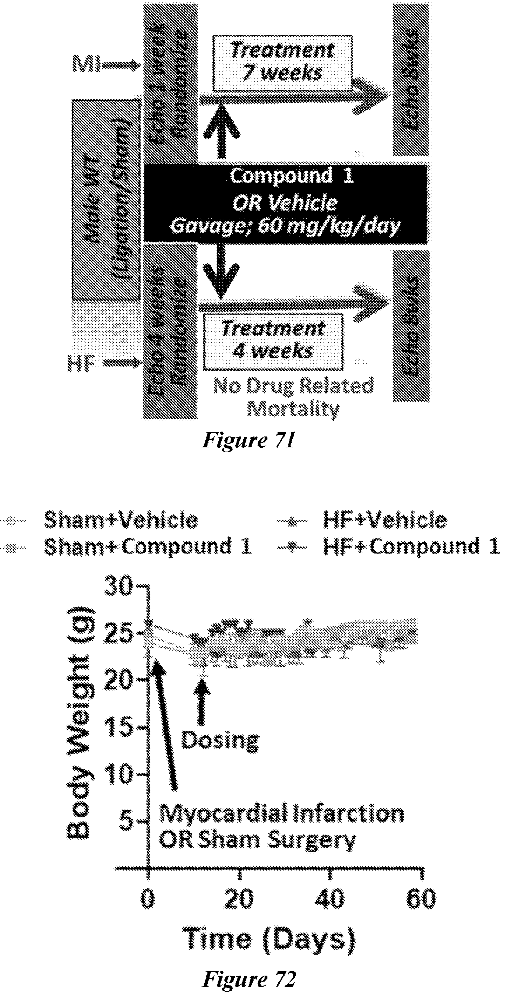

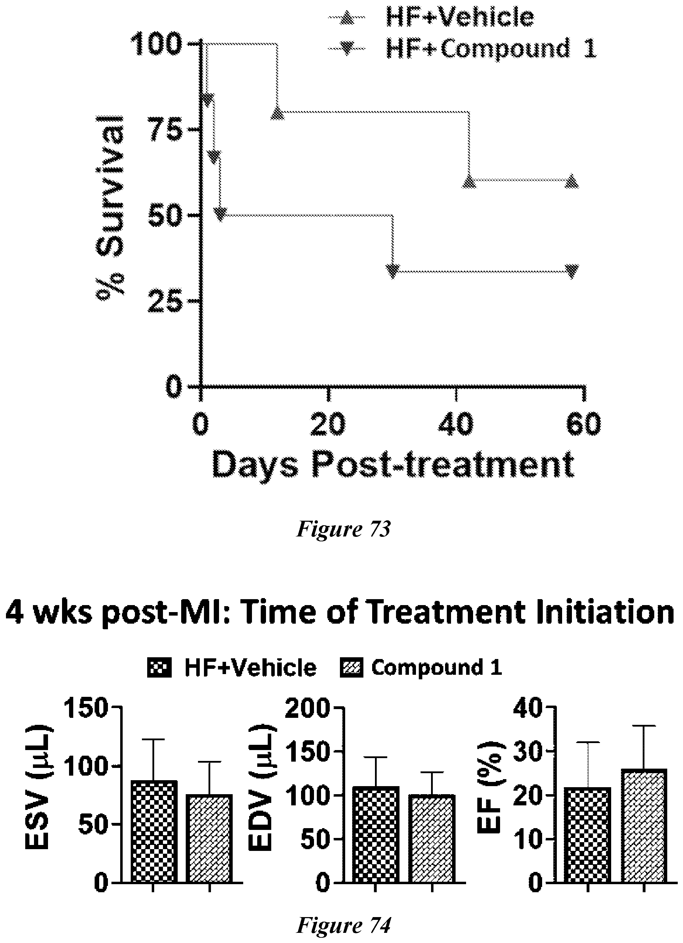

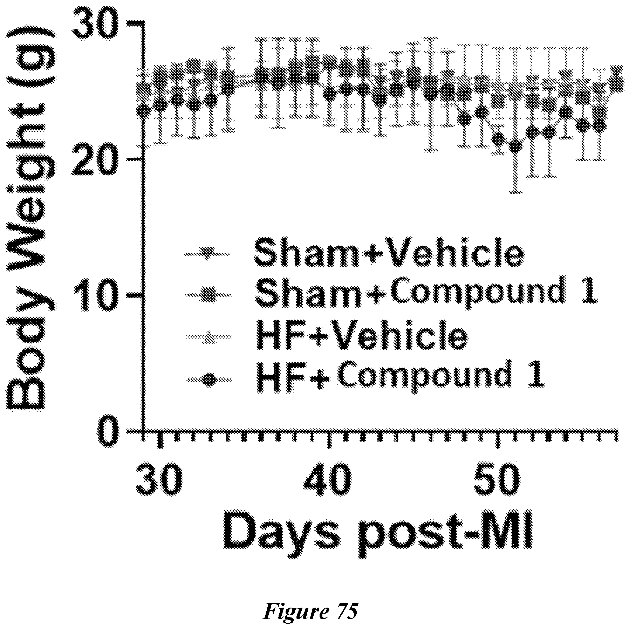

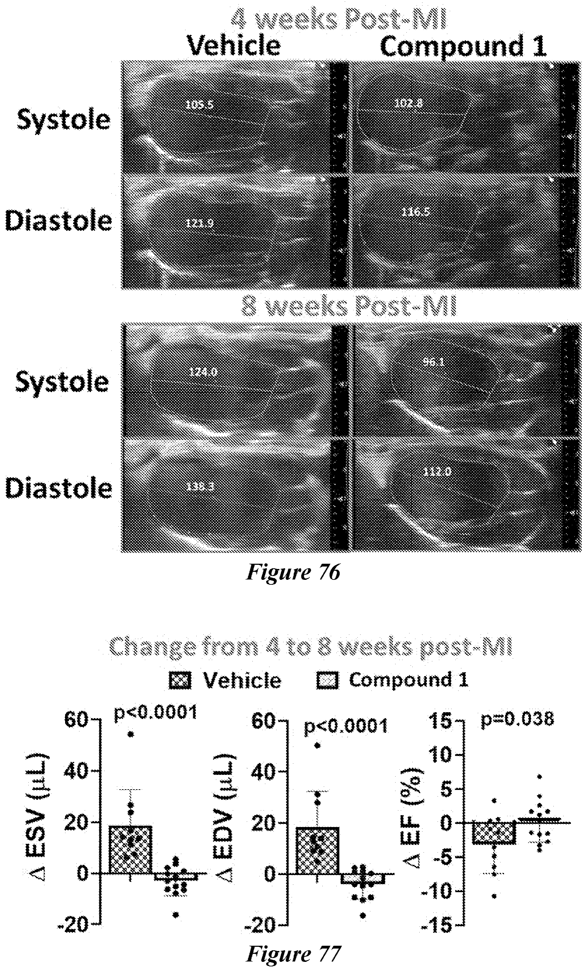

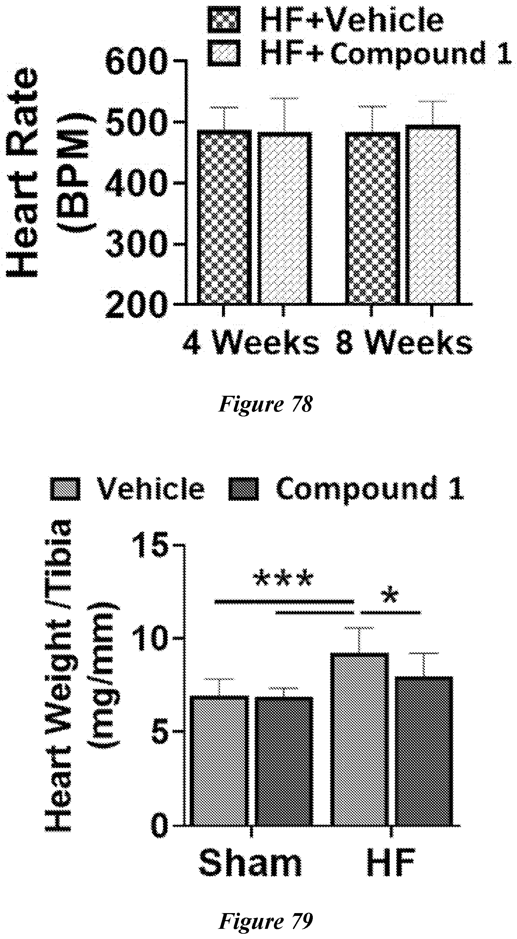

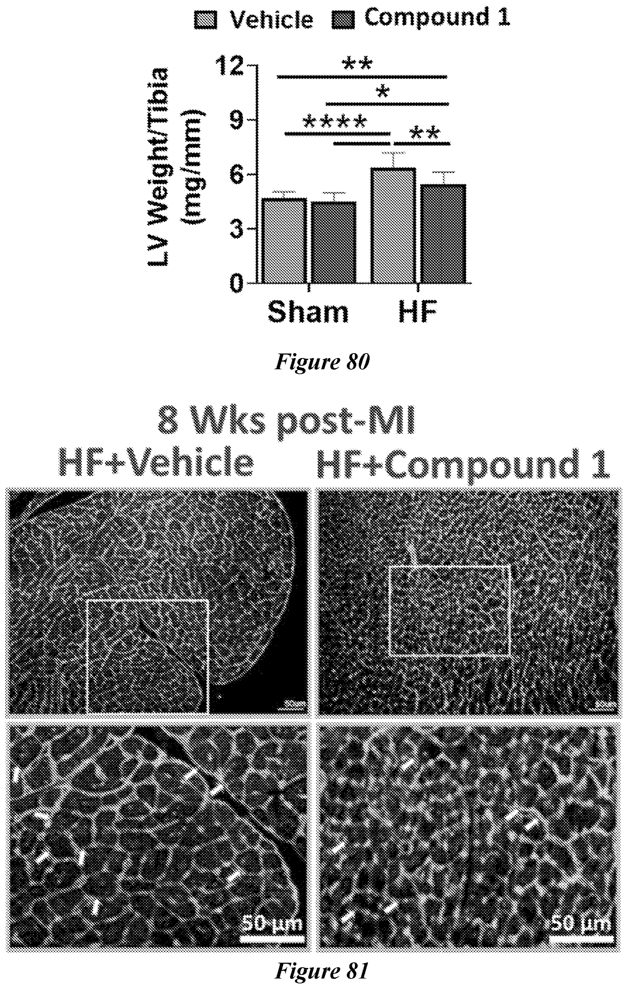

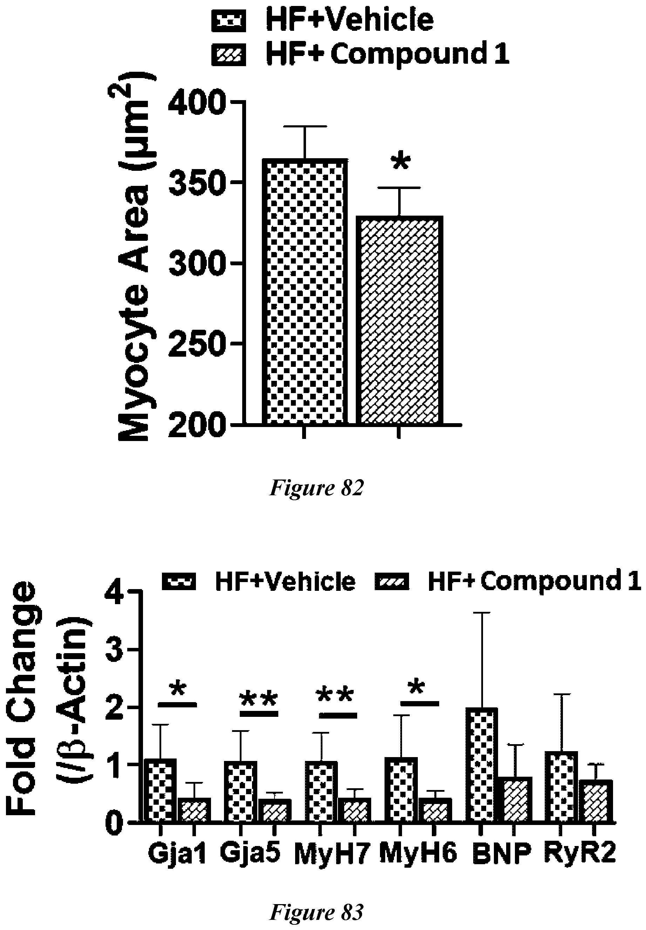

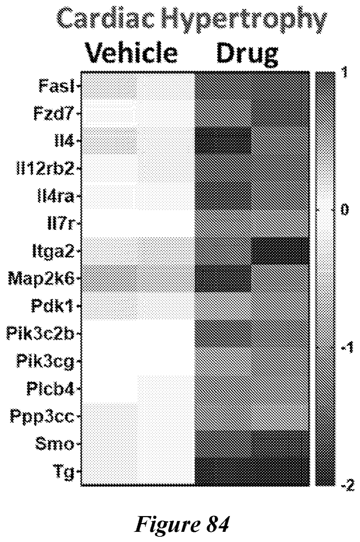

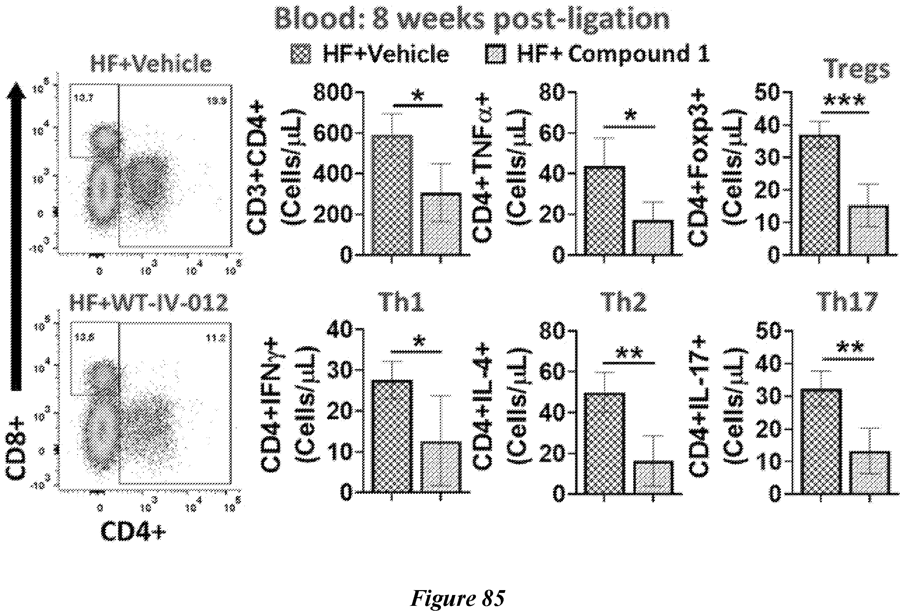

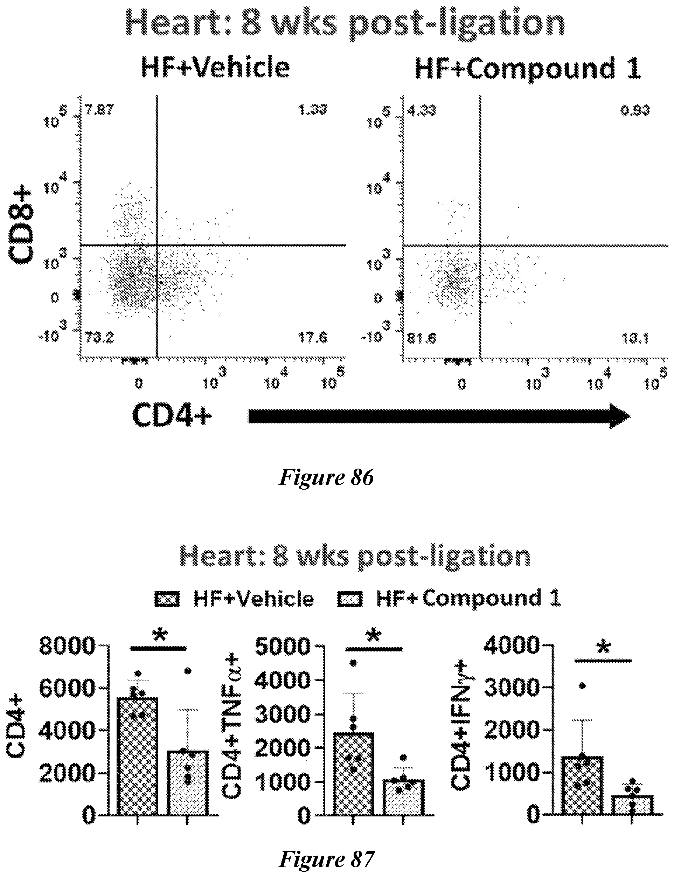

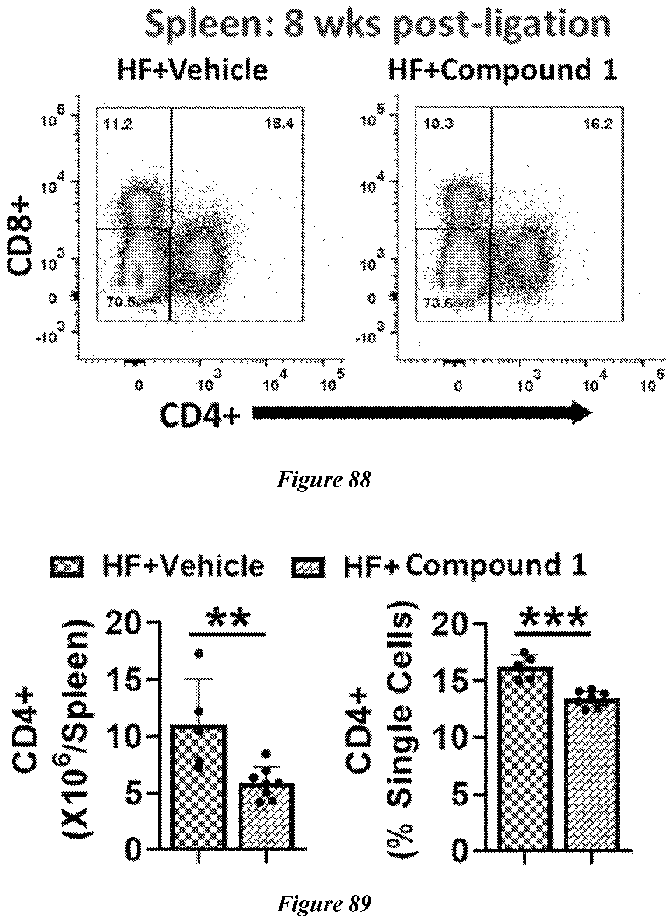

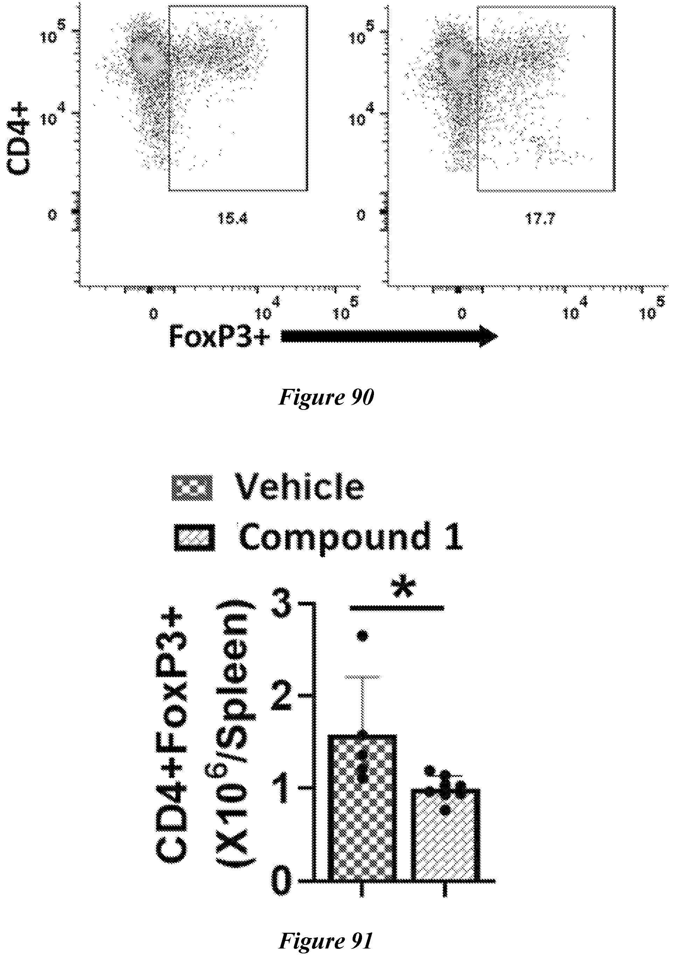

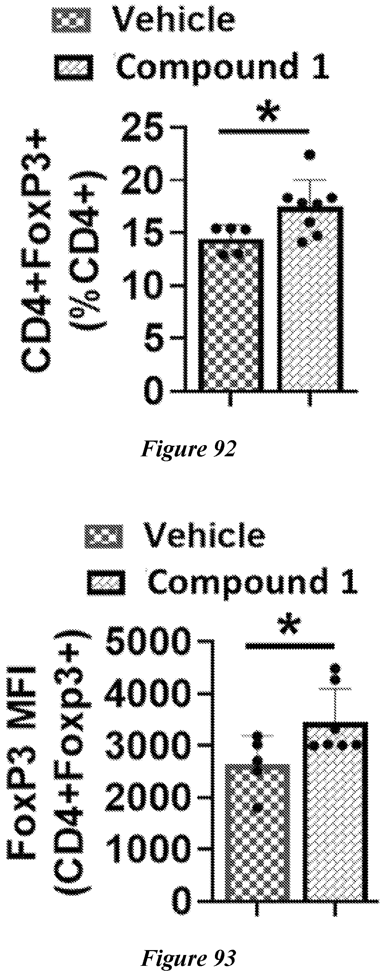

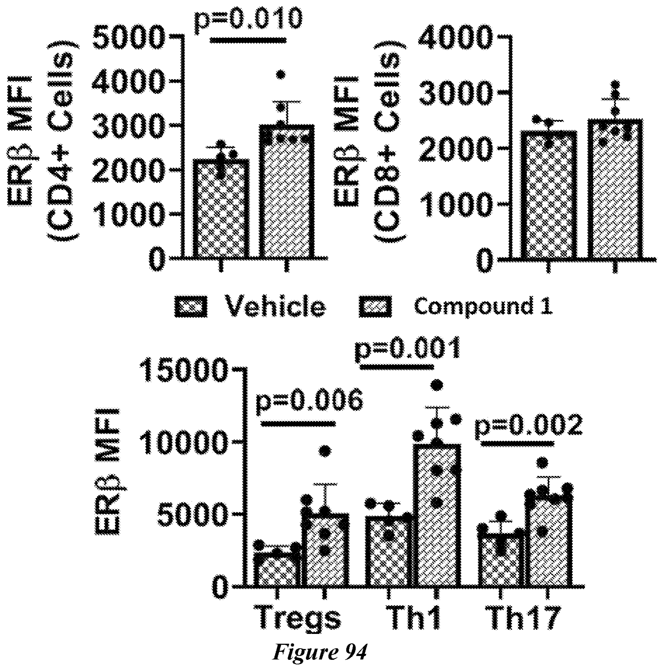

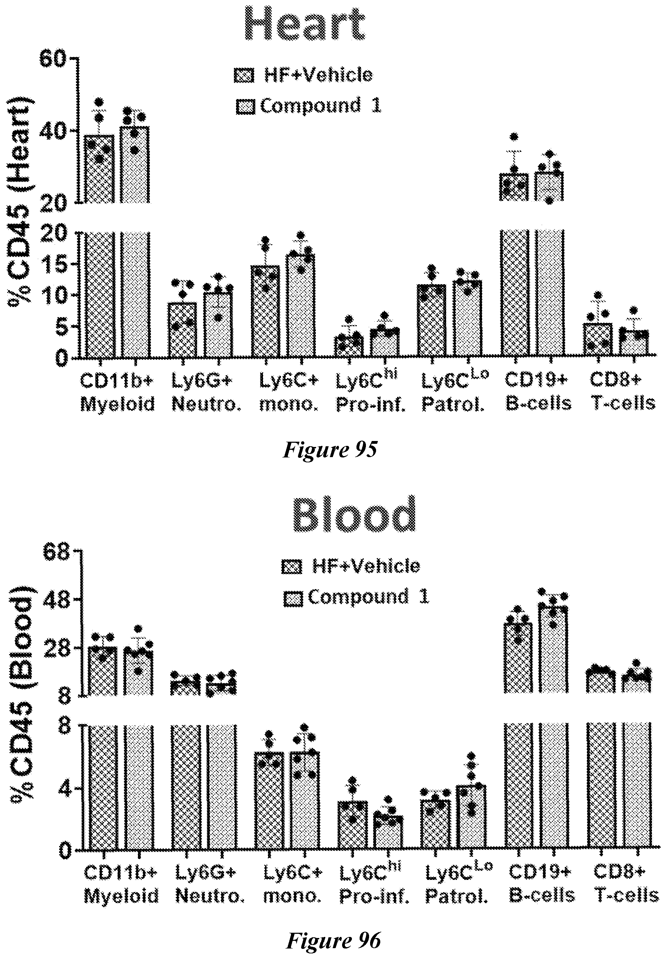

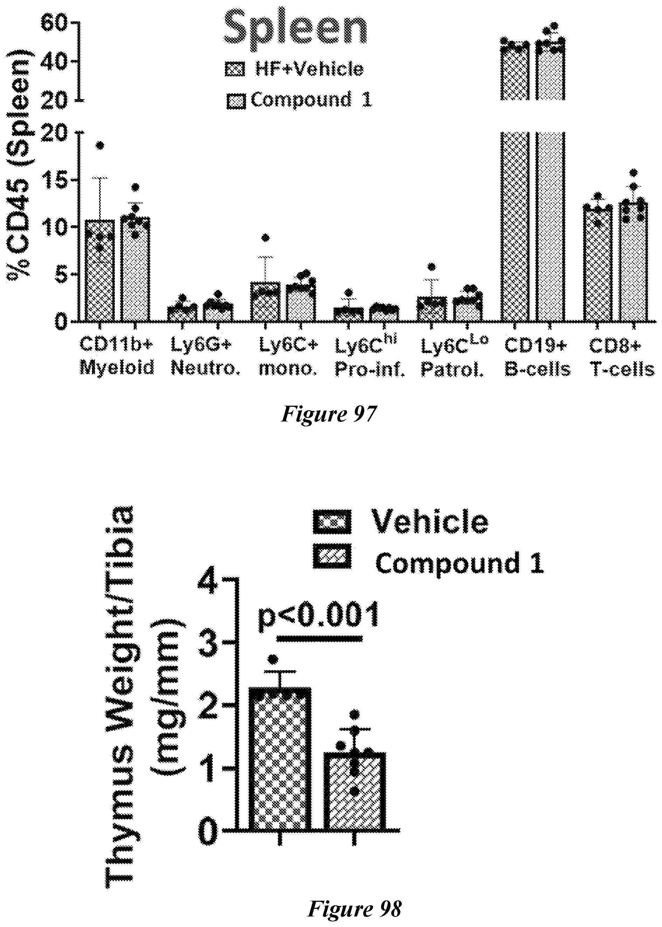

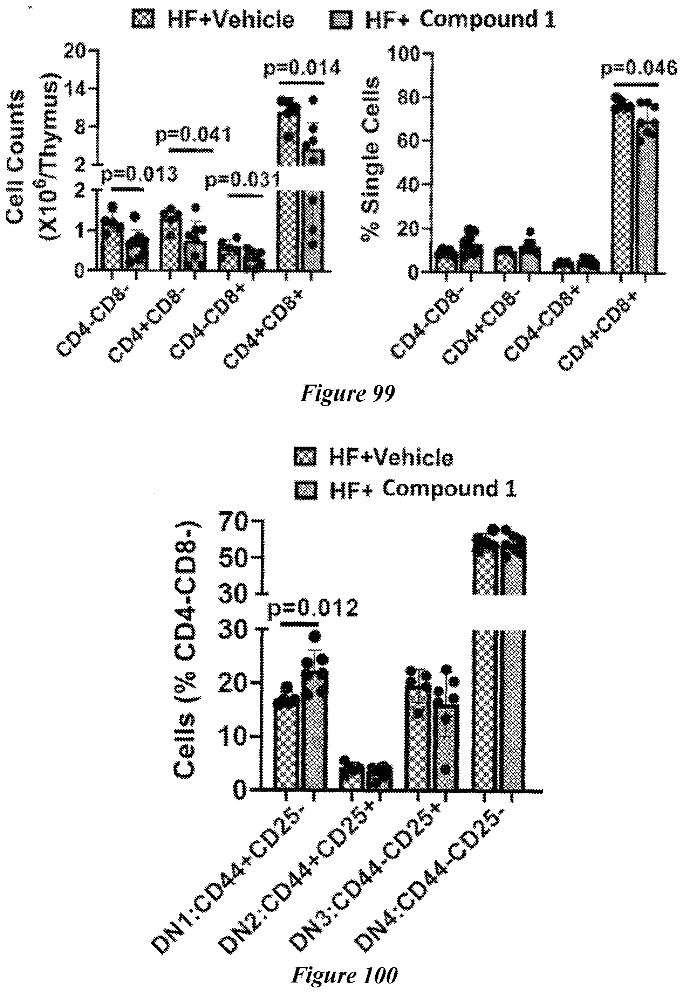

Figure 1. Gene expression for estrogen receptors (ERs) α and β (ERα and ERβ) in the ovaries (positive control), hearts from both males and females (M+F) and male spleens.
Figure 2. Representative flow cytometric histograms for ERα (top panel) and ERβ (bottom panel) expression in different circulating and splenic immune cells in a male mouse.

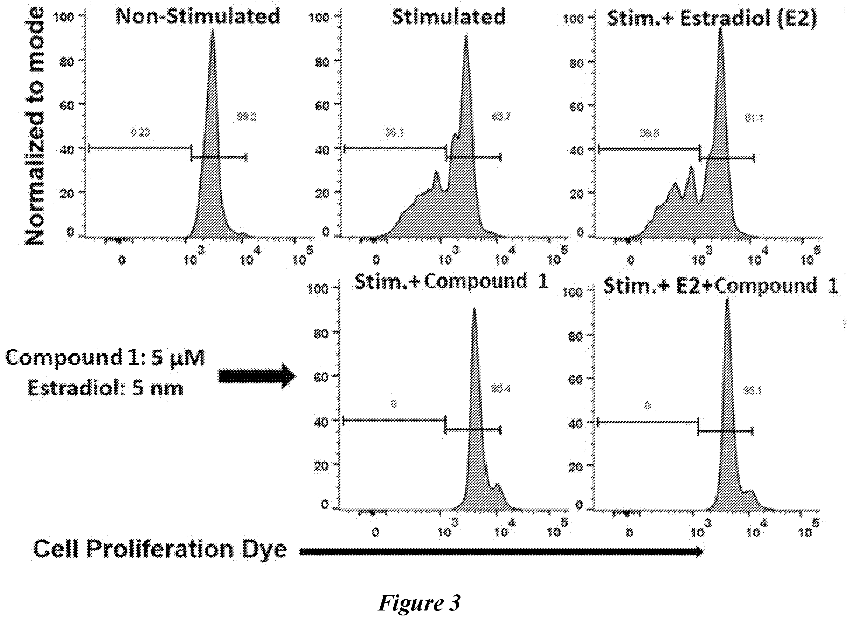

Figure 3. Representative flow cytometric histograms for cell trace violet labeled (CTV; a cell proliferation dye) CD4+ T-cells either unstimulated or CD3/CD28 TCR stimulated and treated with either Estradiol (5 and 50 nM) or Compound 1 (5 µM) or both are shown in Figure 3. Peak patterns from high to low fluorescence intensity in stimulated cells represent halving of dye concentration in the cell membranes of the daughter cells with every successive cell division.

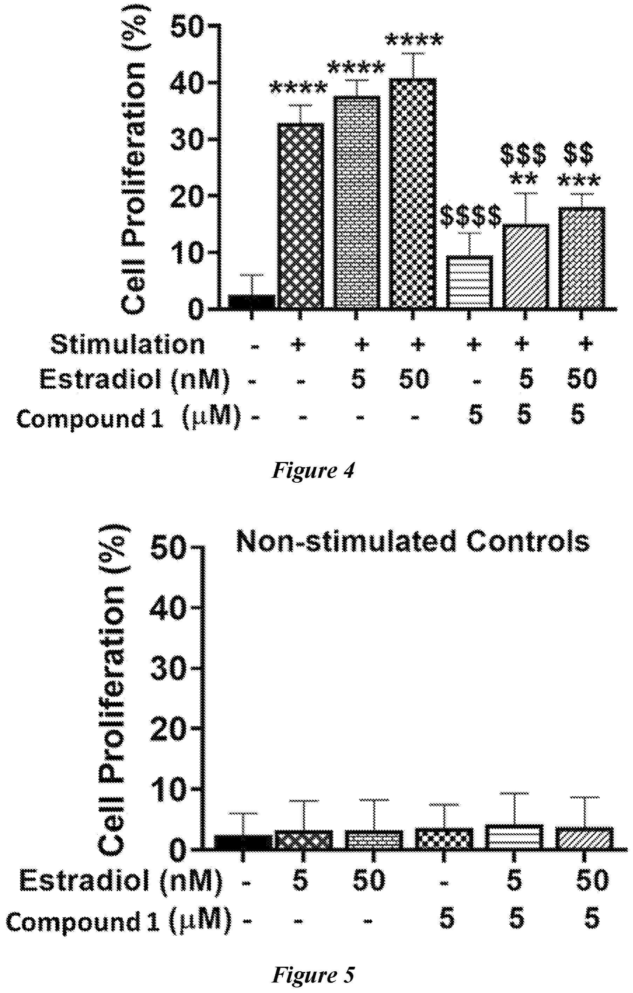

Figure 4. Group quantitation for cell proliferation (%) measured as dye dilution with every successive cell division for stimulated groups. Mean values from 3-separate experiments conducted in quadruplicate by isolating splenic CD4+ T-cells from 3-male mice are reported. One way Anova was used for the data analysis. **P<0.01, ***p<0.001 and ****p<0.0001 represent significance with respect to non-stimulated group whereas ^{$$}P<0.01, ^{$$$}p<0.001 and ^{$$$$}p<0.0001 represent significance with respect to stimulated group.
Figure 5. Group quantitation for cell proliferation (%) measured as dye dilution with every successive cell division for non-stimulated group. Mean values from 3-separate experiments conducted in quadruplicate by isolating splenic CD4+ T-cells from 3-male mice are reported. One way Anova was used for the data analysis. **P<0.01, ***p<0.001 and ****p<0.0001 represent significance with respect to non-stimulated group whereas ^{$$}P<0.01, ^{$$$}p<0.001 and ^{$$$$}p<0.0001 represent significance with respect to stimulated group.
Figure 6. Cell survival of CD3/CD28 mediated *in-vitro* TCR stimulation with and without Compound 1 treatment. Mean values from 3-separate experiments conducted in quadruplicate by isolating splenic CD4+ T-cells from 3-male mice are reported. One way Anova was used for the data analysis.
Figure 7. Representative flow cytometric histograms for TNFα expression in CD4+ T-cells either unstimulated or CD3/CD28 TCR stimulated and treated with either Estradiol (5 and 50 nM) or Compound 1 (5 µM) or both.
Figure 8. Group quantitation for TNFα expressing CD4+ T-cells in stimulated groups. Mean values from 3-separate experiments conducted in quadruplicate by isolating splenic CD4+ T-cells from 3-male mice are reported. One way Anova was used for the data analysis. **p<0.01, and ****p<0.0001 represent significance with respect to non-stimulated group whereas ^{$}P<0.05, and ^{$$$}p<0.001 represent significance with respect to stimulated group without any other treatment.
Figure 9. Group quantitation for TNFα expressing CD4+ helper T-cells in unstimulated control groups. Mean values from 3-separate experiments conducted in quadruplicate by isolating splenic CD4+ T-cells from 3-male mice are reported. One way Anova was used for the data analysis. **p<0.01, and ****p<0.0001 represent significance with respect to non-stimulated group whereas ^{$}P<0.05, and ^{$$$}p<0.001 represent significance with respect to stimulated group without any other treatment.
Figure 10. Representative flow cytometric histograms for IFNγ expression in CD4+ T-cells either unstimulated or CD3/CD28 TCR stimulated and treated with either Estradiol (5 and 50 nM) or Compound 1 (5 µM) or both.
Figure 11. Group quantitation for IFNγ expressing CD4+ T-cells in stimulated groups. Mean values from 3-separate experiments conducted in quadruplicate by isolating splenic CD4+ T-cells from 3-male mice are reported. One way Anova was used for the data analysis. *p<0.05 and **p<0.01 represent significance with respect to non-stimulated group.
Figure 12. Group quantitation for IFNγ expressing CD4+ T-cells in unstimulated control groups. Mean values from 3-separate experiments conducted in quadruplicate by isolating splenic CD4+ T-cells from 3-male mice are reported. One way Anova was used for the data analysis. *p<0.05 and **p<0.01 represent significance with respect to non-stimulated group.
Figure 13. Group quantitation for cell-survival of unstimulated or CD3/CD28 TCR stimulated T-cells isolated from female mice and treated with either Estradiol (5 and 50 nM) or Compound 1 (5 µM) or both. Mean values from 2-separate experiments conducted in quadruplicate by isolating splenic CD4+ T-cells from 2-female mice are reported. Statistical analysis could not be conducted on these data sets as these experiments were repeated from 2-mice only. However, T-cells from female mice exhibited similar trends as were observed with T-cells isolated from male mice.
Figure 14. Group quantitation for proliferation of unstimulated or CD3/CD28 TCR stimulated and treated with either Estradiol (5 and 50 nM) or Compound 1 (5 µM) or both. Mean values from 2-separate experiments conducted in quadruplicate by isolating splenic CD4+ T-cells from 2-female mice are reported. Statistical analysis could not be conducted on these data sets as these experiments were repeated from 2-mice only. However, T-cells from female mice exhibited similar trends as were observed with T-cells isolated from male mice.
Figure 15. Group quantitation for TNFα expressing CD4+ helper T-cells either unstimulated or CD3/CD28 TCR stimulated and treated with either Estradiol (5 and 50 nM) or Compound 1 (5 µM) or both. Mean values from 2-separate experiments conducted in quadruplicate by isolating splenic CD4+ T-cells from 2-female mice are reported. Statistical analysis could not be conducted on these data sets as these experiments were repeated from 2-mice only. However, T-cells from female mice exhibited similar trends as were observed with T-cells isolated from male mice.
Figure 16. Group quantitation for IFNγ expressing CD4+ helper T-cells either unstimulated or CD3/CD28 TCR stimulated and treated with either Estradiol (5 and 50 nM) or Compound 1 (5 µM) or both. Mean values from 2-separate experiments conducted in quadruplicate by isolating splenic CD4+ T-cells from 2-female mice are reported. Statistical analysis could not be conducted on these data sets as these experiments were repeated from 2-mice only. However, T-cells from female mice exhibited similar trends as were observed with T-cells isolated from male mice.
Figure 17. Group quantitation for cell-survival of CD4+ T-cells either unstimulated or stimulated with PMA/Ionomycin and treated with Compound 1 (5 µM). Mean values from 3-separate experiments conducted in quadruplicate by isolating splenic CD4+ T-cells from 3-male mice are reported. Two-way Anova was used for the data analysis.
Figure 18. Group quantitation for TNFα expressing CD4+ helper T-cells either unstimulated or stimulated with PMA/Ionomycin and treated with Compound 1 (5 µM). Mean values from 3-separate experiments conducted in quadruplicate by isolating splenic CD4+ T-cells from 3-male mice are reported. Two-way Anova was used for the data analysis.
Figure 19. Group quantitation for IFNγ expressing CD4+ helper T-cells either unstimulated or stimulated with PMA/Ionomycin and treated with Compound 1 (5 µM). Mean values from 3-separate experiments conducted in quadruplicate by isolating splenic CD4+ T-cells from 3-male mice are reported. Two-way Anova was used for the data analysis.
Figure 20. Group quantitation for CD69+ CD4+ activated helper T-cells either unstimulated or stimulated with PMA/Ionomycin and treated with Compound 1 (5 µM). Mean values from 3-separate experiments conducted in quadruplicate by isolating splenic CD4+ T-cells from 3-male mice are reported. Two-way Anova was used for the data analysis.
Figure 21. Body weight (g) of sham-operated and myocardial infarction (MI) mice treated with either vehicle control or Compound 1. Treatment was started at 7 days post-infarction (designated as day 0 in the graph). For clarity of data, SD is shown only for heart failure group treated with vehicle and was comparable in all groups.
Figure 22. Kaplan-Meier curve to show mortality rate in myocardial infarction and sham-operated mice treated with vehicle or drug. Treatment was started at 7 days post-myocardial infarction (designated as day 0 in both the graphs).
Figure 23. Body weight (g) of sham-operated and myocardial infarction (MI) mice treated with either vehicle control or Compound 1. Treatment was started at 28 days post-infarction to inhibit immune activation in the chronic phase associated with left-ventricular remodeling. For clarity of data, SD is shown only for heart failure groups treated with either vehicle or the Compound 1 and was comparable in all groups.
Figure 24. Tibia normalized heart weights (mg/mm) of sham-operated and myocardial infarction (MI) mice treated with either vehicle control or Compound 1. Treatment was started at 28 days post-infarction to inhibit immune activation in the chronic phase associated with left-ventricular remodeling. Two-way Anova was used for the data analysis: *p<0.05, and ***p<0.001.
Figure 25. Levels of circulating CD4+ Helper T-cells (per µL blood) and its subsets viz CD4+Foxp3+ (Tregs), CD4+TNFα+ cells, CD4+IFNγ+ (Th1), CD4+IL-4+ (Th2) and CD4+IL-17+ (Th17) T-cells at 8 weeks post-surgery in mice treated with either vehicle or Compound 1 from 4 to at 8 weeks post-surgery. Two-way Anova was used for the data analysis.
Figure 26. Quantitative group data for changes in left ventricular end-systolic volume (ESV, left panel), end-diastolic volume (EDV, middle panel), and ejection fraction (EF, right panel) in ligated mice before (4 weeks post-myocardial infarction) and after (8 weeks post-myocardial infarction) treatment with either vehicle or Compound 1. Students unpaired 2-tailed T-test was used for the data analysis.
Figure 27. Schematic showing kinetics of CD4+ T-Cells in the myocardium at different time intervals post-myocardial infarction.
Figure 28. Experimental design for study #1. An attrition rate of 10 and 40% for sham and heart failure groups is considered. LAD: Left-anterior descending coronary artery ligation.
Figure 29. Experimental design for study #2. An attrition rate of 10 and 40% for sham and heart failure groups is considered.
Figure 30: Schematic to show experimental protocol. At 8 weeks post-MI, CD4+ T-cells from the failing hearts (150 cells) and mediastinal lymph-nodes (300 cells) were flow sorted and the RNA sequencing was conducted to identify differential gene expression changes.
Figure 31: IPA analysis identified SIRT1 activation as a positive upstream regulator in cardiac CD4+ T-cells vs lymph nodes. The strongest activation node downstream of SIRT1 was found to be ESR1 (ERα).
Figure 32: Predicted ESR1 dependent gene expression changes in the dataset are indicated.
Figure 33: Gene expression ERα and ERβ in female ovaries, hearts from males and females and spleens from males. *P<0.05, **P<0.01, ***p<0.001, and ****p<0.0001 represent significance with respect to groups shown.
Figure 34: Gene expression of ERα and ERβ in splenic T-cells isolated from naive mice. *P<0.05, **P<0.01, ***p<0.001, and ****p<0.0001 represent significance with respect to groups shown.
Figure 35: Representative flow cytometric histograms for ERβ expression in different circulating *(left)* and splenic *(right)* immune cells in male mice.
Figure 36: Group quantitation for ERβ expression in different circulating *(left)* and splenic *(right)* immune cells in male mice. Data was analyzed using 1-way ANOVA with correction for multiple comparisons using Two-stage method of Benjamini, Krieger and Yekutieli by controlling the false discovery rate. *p<0.05, **p<0.01, ***p<0.001, and ****p<0.0001 represent significance with respect to CD19+ B-Cells, ^{$}P<0.05, ^{$$}p<0.01 and ^{$$$}p<0.001 represent significance with respect to CD4+ T-cells, ^{##}P<0.05 represent significance with respect to Ly6G+ neutrophils, and ^{@}P<0.05 represent significance with respect to Ly6C^{low} monocytes.
Figure 37: Representative flow histograms showing ERα and ERβ expression in cardiac T-cells at 3 days post-MI.
Figure 38: Group quantitation for ERα expression in cardiac T-cells at 3 d and at 8 w post-MI. Data was analyzed using two-tailed students T-test. *P<0.05 represents significance with respect to groups shown.
Figure 39: Group quantitation for ERβ expression in cardiac T-cells at 3 d and at 8 w post-MI. Data was analyzed using two-tailed students T-test. *P<0.05 represents significance with respect to groups shown.
Figure 40: Representative flow histograms showing ERβ expression in different splenic (*left*) and cardiac (*right*) immune cells at 3 d post-MI.
Figure 41: Group quantitation for mean fluorescence intensity (MFI) of ERβ in CD19⁺ B-cells, CD4⁺ T-cells, CD11b⁺Ly6G⁺ neutrophils and CD11b⁺Ly6G⁻Ly6C⁺ monocytes in the spleens at 3d (*left*) and at 8 w post-MI (*right*). Data was analyzed using 1-way ANOVA with Tukey's post-hoc test. ****p<0.0001 represent significance with respect to CD19+ B-Cells, ^{$$}P<0.01, ^{$$$}p<0.001 and ^{$$$$}p<0.0001 represent significance with respect to CD4+ T-cells, ^{&&}P<0.01 represent significance with respect to Ly6C^{low} monocytes, and ^{####}P<0.01 represent significance with respect to Ly6G+ neutrophils.
Figure 42: Group quantitation for mean fluorescence intensity (MFI) of ERβ in CD19⁺ B-cells, CD4⁺ T-cells, CD11b⁺Ly6G⁺ neutrophils and CD11b⁺Ly6G⁻Ly6C⁺ monocytes in the hearts at 3d (*left*) and at 8 w post-MI (*right*). Data was analyzed using 1-way ANOVA with Tukey's post-hoc test. ****p<0.0001 represent significance with respect to CD19+ B-Cells, ^{$$}P<0.01, ^{$$$}p<0.001 and ^{$$$$}p<0.0001 represent significance with respect to CD4+ T-cells, ^{&&}P<0.01 represent significance with respect to Ly6C^{low} monocytes, and ^{####}P<0.01 represent significance with respect to Ly6G+ neutrophils. *P<0.05, **P<0.01, and ***p<0.001 represent significance with respect to groups shown.
Figure 43: Group quantitation for mean fluorescence intensity (MFI) of ERβ in CD4⁺ T-cells in the spleen, blood, and the hearts at 3 d. Data was analyzed using 1-way ANOVA with Tukey's post-hoc test. *P<0.05, **P<0.01, and ***p<0.001 represent significance with respect to groups shown.
Figure 44: Group quantitation for mean fluorescence intensity (MFI) of ERβ in CD4⁺ T-cells in the spleen, blood, and the hearts at 8 w post-MI. Data was analyzed using 1-way ANOVA with Tukey's post-hoc test. *P<0.05, **P<0.01, and ***p<0.001 represent significance with respect to groups shown.
Figure 45: ERβ expression (mean fluorescence intensity) in circulating, splenic, and cardiac CD19+ B-cells at 3d post-MI.
Figure 46: Representative flow cytometric histograms for cell trace violet (CTV) labeled CD4+ T-cells either non-stimulated or stimulated with anti-CD3 and anti-CD28 antibodies in the absence and presence of different concentrations of compound 1. Peak patterns from high to low fluorescence intensity in stimulated cells represent halving of dye concentration in the cell membranes of the daughter cells with every successive cell division. Cell proliferation (%) in the presence of different concentrations of the drug is used to derive dose-response curve (*lower panel, right*).
Figure 47: Cell proliferation (%) in non-stimulated CD4+ T-cells treated either with the vehicle control or estradiol (5 and 50 nM) in the presence or absence of compound 1. Mean±SD from 3-separate experiments conducted in triplicate by isolating splenic CD4+ T-cells from 3-male mice are reported. Data was analyzed using 1-way ANOVA with correction for multiple comparisons using Two-stage method of Benjamini, Krieger and Yekutieli by controlling the false discovery rate. **p<0.01, and ***p<0.001 represent significance with respect to non-stimulated group whereas ^{$}P<0.05 represent significance with respect to stimulated group treated with vehicle.
Figure 48: Representative flow cytometric histograms for CTV labeled CD4+ T-cells either non-stimulated or stimulated with anti-CD3 and anti-CD28 antibodies and treated with either Estradiol (5 and 50 nM) or compound 1 (5 µM) or both.
Figure 49: Group quantitation for % cell proliferation. Mean values from 3-separate experiments (conducted by isolating splenic CD4+ T-cells from 3-male mice) done in triplicate are reported. One way Anova with Tukey's post-hoc test was used for the data analysis. **P<0.01, ***p<0.001 and ****p<0.0001 represent significance with respect to non-stimulated group whereas ^{$$}P<0.01, ^{$$$}p<0.001 and ^{$$$$}p<0.0001 represent significance with respect to stimulated group.
Figure 50: Frequency of live cells (%CD4) in stimulated (*left*) or non-stimulated (*right*) CD4+ T-cells treated either with the vehicle control or estradiol (5 and 50 nM) in the presence or absence of compound 1. Mean±SD from 3-separate experiments conducted in triplicate by isolating splenic CD4+ T-cells from 3-male mice are reported. Data was analyzed using 1-way ANOVA with correction for multiple comparisons using Two-stage method of Benjamini, Krieger and Yekutieli by controlling the false discovery rate. **p<0.01, and ***p<0.001 represent significance with respect to non-stimulated group whereas ^{$}P<0.05 represent significance with respect to stimulated group treated with vehicle.
Figure 51: Representative flow histograms showing TNFα expression in non-stimulated and stimulated CD4+ T-cells treated either with estradiol or compound 1 or both. Mean values from 3-separate experiments (conducted by isolating splenic CD4+ T-cells from 3-male mice) done in triplicate are reported. One way Anova with Tukey's post-hoc test was used for the data analysis. **P<0.01, ***p<0.001 and ****p<0.0001 represent significance with respect to non-stimulated group whereas ^{$$}P<0.01, ^{$$$}p<0.001 and ^{$$$$}p<0.0001 represent significance with respect to stimulated group.
Figure 52: Group quantitation for frequency of CD4+TNFα+ cells. Mean values from 3-separate experiments (conducted by isolating splenic CD4+ T-cells from 3-male mice) done in triplicate are reported. One way Anova with Tukey's post-hoc test was used for the data analysis. **P<0.01, ***p<0.001 and ****p<0.0001 represent significance with respect to non-stimulated group whereas ^{$$}P<0.01, ^{$$$}p<0.001 and ^{$$$$}p<0.0001 represent significance with respect to stimulated group.
Figure 53: Group quantitation for frequency of CD4+IFNγ+ cells. Mean values from 3-separate experiments (conducted by isolating splenic CD4+ T-cells from 3-male mice) done in triplicate are reported. One way Anova with Tukey's post-hoc test was used for the data analysis. **P<0.01, ***p<0.001 and ****p<0.0001 represent significance with respect to non-stimulated group whereas ^{$$}P<0.01, ^{$$$}p<0.001 and ^{$$$$}p<0.0001 represent significance with respect to stimulated group.
Figure 54: Expression of TNFα in non-stimulated CD4+ T-cells treated either with the vehicle control or estradiol (5 and 50 nM) in the presence or absence of compound 1. Mean±SD from 3-separate experiments conducted in triplicate by isolating splenic CD4+ T-cells from 3-male mice are reported. Data was analyzed using 1-way ANOVA with correction for multiple comparisons using Two-stage method of Benjamini, Krieger and Yekutieli by controlling the false discovery rate. **p<0.01, and ***p<0.001 represent significance with respect to non-stimulated group whereas ^{$}P<0.05 represent significance with respect to stimulated group treated with vehicle.
Figure 55: Expression of IFNγ in non-stimulated CD4+ T-cells treated either with the vehicle control or estradiol (5 and 50 nM) in the presence or absence of compound 1. Mean±SD from 3-separate experiments conducted in triplicate by isolating splenic CD4+ T-cells from 3-male mice are reported. Data was analyzed using 1-way ANOVA with correction for multiple comparisons using Two-stage method of Benjamini, Krieger and Yekutieli by controlling the false discovery rate. **p<0.01, and ***p<0.001 represent significance with respect to non-stimulated group whereas ^{$}P<0.05 represent significance with respect to stimulated group treated with vehicle.
Figure 56: Frequency CD69+ cells (% live cells) in stimulated CD4+ T-cells treated either with the vehicle, Estradiol (5 and 50 nM) or compound 1 drug (5 µM) or both. Mean±SD from 3-separate experiments conducted in triplicate by isolating splenic CD4+ T-cells from 3-male mice are reported. Data was analyzed using 1-way ANOVA with correction for multiple comparisons using Two-stage method of Benjamini, Krieger and Yekutieli by controlling the false discovery rate. **p<0.01, and ***p<0.001 represent significance with respect to non-stimulated group whereas ^{$}P<0.05 represent significance with respect to stimulated group treated with vehicle.
Figure 57: Group quantitation for live cells (%CD4) either unstimulated or CD3/CD28 TCR stimulated and treated with either Estradiol (5 and 50 nM) or compound 1 drug (5 µM) or both. Mean values from 2-separate experiments conducted in quadruplicate by isolating splenic CD4+ T-cells from 2 female mice are reported. Data was analyzed using 1-way ANOVA with correction for multiple comparisons using Two-stage method of Benjamini, Krieger and Yekutieli by controlling the false discovery rate. *p<0.05, **p<0.01 ***p<0.001, and ****p<0.0001 represent significance with respect to vehicle treated non-stimulated group whereas ^{$}P<0.05, ^{$$}P<0.01, and ^{$$$}P<0.001 represent significance with respect to vehicle treated stimulated group.
Figure 58: Group quantitation for proliferation (% live cells) either unstimulated or CD3/CD28 TCR stimulated and treated with either Estradiol (5 and 50 nM) or compound 1 drug (5 µM) or both. Mean values from 2-separate experiments conducted in quadruplicate by isolating splenic CD4+ T-cells from 2 female mice are reported. Data was analyzed using 1-way ANOVA with correction for multiple comparisons using Two-stage method of Benjamini, Krieger and Yekutieli by controlling the false discovery rate. *p<0.05, **p<0.01 ***p<0.001, and ****p<0.0001 represent significance with respect to vehicle treated non-stimulated group whereas ^{$}P<0.05, ^{$$}P<0.01, and ^{$$$}P<0.001 represent significance with respect to vehicle treated stimulated group.
Figure 59: Group quantitation for TNFα+ either unstimulated or CD3/CD28 TCR stimulated and treated with either Estradiol (5 and 50 nM) or compound 1 drug (5 µM) or both. Mean values from 2-separate experiments conducted in quadruplicate by isolating splenic CD4+ T-cells from 2 female mice are reported. Data was analyzed using 1-way ANOVA with correction for multiple comparisons using Two-stage method of Benjamini, Krieger and Yekutieli by controlling the false discovery rate. *p<0.05, **p<0.01 ***p<0.001, and ****p<0.0001 represent significance with respect to vehicle treated non-stimulated group whereas ^{$}P<0.05, ^{$$}P<0.01, and ^{$$$}P<0.001 represent significance with respect to vehicle treated stimulated group.
Figure 60: Group quantitation for IFNγ+ helper CD4+ T-cells either unstimulated or CD3/CD28 TCR stimulated and treated with either Estradiol (5 and 50 nM) or compound 1 drug (5 µM) or both. Mean values from 2-separate experiments conducted in quadruplicate by isolating splenic CD4+ T-cells from 2 female mice are reported. Data was analyzed using 1-way ANOVA with correction for multiple comparisons using Two-stage method of Benjamini, Krieger and Yekutieli by controlling the false discovery rate. *p<0.05, **p<0.01 ***p<0.001, and ****p<0.0001 represent significance with respect to vehicle treated non-stimulated group whereas ^{$}P<0.05, ^{$$}P<0.01, and ^{$$$}P<0.001 represent significance with respect to vehicle treated stimulated group.
Figure 61: Group quantitation for cell-survival either unstimulated or stimulated with PMA/Ionomycin and treated with compound 1 drug (5 µM). Mean values from 3-separate experiments conducted in quadruplicate by isolating splenic CD4+ T-cells from 3-male mice are reported.
Figure 62: Group quantitation for TNFα+ either unstimulated or stimulated with PMA/Ionomycin and treated with compound 1 drug (5 µM). Mean values from 3-separate experiments conducted in quadruplicate by isolating splenic CD4+ T-cells from 3-male mice are reported. Two-way Anova with Tukey's post-hoc test was used for the data analysis and respective p-values are shown. *P<0.05, **P<0.01, ***p<0.001, and ****p<0.0001 represent significance with respect to groups shown.
Figure 63: Group quantitation for IFNγ+ either unstimulated or stimulated with PMA/Ionomycin and treated with compound 1 drug (5 µM). Mean values from 3-separate experiments conducted in quadruplicate by isolating splenic CD4+ T-cells from 3-male mice are reported. Two-way Anova with Tukey's post-hoc test was used for the data analysis and respective p-values are shown. *P<0.05, **P<0.01, ***p<0.001, and ****p<0.0001 represent significance with respect to groups shown.
Figure 64: Group quantitation for CD69+ helper T-cells (CD4+) either unstimulated or stimulated with PMA/Ionomycin and treated with compound 1 drug (5 µM). Mean values from 3-separate experiments conducted in quadruplicate by isolating splenic CD4+ T-cells from 3-male mice are reported. Two-way Anova with Tukey's post-hoc test was used for the data analysis and respective p-values are shown. *P<0.05, **P<0.01, ***p<0.001, and ****p<0.0001 represent significance with respect to groups shown.
Figure 65: Principal Component Analysis of RNA transcriptomes of naive and stimulated CD4+ T-cells treated either with the vehicle (DMSO) or compound 1 (5 µM).
Figure 66: Volcano plot showing several genes (marked as red) are either upregulated or downregulated by more than 2-fold in stimulated CD4+ T-cells treated with compound 1 (5 µM). Some of the representative genes that showed either very high LogP values or very high fold-changes are shown.
Figure 67: Ingenuity Pathway analysis of RNA transcriptomes of stimulated CD4+ T-cells treated either with the vehicle control (DMSO) or compound 1 (5 µM) to show activation of ERβ and downregulation of ERα pathway demonstrating specificity of the drug.
Figure 68: Heat maps depicting genes in the ERβ pathway that are either significantly upregulated or downregulated in stimulated CD4+ T-cells upon treatment with compound 1 (5 µM).
Figure 69: Heat maps depicting genes in the TCR pathway that are either significantly upregulated or downregulated in stimulated CD4+ T-cells upon treatment with compound 1 (5 µM).
Figure 70: Depiction of genes that are either significantly upregulated or downregulated in stimulated CD4+ T-cells upon treatment with compound 1 (5 µM).
Figure 71: Schematic for the experimental plan to test the efficacy of compound 1 during acute phase of MI and during chronic HF.
Figure 72: Body weight (g) of sham-operated and myocardial infarction (MI) mice treated either with the vehicle control or compound 1 (60 mg/kg/day; gavage). For clarity of data, SD is shown only for HF group treated with vehicle and was comparable in all groups.
Figure 73: Kaplan-Meier curve to show mortality rate in MI and sham-operated mice treated with the vehicle or drug. Treatment was started at 7d post-MI (designated as day 0 in Figure 72 and Figure 73 graphs).
Figure 74: End-systolic and end-diastolic volumes (ESV and EDV), and ejection fraction (EF) of mice at 4 w post-MI.
Figure 75: Body weight (g) of sham-operated and MI mice treated either with the vehicle control or compound 1. For clarity of data, SD is shown only for both the HF groups and was comparable in all groups.
Figure 76: Representative B-mode tracings depicting systole and diastole of failing hearts at 4 w (at the time of randomization) and 8 w post-MI after treatment with either the vehicle or the drug.
Figure 77: Group quantitation for the change in end-systolic and end-diastolic volumes (ESV and EV), and the ejection fraction (EF) from 4 to 8 w post-MI after treatment with either the vehicle or the drug. Unpaired students two-tailed T-test was used for analyzing data. *P<0.05 and ****p<0.0001 represent significance with respect to groups shown.
Figure 78: Heart rate (BPM) of mice treated either with the vehicle control or compound 1 at 4 and 8 w post-MI.
Figure 79: Gravimetric data for tibia normalized heart weights of sham and HF mice treated either with the vehicle or compound 1. Two way Anova with Tukey's post-hoc test was used for data analysis. *P<0.05, **P<0.01, ***p<0.001 and ****p<0.0001 represent significance with respect to groups shown.
Figure 80: Gravimetric data for tibia normalized LV weights of sham and HF mice treated either with the vehicle or compound 1. Two way Anova with Tukey's post-hoc test was used for data analysis. *P<0.05, **P<0.01, ***p<0.001 and ****p<0.0001 represent significance with respect to groups shown.
Figure 81: Representative images of LV sections stained with FITC conjugated Wheat-germ agglutinin to show cardiac hypertrophy. Boxed area in the upper panel is shown at its original magnification in the lower panel.
Figure 82: Group quantitation for cardiomyocyte area. Two-tailed students T-test was used for data analysis. *P<0.05, **P<0.01, ***p<0.001 and ****p<0.0001 represent significance with respect to groups shown.
Figure 83: Gene expression of cardiac hypertrophy markers in the remote-zone LV of HF mice treated either with the vehicle or compound 1 from 4 to 8 w post-MI. Two-tailed students T-test was used for data analysis. *P<0.05, **P<0.01, ***p<0.001 and ****p<0.0001 represent significance with respect to groups shown.
Figure 84; Heat map depicting cardiac hypertrophy genes in stimulated CD4+ T-cells upon treatment with compound 1 (5 µM).
Figure 85: Representative flow scatter plots for CD4+ and CD8+ T-cells and quantitative data for circulating CD4+ Helper T-cells (/µL blood) and its subsets viz CD4+TNFα+ cells, CD4+Foxp3+ (Tregs), CD4+IFNγ+ (Th1), CD4+IL-4+ (Th2) and CD4+IL-17+ (Th17) T-cells at 8 w post-MI in mice treated either with vehicle or compound 1 from 4 to 8 w post-surgery. Unpaired students two-tailed T-test was used for data analysis, and *p<0.05, **p<0.01 and ***p<0.001, and ****p<0.0001 were considered significant.
Figure 86: Representative flow scatter plots depicting CD4+ and CD8+ T-cells in total CD45+ cells.
Figure 87: Levels of CD4+ Helper T-cells and its pro-inflammatory subsets viz CD4+TNFα+ cells and CD4+IFNγ+ (Th1), T-cells at 8 weeks post-MI in mice treated either with the vehicle or compound 1 from 4 to at 8 w post-MI. Unpaired Students 2-tailed T-test was used for the data analysis. *P<0.05, **P<0.01, and ***p<0.001 represent significance with respect to groups shown.
Figure 88: Representative flow scatter plots for splenic CD4+ and CD8+ T-cells in CD45+ leukocytes.
Figure 89: Group quantitation for splenic CD4+ Helper T-cells (total cells and frequency) at 8 w post-MI in mice treated either with the vehicle or compound 1 drug from 4 to 8 w post-surgery. Unpaired Students 2-tailed T-test was used for the data analysis. *P<0.05, **P<0.01, and ***p<0.001 represent significance with respect to groups shown.
Figure 90: Representative flow scatter plots for splenic CD4+ and FoxP3+ T-cells.
Figure 91: Quantitative data for splenic CD4+FoxP3+ regulatory T-cells (total cells) at 8 w post-MI in mice treated either with vehicle or compound 1 from 4 to at 8 w post-surgery. Students 2-tailed T-test was used for the data analysis and *p<0.05 was considered significant.
Figure 92: Quantitative data for splenic CD4+FoxP3+ regulatory T-cells (frequency) at 8 w post-MI in mice treated either with vehicle or compound 1 from 4 to at 8 w post-surgery. Students 2-tailed T-test was used for the data analysis and *p<0.05 was considered significant.
Figure 93: Quantitative data for FoxP3 MFI (protein expression) at 8 w post-MI in mice treated either with vehicle or compound 1 from 4 to at 8 w post-surgery. Students 2-tailed T-test was used for the data analysis and *p<0.05 was considered significant.
Figure 94: ERβ MFI (protein expression) in CD4+ and CD8+ T-cells (upper), and in different CD4+ helper T-cell subsets viz Tregs, Th1, and Th17 T-cells at 8 weeks post-surgery in mice treated either with vehicle or compound 1 drug from 4 to at 8 weeks post-surgery. Students 2-tailed T-test was used for the data analysis.
Figure 95: Quantitative data (frequency) for cardiac CD11b+ myeloid cells, CD11b+Ly6G+ neutrophils, CD11b+Ly6G-Ly6C+ monocytes (Ly6C^{high}pro-inflammatory and Ly6C^{low} patrolling), CD19+ B-cells and CD8+ T-cells at 8 w post-surgery in mice treated either with vehicle or compound 1 drug from 4 to 8 w post-surgery. Students 2-tailed T-test was used to compare each cell type.
Figure 96: Quantitative data (frequency) for circulating CD11b+ myeloid cells, CD11b+Ly6G+ neutrophils, CD11b+Ly6G-Ly6C+ monocytes (Ly6C^{high}pro-inflammatory and Ly6C^{low} patrolling), CD19+ B-cells and CD8+ T-cells at 8 w post-surgery in mice treated either with vehicle or compound 1 drug from 4 to 8 w post-surgery. Students 2-tailed T-test was used to compare each cell type.
Figure 97: Quantitative data (frequency) for splenic CD11b+ myeloid cells, CD11b+Ly6G+ neutrophils, CD11b+Ly6G-Ly6C+ monocytes (Ly6C^{high}pro-inflammatory and Ly6C^{low} patrolling), CD19+ B-cells and CD8+ T-cells at 8 w post-surgery in mice treated either with vehicle or compound 1 drug from 4 to 8 w post-surgery. Students 2-tailed T-test was used to compare each cell type.
Figure 98: Tibia normalized thymus weights at 8 weeks post-surgery in mice treated either with vehicle or compound 1 drug from 4 to 8 weeks post-surgery. Students 2-tailed T-test was used to compare each cell type.
Figure 99: Cell counts (left) and frequency (right) of single positive double negative (DN; CD4-CD8-), single positive (SP; CD4+CD8- and CD4-CD8+), and double positive (DP; CD4+CD8+) T-cells in thymus at 8 weeks post-surgery in mice treated either with vehicle or compound 1 drug from 4 to 8 weeks post-surgery. Students 2-tailed T-test was used to compare each cell type.
Figure 100: Double negative (DN) T-cells were further separated into DN1 (CD44+CD25-), DN2 (CD44+CD25+), DN3 (CD44-CD25+), and DN4 (CD44-CD25-) T-cells at 8 weeks post-surgery in mice treated either with vehicle or compound 1 drug from 4 to 8 weeks post-surgery. Students 2-tailed T-test was used to compare each cell type.

### DETAILED DESCRIPTION

The compounds, compositions, and methods described herein may be understood more readily by reference to the following detailed description of specific aspects of the disclosed subject matter and the Examples included therein.

Before the present compounds, compositions, and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

### General Definitions

In this specification and in the claims that follow, reference will be made to a number of terms, which shall be defined to have the following meanings.

Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions, reference to "an agent" includes mixtures of two or more such agents, reference to "the component" includes mixtures of two or more such components, and the like.

"Optional" or "optionally" means that the subsequently described event or circumstance can or cannot occur, and that the description includes instances where the event or circumstance occurs and instances where it does not.

Ranges can be expressed herein as from "about" one particular value, and/or to "about" another particular value. By "about" is meant within 5% of the value, e.g., within 4, 3, 2, or 1% of the value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

It is understood that throughout this specification the identifiers "first" and "second" are used solely to aid in distinguishing the various components and steps of the disclosed subject matter. The identifiers "first" and "second" are not intended to imply any particular order, amount, preference, or importance to the components or steps modified by these terms.

As used herein, by a "subject" is meant an individual. Thus, the "subject" can include domesticated animals (*e.g.,* cats, dogs, etc.), livestock (*e.g.,* cattle, horses, pigs, sheep, goats, etc.), laboratory animals (*e.g.*, mouse, rabbit, rat, guinea pig, etc.), and birds. "Subject" can also include a mammal, such as a primate or a human. Thus, the subject can be a human or veterinary patient. The term "patient" refers to a subject under the treatment of a clinician, e.g., physician.

The term "inhibit" refers to a decrease in an activity, response, condition, disease, or other biological parameter. This can include but is not limited to the complete ablation of the activity, response, condition, or disease. This can also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

By "reduce" or other forms of the word, such as "reducing" or "reduction," is meant lowering of an event or characteristic (e.g., tumor growth). It is understood that this is typically in relation to some standard or expected value, in other words it is relative, but that it is not always necessary for the standard or relative value to be referred to. For example, "reduces tumor growth" means reducing the rate of growth of a tumor relative to a standard or a control.

By "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed. For example, the terms "prevent" or "suppress" can refer to a treatment that forestalls or slows the onset of a disease or condition or reduced the severity of the disease or condition. Thus, if a treatment can treat a disease in a subject having symptoms of the disease, it can also prevent or suppress that disease in a subject who has yet to suffer some or all of the symptoms.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. By way of example, in the context of fibrotic conditions, "treating," "treat," and "treatment" as used herein, refers to partially or completely inhibiting or reducing the fibrotic condition which the subject is suffering. In one embodiment, this term refers to an action that occurs while a patient is suffering from, or is diagnosed with, the fibrotic condition, which reduces the severity of the condition, or retards or slows the progression of the condition. Treatment need not result in a complete cure of the condition; partial inhibition or reduction of the fibrotic condition is encompassed by this term.

"Therapeutically effective amount," as used herein, refers to a minimal amount or concentration of an ERβ agonist that, when administered alone or in combination, is sufficient to provide a therapeutic benefit in the treatment of the condition, or to delay or minimize one or more symptoms associated with the condition. The term "therapeutically effective amount" can encompass an amount that improves overall therapy, reduces or avoids symptoms or causes of the condition, or enhances the therapeutic efficacy of another therapeutic agent. The therapeutic amount need not result in a complete cure of the condition; partial inhibition or reduction of the fibrotic condition is encompassed by this term.

As used herein, unless otherwise specified, the terms "prevent," "preventing" and "prevention" refers to an action that occurs before the subject begins to suffer from the condition, or relapse of such condition. The prevention need not result in a complete prevention of the condition; partial prevention or reduction of the fibrotic condition is encompassed by this term.

As used herein, unless otherwise specified, a "prophylactically effective amount" of an ERβ that, when administered alone or in combination, prevent the condition, or one or more symptoms associated with the condition, or prevent its recurrence. The term "prophylactically effective amount" can encompass an amount that improves overall prophylaxis or enhances the prophylactic efficacy of another prophylactic agent. The prophylactic amount need not result in a complete prevention of the condition; partial prevention or reduction of the fibrotic condition is encompassed by this term.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

### Chemical Definitions

Terms used herein will have their customary meaning in the art unless specified otherwise. The organic moieties mentioned when defining variable positions within the general formulae described herein (e.g., the term "halogen") are collective terms for the individual substituents encompassed by the organic moiety. The prefix Cₙ-Cₘ preceding a group or moiety indicates, in each case, the possible number of carbon atoms in the group or moiety that follows.

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, heteroatoms present in a compound or moiety, such as nitrogen, can have hydrogen substituents and/or any permissible substituents of organic compounds described herein which satisfy the valency of the heteroatom. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound (e.g., a compound that does not spontaneously undergo transformation such as by rearrangement, cyclization, elimination, etc.

"Z¹," "Z²," "Z³," and "Z⁴" are used herein as generic symbols to represent various specific substituents. These symbols can be any substituent, not limited to those disclosed herein, and when they are defined to be certain substituents in one instance, they can, in another instance, be defined as some other substituents.

As used herein, the term "alkyl" refers to saturated, straight-chained or branched saturated hydrocarbon moieties. Unless otherwise specified, C₁-C₂₄ (e.g., C₁-C₂₂, C₁-C₂₀, C₁-C₁₈, C₁-C₁₆, C₁-C₁₄, C₁-C₁₂, C₁-C₁₀, C₁-Cs, C₁-C₆, or C₁-C₄) alkyl groups are intended. Examples of alkyl groups include methyl, ethyl, propyl, 1-methyl-ethyl, butyl, 1-methyl-propyl, 2-methyl-propyl, 1,1-dimethyl-ethyl, pentyl, 1-methyl-butyl, 2-methyl-butyl, 3-methyl-butyl, 2,2-dimethyl-propyl, 1-ethyl-propyl, hexyl, 1,1-dimethyl-propyl, 1,2-dimethyl-propyl, 1-methyl-pentyl, 2-methyl-pentyl, 3-methyl-pentyl, 4-methyl-pentyl, 1,1-dimethyl-butyl, 1,2-dimethyl-butyl, 1,3-dimethyl-butyl, 2,2-dimethyl-butyl, 2,3-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl, and 1-ethyl-2-methyl-propyl. Alkyl substituents may be unsubstituted or substituted with one or more chemical moieties. The alkyl group can be substituted with one or more groups including, but not limited to, hydroxy, halogen, acyl, alkyl, alkoxy, alkenyl, alkynyl, aryl, heteroaryl, acyl, aldehyde, amino, carboxylic acid, ester, ether, ketone, nitro, silyl, sulfo-oxo, sulfonyl, sulfone, sulfoxide, or thiol, as described below, provided that the substituents are sterically compatible and the rules of chemical bonding and strain energy are satisfied. The alkyl group can also include one or more heteroatoms (e.g., from one to three heteroatoms) incorporated within the hydrocarbon moiety. Examples of heteroatoms include, but are not limited to, nitrogen, oxygen, sulfur, and phosphorus.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" specifically refers to an alkyl group that is substituted with one or more halides (halogens; e.g., fluorine, chlorine, bromine, or iodine). The term "alkoxyalkyl" specifically refers to an alkyl group that is substituted with one or more alkoxy groups, as described below. The term "alkylamino" specifically refers to an alkyl group that is substituted with one or more amino groups, as described below, and the like. When "alkyl" is used in one instance and a specific term such as "alkylalcohol" is used in another, it is not meant to imply that the term "alkyl" does not also refer to specific terms such as "alkylalcohol" and the like.

This practice is also used for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, *e.g.*, an "alkylcycloalkyl." Similarly, a substituted alkoxy can be specifically referred to as, *e.g.*, a "halogenated alkoxy," a particular substituted alkenyl can be, *e.g.*, an "alkenylalcohol," and the like. Again, the practice of using a general term, such as "cycloalkyl," and a specific term, such as "alkylcycloalkyl," is not meant to imply that the general term does not also include the specific term.

As used herein, the term "alkenyl" refers to unsaturated, straight-chained, or branched hydrocarbon moieties containing a double bond. Unless otherwise specified, C₂-C₂₄ (e.g., C₂-C₂₂, C₂-C₂₀, C₂-C₁₈, C₂-C₁₆, C₂-C₁₄, C₂-C₁₂, C₂-C_{IO}, C₂-Cs, C₂-C₆, C₂-C₄) alkenyl groups are intended. Alkenyl groups may contain more than one unsaturated bond. Examples include ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, and 1-ethyl-2-methyl-2-propenyl. The term "vinyl" refers to a group having the structure -CH=CH₂; 1-propenyl refers to a group with the structure-CH=CH-CH₃; and 2- propenyl refers to a group with the structure -CH₂-CH=CH₂. Asymmetric structures such as (Z¹Z²)C=C(Z³Z⁴) are intended to include both the E and Z isomers. This can be presumed in structural formulae herein wherein an asymmetric alkene is present, or it can be explicitly indicated by the bond symbol C=C. Alkenyl substituents may be unsubstituted or substituted with one or more chemical moieties. Examples of suitable substituents include, for example, alkyl, halogenated alkyl, alkoxy, alkenyl, alkynyl, aryl, heteroaryl, acyl, aldehyde, amino, carboxylic acid, ester, ether, halide, hydroxy, ketone, nitro, silyl, sulfo-oxo, sulfonyl, sulfone, sulfoxide, or thiol, as described below, provided that the substituents are sterically compatible and the rules of chemical bonding and strain energy are satisfied.

As used herein, the term "alkynyl" represents straight-chained or branched hydrocarbon moieties containing a triple bond. Unless otherwise specified, C₂-C₂₄ (e.g., C₂-C₂₂, C₂-C₂₀, C₂-C₁₈, C₂-C₁₆, C₂-C₁₄, C₂-C₁₂, C₂-C_{IO}, C₂-C₈, C₂-C₆, C₂-C₄) alkynyl groups are intended. Alkynyl groups may contain more than one unsaturated bond. Examples include C₂-C₆-alkynyl, such as ethynyl, 1-propynyl, 2-propynyl (or propargyl), 1-butynyl, 2-butynyl, 3-butynyl, 1-methyl-2-propynyl, 1-pentynyl, 2-pentynyl, 3-pentynyl, 4-pentynyl, 3-methyl-1-butynyl, 1-methyl-2-butynyl, 1-methyl-3-butynyl, 2-methyl-3-butynyl, 1,1-dimethyl-2-propynyl, 1-ethyl-2-propynyl, 1-hexynyl, 2-hexynyl, 3-hexynyl, 4-hexynyl, 5-hexynyl, 3-methyl-1-pentynyl, 4-methyl-1-pentynyl, 1-methyl-2-pentynyl, 4-methyl-2-pentynyl, 1-methyl-3-pentynyl, 2-methyl-3-pentynyl, 1-methyl-4-pentynyl, 2-methyl-4-pentynyl, 3-methyl-4-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, and 1-ethyl-1-methyl-2-propynyl. Alkynyl substituents may be unsubstituted or substituted with one or more chemical moieties. Examples of suitable substituents include, for example, alkyl, halogenated alkyl, alkoxy, alkenyl, alkynyl, aryl, heteroaryl, acyl, aldehyde, amino, carboxylic acid, ester, ether, halide, hydroxy, ketone, nitro, silyl, sulfo-oxo, sulfonyl, sulfone, sulfoxide, or thiol, as described below.

As used herein, the term "aryl," as well as derivative terms such as aryloxy, refers to groups that include a monovalent aromatic carbocyclic group of from 3 to 20 carbon atoms. Aryl groups can include a single ring or multiple condensed rings. In some embodiments, aryl groups include C₆-C₁₀ aryl groups. Examples of aryl groups include, but are not limited to, phenyl, biphenyl, naphthyl, tetrahydronaphthyl, phenylcyclopropyl, and indanyl. In some embodiments, the aryl group can be a phenyl, indanyl or naphthyl group. The term "heteroaryl" is defined as a group that contains an aromatic group that has at least one heteroatom incorporated within the ring of the aromatic group. Examples of heteroatoms include, but are not limited to, nitrogen, oxygen, sulfur, and phosphorus. The term "non-heteroaryl," which is included in the term "aryl," defines a group that contains an aromatic group that does not contain a heteroatom. The aryl or heteroaryl substituents may be unsubstituted or substituted with one or more chemical moieties. Examples of suitable substituents include, for example, alkyl, halogenated alkyl, alkoxy, alkenyl, alkynyl, aryl, heteroaryl, acyl, aldehyde, amino, carboxylic acid, cycloalkyl, ester, ether, halide, hydroxy, ketone, nitro, silyl, sulfo-oxo, sulfonyl, sulfone, sulfoxide, or thiol as described herein. The term "biaryl" is a specific type of aryl group and is included in the definition of aryl. Biaryl refers to two aryl groups that are bound together *via* a fused ring structure, as in naphthalene, or are attached *via* one or more carbon-carbon bonds, as in biphenyl.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, etc. The term "heterocycloalkyl" is a cycloalkyl group as defined above where at least one of the carbon atoms of the ring is substituted with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, alkyl, alkoxy, alkenyl, alkynyl, aryl, heteroaryl, acyl, aldehyde, amino, carboxylic acid, ester, ether, halide, hydroxy, ketone, nitro, silyl, sulfo-oxo, sulfonyl, sulfone, sulfoxide, or thiol as described herein.

The term "cycloalkenyl" as used herein is a non-aromatic carbon-based ring composed of at least three carbon atoms and containing at least one double bound, *i.e.,* C=C. Examples of cycloalkenyl groups include, but are not limited to, cyclopropenyl, cyclobutenyl, cyclopentenyl, cyclopentadienyl, cyclohexenyl, cyclohexadienyl, and the like. The term "heterocycloalkenyl" is a type of cycloalkenyl group as defined above, and is included within the meaning of the term "cycloalkenyl," where at least one of the carbon atoms of the ring is substituted with a heteroatom such as, but not limited to, nitrogen, oxygen, sulfur, or phosphorus. The cycloalkenyl group and heterocycloalkenyl group can be substituted or unsubstituted. The cycloalkenyl group and heterocycloalkenyl group can be substituted with one or more groups including, but not limited to, alkyl, alkoxy, alkenyl, alkynyl, aryl, heteroaryl, acyl, aldehyde, amino, carboxylic acid, ester, ether, halide, hydroxy, ketone, nitro, silyl, sulfo-oxo, sulfonyl, sulfone, sulfoxide, or thiol as described herein.

The term "cyclic group" is used herein to refer to either aryl groups, non-aryl groups (*i.e.,* cycloalkyl, heterocycloalkyl, cycloalkenyl, and heterocycloalkenyl groups), or both. Cyclic groups have one or more ring systems that can be substituted or unsubstituted. A cyclic group can contain one or more aryl groups, one or more non-aryl groups, or one or more aryl groups and one or more non-aryl groups.

As used herein, "heteroaryl" refers to a monocyclic or polycyclic aromatic heterocycle having at least one heteroatom ring member selected from sulfur, oxygen, and nitrogen. In some embodiments, the heteroaryl ring has 1, 2, 3, or 4 heteroatom ring members independently selected from nitrogen, sulfur and oxygen. In some embodiments, any ring-forming N in a heteroaryl moiety can be an N-oxide. In some embodiments, the heteroaryl has 5-10 ring atoms and 1, 2, 3 or 4 heteroatom ring members independently selected from nitrogen, sulfur and oxygen. In some embodiments, the heteroaryl has 5-6 ring atoms and 1 or 2 heteroatom ring members independently selected from nitrogen, sulfur and oxygen. In some embodiments, the heteroaryl is a five-membered or six-membered heteroaryl ring. A five-membered heteroaryl ring is a heteroaryl with a ring having five ring atoms wherein one or more (e.g., 1, 2, or 3) ring atoms are independently selected from N, O, and S. Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4-oxadiazolyl. A six-membered heteroaryl ring is a heteroaryl with a ring having six ring atoms wherein one or more (e.g., 1, 2, or 3) ring atoms are independently selected from N, O, and S. Exemplary six-membered ring heteroaryls are pyridyl, pyrazinyl, pyrimidinyl, triazinyl and pyridazinyl.

As used herein, "heterocycloalkyl" refers to non-aromatic monocyclic or polycyclic heterocycles having one or more ring-forming heteroatoms selected from O, N, or S. Included in heterocycloalkyl are monocyclic 4-, 5-, 6-, and 7-membered heterocycloalkyl groups. Heterocycloalkyl groups can also include spirocycles. Example heterocycloalkyl groups include pyrrolidin-2-one, 1,3-isoxazolidin-2-one, pyranyl, tetrahydropuran, oxetanyl, azetidinyl, morpholino, thiomorpholino, piperazinyl, tetrahydrofuranyl, tetrahydrothienyl, piperidinyl, pyrrolidinyl, isoxazolidinyl, isothiazolidinyl, pyrazolidinyl, oxazolidinyl, thiazolidinyl, imidazolidinyl, azepanyl, benzazapene, and the like. Ring-forming carbon atoms and heteroatoms of a heterocycloalkyl group can be optionally substituted by oxo or sulfido (e.g., C(O), S(O), C(S), or S(O)₂, etc.). The heterocycloalkyl group can be attached through a ring-forming carbon atom or a ring-forming heteroatom. In some embodiments, the heterocycloalkyl group contains 0 to 3 double bonds. In some embodiments, the heterocycloalkyl group contains 0 to 2 double bonds. Also included in the definition of heterocycloalkyl are moieties that have one or more aromatic rings fused (*i.e.,* having a bond in common with) to the cycloalkyl ring, for example, benzo or thienyl derivatives of piperidine, morpholine, azepine, etc. A heterocycloalkyl group containing a fused aromatic ring can be attached through any ring-forming atom including a ring-forming atom of the fused aromatic ring. In some embodiments, the heterocycloalkyl has 4-10, 4-7 or 4-6 ring atoms with 1 or 2 heteroatoms independently selected from nitrogen, oxygen, or sulfur and having one or more oxidized ring members.

At certain places, the definitions or embodiments refer to specific rings (e.g., an azetidine ring, a pyridine ring, etc.). Unless otherwise indicated, these rings can be attached to any ring member provided that the valency of the atom is not exceeded. For example, an azetidine ring may be attached at any position of the ring, whereas a pyridin-3-yl ring is attached at the 3-position.

The term "acyl" as used herein is represented by the formula -C(O)Z¹ where Z¹ can be a hydrogen, hydroxyl, alkoxy, alkyl, halogenated alkyl, alkenyl, alkynyl, aryl, heteroaryl, cycloalkyl, cycloalkenyl, heterocycloalkyl, or heterocycloalkenyl group described above. As used herein, the term "acyl" can be used interchangeably with "carbonyl." Throughout this specification "C(O)" or "CO" is a short hand notation for C=O.

As used herein, the term "alkoxy" refers to a group of the formula Z¹-O-, where Z¹ is unsubstituted or substituted alkyl as defined above. Unless otherwise specified, alkoxy groups wherein Z¹ is a C₁-C₂₄ (e.g., C₁-C₂₂, C₁-C₂₀, C₁-C₁₈, C₁-C₁₆, C₁-C₁₄, C₁-C₁₂, C₁-C₁₀, C₁-C₈, C₁-C₆, C₁-C₄) alkyl group are intended. Examples include methoxy, ethoxy, propoxy, 1-methyl-ethoxy, butoxy, 1-methyl-propoxy, 2-methyl-propoxy, 1,1-dimethyl-ethoxy, pentoxy, 1-methyl-butyloxy, 2-methyl-butoxy, 3-methyl-butoxy, 2,2-di-methyl-propoxy, 1-ethyl-propoxy, hexoxy, 1,1-dimethyl-propoxy, 1,2-dimethyl-propoxy, 1-methyl-pentoxy, 2-methyl-pentoxy, 3-methyl-pentoxy, 4-methyl-penoxy, 1,1-dimethyl-butoxy, 1,2-dimethyl-butoxy, 1,3-dimethyl-butoxy, 2,2-dimethyl-butoxy, 2,3-dimethyl-butoxy, 3,3-dimethyl-butoxy, 1-ethyl-butoxy, 2-ethylbutoxy, 1,1,2-trimethyl-propoxy, 1,2,2-trimethyl-propoxy, 1-ethyl-1-methyl-propoxy, and 1-ethyl-2-methyl-propoxy.

The term "aldehyde" as used herein is represented by the formula -C(O)H.

The terms "amine" or "amino" as used herein are represented by the formula -NZ¹Z², where Z¹ and Z² can each be substitution group as described herein, such as hydrogen, an alkyl, halogenated alkyl, alkenyl, alkynyl, aryl, heteroaryl, cycloalkyl, cycloalkenyl, heterocycloalkyl, or heterocycloalkenyl group described above. "Amido" is -C(O)NZ¹Z².

The term "carboxylic acid" as used herein is represented by the formula -C(O)OH. A "carboxylate" or "carboxyl" group as used herein is represented by the formula -C(O)O⁻.

The term "ester" as used herein is represented by the formula -OC(O)Z¹ or -C(O)OZ¹, where Z¹ can be an alkyl, halogenated alkyl, alkenyl, alkynyl, aryl, heteroaryl, cycloalkyl, cycloalkenyl, heterocycloalkyl, or heterocycloalkenyl group described above.

The term "ether" as used herein is represented by the formula Z¹OZ², where Z¹ and Z² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "ketone" as used herein is represented by the formula Z¹C(O)Z², where Z¹ and Z² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "halide" or "halogen" or "halo" as used herein refers to fluorine, chlorine, bromine, and iodine.

The term "hydroxyl" as used herein is represented by the formula -OH.

The term "nitro" as used herein is represented by the formula -NO₂.

The term "silyl" as used herein is represented by the formula -SiZ¹Z²Z³, where Z¹, Z², and Z³ can be, independently, hydrogen, alkyl, halogenated alkyl, alkoxy, alkenyl, alkynyl, aryl, heteroaryl, cycloalkyl, cycloalkenyl, heterocycloalkyl, or heterocycloalkenyl group described above.

The term "sulfonyl" is used herein to refer to the sulfo-oxo group represented by the formula -S(O)₂Z¹, where Z¹ can be hydrogen, an alkyl, halogenated alkyl, alkenyl, alkynyl, aryl, heteroaryl, cycloalkyl, cycloalkenyl, heterocycloalkyl, or heterocycloalkenyl group described above.

The term "sulfonylamino" or "sulfonamide" as used herein is represented by the formula -S(O)₂NH-.

The term "thiol" as used herein is represented by the formula -SH.

The term "thio" as used herein is represented by the formula -S-.

As used herein, Me refers to a methyl group; OMe refers to a methoxy group; and i-Pr refers to an isopropyl group.

"R¹," "R²," "R³," "Rⁿ," etc., where n is some integer, as used herein can, independently, possess one or more of the groups listed above. For example, if R¹ is a straight chain alkyl group, one of the hydrogen atoms of the alkyl group can optionally be substituted with a hydroxyl group, an alkoxy group, an amine group, an alkyl group, a halide, and the like. Depending upon the groups that are selected, a first group can be incorporated within second group or, alternatively, the first group can be pendant (i.e., attached) to the second group. For example, with the phrase "an alkyl group comprising an amino group," the amino group can be incorporated within the backbone of the alkyl group. Alternatively, the amino group can be attached to the backbone of the alkyl group. The nature of the group(s) that is (are) selected will determine if the first group is embedded or attached to the second group.

Unless stated to the contrary, a formula with chemical bonds shown only as solid lines and not as wedges or dashed lines contemplates each possible stereoisomer or mixture of stereoisomer (e.g., each enantiomer, each diastereomer, each meso compound, a racemic mixture, or scalemic mixture).

Reference will now be made in detail to specific aspects of the disclosed materials, compounds, compositions, articles, and methods, examples of which are illustrated in the accompanying Examples and Figures.

### Carboranes and Carborane Analogs

Dicarba-closo-dodecaborane (also referred to herein as "carborane") is an icosahedral cluster containing two carbon atoms and ten boron atoms in which both atoms are hexacoordinated. In carboranes, depending on the position of the carbon atoms in the cluster, 3 kinds of isomers exist, i.e., 1,2-dicarba-closo-dodecaborane (ortho-carborane), 1,7-dicarba-closo-dodecaborane (meta-carborane), and 1,12-dicarba-closo-dodecaborane (para-carborane). These structures are unique among boron compounds, as they can have high thermal stabilities and hydrophobicities, for example, comparable to hydrocarbons.

Carboranes can be used, for example, in ¹⁰Boron-Neutron Capture Therapy (BNCT). BNCT has been developed as a therapy for glioma and melanoma. When ¹⁰B is irradiated with thermal neutron (slow neutron), and α ray with 2.4 MeV energy is emitted and the atom decomposed to ⁷Li and ⁴He. The range of α ray is about 10 µm, which corresponds to the diameter of cells Therefore, effects are expected that only cells in which ¹⁰B atoms are uptaken are destroyed and other cells are not damaged. For the development of BNCT, it is important to have cancer cells selectively uptake ¹⁰B atoms in a concentration capable of destroying cells with neutron radiation. For that purpose, other-carborane skeleton has been utilized which has been utilized which has low toxicity and a high ¹⁰B content, and is easy to be synthesized. Moreover, nucleic acid precursors, amino acids, and porphyrins which contain ortho-carboranes have been synthesized and subjected to evaluation.

Carborane-based ERβ agonists and carborane analogs are described, for example, in U.S. Patent No. 6,838,574 to Endo, U.S. Patent Application Publication No. 2018/0264017 to Tjarks et al., and PCT/US2019/064228 to Coss et al.,

In some embodiments, the carborane can be defined by Formula I below 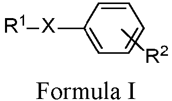 wherein
R¹ represents a dicarba-closo-dodecaboran-yl group which may have one or more substituents selected from the group consisting of an alkyl group, an alkenyl group, a carboxyl group, an alkoxycarbonyl group, an amino group, a hydroxyl group, a hydroxyalkyl group, a mono or di-alkylcarbamoyl-substituted alkyl group, an alkanoyl group, an aryl group, and an aralkyl group, each of which may be substituted or unsubstituted;
R² represents a carboxyl group, an alkoxycarbonyl group, or a hydroxyl group;
X represents a single bond, or a linking group selected from the group consisting of groups represented by the following formulas: 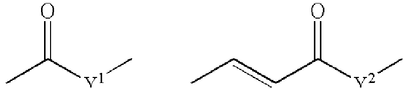 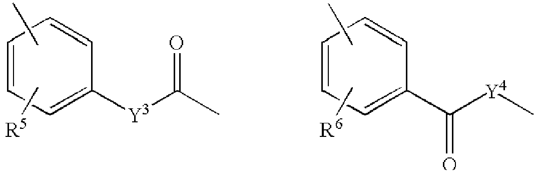 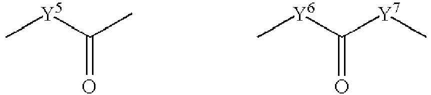 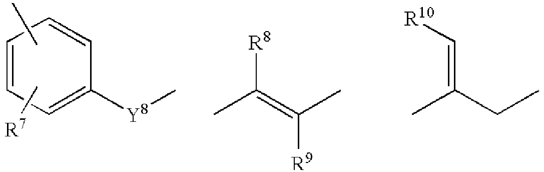 wherein Y¹, Y², Y³, Y⁴, Y⁵, Y⁶, and Y⁷ independently represent an oxygen atom or -N(R³)-wherein R³ represents hydrogen atom or an alkyl group; Y⁸ represents an oxygen atom,-N(R⁴)- wherein R⁴ represents hydrogen atom or an alkyl group, -CO-, -CH₂-, or-C(=CH²)-; R⁵, R⁶, and R⁷ independently represent hydrogen or one or more substituents on the phenyl group; R⁸ represents an alkyl group or an aryl group which may be substituted; R⁹ represents an alkyl group; and R¹⁰ represents a substituted or unsubstituted aryl group.

In some embodiments, the carborane can be defined by Formula II, or a pharmaceutically acceptable salts thereof: 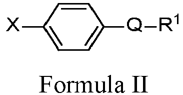 wherein
Q is a substituted or unsubstituted dicarba-closo-dodecaborane cluster, and 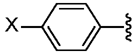 and R¹ are attached to Q in a para configuration;
X is OH, NHR², SH, or S(O)(O)NHR²;
R¹ is substituted or unsubstituted C₄-C₂₀ alkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, or NR³R⁴;
R² is H, OH, halogen, or substituted or unsubstituted C₁-C₄ alkyl;
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl;
with the proviso that when X is OH, R¹ is not (CH₂)₅CH(CH₃)₂ or NH₂.

In some examples of Formula II, the carborane cluster can include a heteroatom. In some examples of Formula II, the carborane cluster can include an isotopically labeled atom (i.e., a radiolabeled atom). In some examples of Formula II, the carborane cluster can include an isotopically labeled Boron atom (e.g., ¹⁰B).

In some examples of Formula II, Q can be: 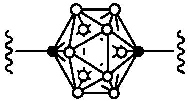 wherein
- is a carbon atom or a boron atom; and

∘ is C-H, C-halogen, C-alkyl, C-OH, C-NH₂, B-H, B-halogen, B-alkyl, B-OH, or B-NH₂.

In some examples of Formula II, X is OH.

In some examples of Formula II, R¹ is a substituted or unsubstituted C₆-C₁₀ alkyl. In some examples of Formula II, R¹ is a C₆-C₁₀ hydroxyalkyl. In some examples of Formula II, R¹ is a substituted or unsubstituted C₃-C₁₆ alkylaryl. In some examples of Formula II, R¹ is a C₃-C₁₆ hydroxyalkylaryl. In some examples of Formula II, R¹ is a substituted or unsubstituted C₅-C₁₀ acyl. In some examples of Formula II, R¹ is a substituted or unsubstituted branched C₄-C₁₀ alkyl. In some examples of Formula II, R¹ is a branched C₄-C₁₀ hydroxyalkyl.

In some examples of Formula II, the compounds can be of Formula III, or a pharmaceutically acceptable salt thereof: 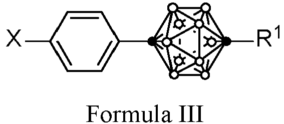 wherein
- is a carbon atom;

∘ is B-H, B-halogen, B-alkyl, B-OH, or B-NH₂;
X is OH, NHR², SH, or S(O)(O)NHR²;
R¹ is substituted or unsubstituted C₄-C₂₀ alkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, or NR³R⁴;
R² is H, OH, halogen, or substituted or unsubstituted C₁-C₄ alkyl; and
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl;
with the proviso that when X is OH, R¹ is not (CH₂)₅CH(CH₃)₂ or NH₂.

In some examples of Formula III, the carborane cluster can include a heteroatom.

In some examples of Formula III, the carborane cluster can include an isotopically labeled atom (i.e., a radiolabeled atom). In some examples of Formula III, the carborane cluster can include an isotopically labeled Boron atom (e.g., ¹⁰B).

In some examples of Formula III, X is OH.

In some examples of Formula III, R¹ is a substituted or unsubstituted C₆-C₁₀ alkyl. In some examples of Formula III, R¹ is a C₆-C₁₀ hydroxyalkyl. In some examples of Formula III, R¹ is a substituted or unsubstituted C₃-C₁₆ alkylaryl. In some examples of Formula III, R¹ is a C₃-C₁₆ hydroxyalkylaryl. In some examples of Formula III, R¹ is a substituted or unsubstituted C₅-C₁₀ acyl. In some examples of Formula III, R¹ is a substituted or unsubstituted branched C₄-C₁₀ alkyl. In some examples of Formula III, R¹ is a branched C₄-C₁₀ hydroxyalkyl.

In some examples of Formula III, the compounds can be of Formula IV, or a pharmaceutically acceptable salt thereof: 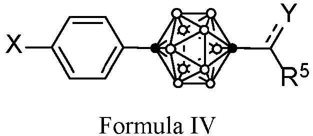 wherein
- is a carbon atom;

o is B-H, B-halogen, B-alkyl, B-OH, or B-NH₂;
the dotted line to Y indicates that the bond can be a single bond or a double bond, as valence permits;
X is OH, NHR², SH, or S(O)(O)NHR²;
Y is O, OR^{2'}, NHR², SH, or S(O)(O)NHR²;
R⁵ is substituted or unsubstituted C₂-C₁₉ alkyl, substituted or unsubstituted C₂-C₁₉ alkenyl, substituted or unsubstituted C₂-C₁₉ alkynyl, substituted or unsubstituted C₂-C₁₉ alkylaryl, substituted or unsubstituted C₂-C₁₉ alkylheteroaryl, substituted or unsubstituted C₃-C₁₉ alkylcycloalkyl, substituted or unsubstituted C₃-C₁₉ alkylheterocycloalkyl, or NR³R⁴;
R² is H, OH, halogen, or substituted or unsubstituted C₁-C₄ alkyl;
R^{2'} is H or substituted or unsubstituted C₁-C₄ alkyl; and
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl.

In some examples of Formula IV, the carborane cluster can include a heteroatom. In some examples of Formula IV, the carborane cluster can include an isotopically labeled atom (i.e., a radiolabeled atom). In some examples of Formula IV, the carborane cluster can include an isotopically labeled Boron atom (e.g., ¹⁰B).

In some examples of Formula IV, X is OH.

In some examples of Formula IV, Y is OH. In some examples of Formula IV, Y is O.

In some examples of Formula IV, R⁵ is a substituted or unsubstituted C₃-C₉ alkyl. In some examples of Formula IV, R⁵ is a substituted or unsubstituted C₆-C₉ alkyl. In some examples of Formula IV, R⁵ is a substituted or unsubstituted C₂-C₁₅ alkylaryl. In some examples of Formula IV, R⁵ is a substituted or unsubstituted branched C₂-C₉ alkyl.

Also disclosed herein are compounds of Formula V, and pharmaceutically acceptable salts thereof: 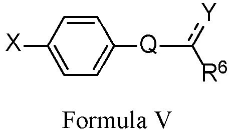 wherein
Q is a substituted or unsubstituted dicarba-closo-dodecaborane cluster, and 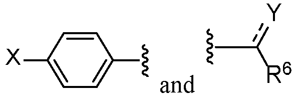 are attached to Q in a para configuration;
the dotted line to Y indicates that the bond can be a single bond or a double bond, as valence permits;
X is OH, NHR², SH, or S(O)(O)NHR²;
Y is O, OR^{2'}, NHR², SH, or S(O)(O)NHR²;
R⁶ is substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₂-C₂₀ alkylheteroaryl, substituted or unsubstituted C₄-C₂₀ alkylcycloalkyl, substituted or unsubstituted C₄-C₂₀ alkylheterocycloalkyl, or NR³R⁴;
R² is H, OH, halogen, or substituted or unsubstituted C₁-C₄ alkyl;
R^{2'} is H or substituted or unsubstituted C₁-C₄ alkyl; and
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl;
with the proviso that when X is OH, R⁶ is not CH₂OH, CH(CH₃)OH, CH₂CH₂OH, CH₂CH₂CH₂OH, (CH₂)₅CH(CH₃)₂, or NH₂.

In some examples of Formula V, the carborane cluster can include a heteroatom. In some examples of Formula V, the carborane cluster can include an isotopically labeled atom (i.e., a radiolabeled atom). In some examples of Formula V, the carborane cluster can include an isotopically labeled Boron atom (e.g., ¹⁰B).

In some examples of Formula V, Q can be 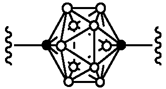 wherein
- is a carbon atom or a boron atom; and

∘ is C-H, C-halogen, C-alkyl, C-OH, C-NH₂, B-H, B-halogen, B-alkyl, B-OH, or B-NH₂.

In some examples of Formula V, X is OH.

In some examples of Formula V, Y is OH. In some examples of Formula V, Y is O.

In some examples of Formula V, R⁶ is a substituted or unsubstituted C₆-C₁₀ alkyl. In some examples of Formula V, R⁶ is a substituted or unsubstituted C₂-C₁₅ alkylaryl. In some examples of Formula V, R⁶ is a substituted or unsubstituted branched C₃-C₁₀ alkyl.

In some examples of Formula V, the compounds can be of Formula VI, or a pharmaceutically acceptable salt thereof: 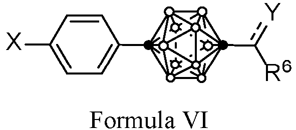 wherein
- is a carbon atom;

o is B-H, B-halogen, B-alkyl, B-OH, or B-NH₂;
the dotted line to Y indicates that the bond can be a single bond or a double bond, as valence permits;
X is OH, NHR², SH, or S(O)(O)NHR²;
Y is O, OR^{2'}, NHR², SH, or S(O)(O)NHR²;
R⁶ is substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₂-C₂₀ alkylheteroaryl, substituted or unsubstituted C₄-C₂₀ alkylcycloalkyl, substituted or unsubstituted C₄-C₂₀ alkylheterocycloalkyl, or NR³R⁴;
R² is H, OH, halogen, or substituted or unsubstituted C₁-C₄ alkyl;
R^{2'} is H or substituted or unsubstituted C₁-C₄ alkyl; and
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl;
with the proviso that when X is OH, R⁶ is not CH₂OH, CH(CH₃)OH, CH₂CH₂OH, CH₂CH₂CH₂OH, (CH₂)₅CH(CH₃)₂, or NH₂.

In some examples of Formula VI, the carborane cluster can include a heteroatom. In some examples of Formula VI, the carborane cluster can include an isotopically labeled atom (i.e., a radiolabeled atom). In some examples of Formula VI, the carborane cluster can include an isotopically labeled Boron atom (e.g., ¹⁰B).

In some examples of Formula VI, X is OH.

In some examples of Formula VI, Y is OH. In some examples of Formula VI, Y is O.

In some examples of Formula VI, R⁶ is a substituted or unsubstituted C₆-C₁₀ alkyl. In some examples of Formula VI, R⁶ is a substituted or unsubstituted C₂-C₁₅ alkylaryl. In some examples of Formula VI, R⁶ is a substituted or unsubstituted branched C₃-C₁₀ alkyl.

Also disclosed herein are compounds of Formula VII, and pharmaceutically acceptable salts thereof: 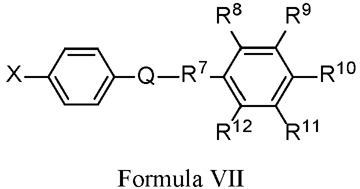 wherein
Q is a substituted or unsubstituted dicarba-closo-dodecaborane cluster, and 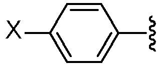 and R⁷ are attached to Q in a para configuration;
X is OH, NHR², SH, or S(O)(O)NHR²;
R⁷ is substituted or unsubstituted C₁-C₁₄ alkyl, substituted or unsubstituted C₂-C₁₄ alkenyl, substituted or unsubstituted C₂-C₁₄ alkynyl, substituted or unsubstituted C₁-C₁₄ acyl, or NR³R⁴;
R⁸, R⁹, R¹⁰, R¹¹, and R¹² are independently H, OH, halogen, substituted or unsubstituted C₁-C₂₀ alkyl, sub substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, substituted or unsubstituted C₁-C₂₀ acyl, or NR³R⁴, or wherein, as valence permits, R⁸ and R⁹, R⁹ and R¹⁰, R¹⁰ and R¹¹, or R¹¹ and R¹², together with the atoms to which they are attached, form a 3-10 membered substituted or unsubstituted cyclic moiety optionally including from 1 to 3 heteroatoms;
R² is H, OH, halogen, or substituted or unsubstituted C₁-C₄ alkyl; and
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl.

In some examples of Formula VII, the carborane cluster can include a heteroatom. In some examples of Formula VII, the carborane cluster can include an isotopically labeled atom (i.e., a radio labeled atom). In some examples of Formula VII, the carborane cluster can include an isotopically labeled Boron atom (e.g., ¹⁰B).

In some examples of Formula VII, Q can be 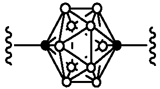 wherein
- is a carbon atom or a boron atom; and

∘ is C-H, C-halogen, C-alkyl, C-OH, C-NH₂, B-H, B-halogen, B-alkyl, B-OH, or B-NH₂.

In some examples of Formula VII, X is OH.

In some examples of Formula VII, R⁷ is a substituted or unsubstituted C₁-C₇ alkyl. In some examples of Formula VII, R⁷ is a C₁-C₇ hydroxyalkyl.

In some examples of Formula VII, R⁸-R¹² are independently H, OH, halogen, or substituted or unsubstituted C₁-C₄ alkyl, or wherein, as valence permits, R⁸ and R⁹, R⁹ and R¹⁰, R¹⁰ and R¹¹, or R¹¹ and R¹², together with the atoms to which they are attached, form a 3-10 membered substituted or unsubstituted cyclic moiety optionally including from 1 to 3 heteroatoms. In some examples of Formula VII, R⁸-R¹² are each H. In some examples of Formula VII, R⁸, R¹⁰, and R¹² are each H, and R⁹ and R¹⁰, together with the atoms to which they are attached, form a substituted or unsubstituted 5-7 membered cyclic moiety.

In some examples of Formula VII, the compounds can be of Formula VIII, or a pharmaceutically acceptable salt thereof: 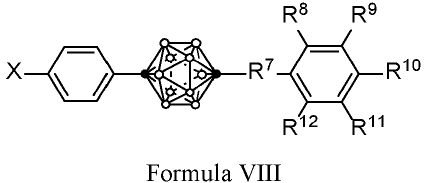 wherein
- is a carbon atom;

o is B-H, B-halogen, B-alkyl, B-OH, or B-NH₂;
X is OH, NHR², SH, or S(O)(O)NHR²;
R⁷ is substituted or unsubstituted C₁-C₁₄ alkyl, substituted or unsubstituted C₂-C₁₄ alkenyl, substituted or unsubstituted C₂-C₁₄ alkynyl, substituted or unsubstituted C₁-C₁₄ acyl, or NR³R⁴;
R⁸, R⁹, R¹⁰, R¹¹, and R¹² are independently H, OH, halogen, substituted or unsubstituted C₁-C₂₀ alkyl, sub substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, substituted or unsubstituted C₁-C₂₀ acyl, or NR³R⁴, or wherein, as valence permits, R⁸ and R⁹, R⁹ and R¹⁰, R¹⁰ and R¹¹, or R¹¹ and R¹², together with the atoms to which they are attached, form a 3-10 membered substituted or unsubstituted cyclic moiety optionally including from 1 to 3 heteroatoms;
R² is H, OH, halogen, or substituted or unsubstituted C₁-C₄ alkyl; and
R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, or substituted or unsubstituted C₁-C₂₀ acyl.

In some examples of Formula VIII, the carborane cluster can include a heteroatom. In some examples of Formula VIII, the carborane cluster can include an isotopically labeled atom (i.e., a radiolabeled atom). In some examples of Formula VIII, the carborane cluster can include an isotopically labeled Boron atom (e.g., ¹⁰B).

In some examples of Formula VIII, X is OH.

In some examples of Formula VIII, R⁷ is a substituted or unsubstituted C₁-C₇ alkyl. In some examples of Formula VIII, R⁷ is a C₁-C₇ hydroxyalkyl.

In some examples of Formula VIII, R⁸-R¹² are independently H, OH, halogen, or substituted or unsubstituted C₁-C₄ alkyl, or wherein, as valence permits, R⁸ and R⁹, R⁹ and R¹⁰, R¹⁰ and R¹¹, or R¹¹ and R¹², together with the atoms to which they are attached, form a 3-10 membered substituted or unsubstituted cyclic moiety optionally including from 1 to 3 heteroatoms. In some examples of Formula VIII, R⁸-R¹² are each H. In some examples of Formula VIII, R⁸, R¹⁰, and R¹² are each H, and R⁹ and R¹⁰, together with the atoms to which they are attached, form a substituted or unsubstituted 5-7 membered cyclic moiety.

Also disclosed herein are compounds of Formula IX, and pharmaceutically acceptable salts thereof: 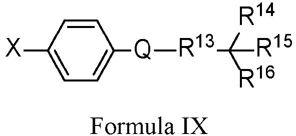 wherein
Q is a substituted or unsubstituted dicarba-closo-dodecaborane cluster, and 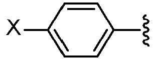 and R¹³ are attached to Q in a para configuration;
X is OH, NHR², SH, or S(O)(O)NHR²;
R¹³ is substituted or unsubstituted C₁-C₁₉ alkyl, substituted or unsubstituted C₂-C₁₉ alkenyl, substituted or unsubstituted C₂-C₁₉ alkynyl, or substituted or unsubstituted C₁-C₂₀ acyl; and
R¹⁴, R¹⁵, and R¹⁶ are independently hydrogen, halogen, hydroxyl, substituted or unsubstituted C₁-C₁₈ alkyl, substituted or unsubstituted C₂-C₁₈ alkenyl, substituted or unsubstituted C₁-C₁₈ alkynyl, substituted or unsubstituted C₂-C₁₈ aryl, substituted or unsubstituted C₃-C₁₈ cycloalkyl, substituted or unsubstituted C₁-C₂₀ acyl, or NR³R⁴, or wherein, as valence permits, R¹⁴ and R¹⁵, R¹⁴ and R¹⁶, or R¹⁵ and R¹⁶, together with the atoms to which they are attached, for a 3-10 membered substituted or unsubstituted cyclic moiety optionally including from 1 to 3 heteroatoms,
with the proviso that at least two of R¹⁴, R¹⁵ and R¹⁶ are not hydrogen, halogen, or hydroxyl; and
with the proviso that when X is OH and R¹³ is a C₅ alkyl, R¹⁴, R¹⁵, and R¹⁶ are not H, methyl, and methyl.

In some examples of Formula IX, the carborane cluster can include a heteroatom. In some examples of Formula IX, the carborane cluster can include an isotopically labeled atom (i.e., a radio labeled atom). In some examples of Formula IX, the carborane cluster can include an isotopically labeled Boron atom (e.g., ¹⁰B). In some examples of Formula IX, Q is 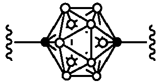 wherein
- is a carbon atom or a boron atom; and

∘ is C-H, C-halogen, C-alkyl, C-OH, C-NH₂, B-H, B-halogen, B-alkyl, B-OH, or B-NH₂.

In some examples of Formula IX, X is OH.

In some examples of Formula IX, R¹³ is a substituted or unsubstituted C₄-C₈ alkyl. In some examples of Formula IX, R¹³ is a C₄-C₈ hydroxyalkyl.

In some examples of Formula IX, R¹⁴-R¹⁶ are independently hydrogen, halogen, hydroxyl, substituted or unsubstituted C₁-C₄ alkyl, with the proviso that at least two of R¹⁴, R¹⁵ and R¹⁶ are not hydrogen, halogen, or hydroxyl; and with the proviso that when X is OH and R¹³ is a C₅ alkyl, R¹⁴, R¹⁵, and R¹⁶ are not H, methyl, and methyl.

In some examples of Formula IX, the compounds can be of Formula X, or a pharmaceutically acceptable salt thereof: 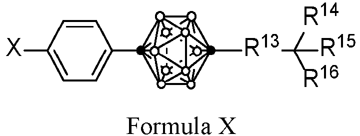 wherein
- is a carbon atom;

o is B-H, B-halogen, B-alkyl, B-OH, or B-NH₂;
X is OH, NHR², SH, or S(O)(O)NHR²;
R¹³ is substituted or unsubstituted C₁-C₁₉ alkyl, substituted or unsubstituted C₂-C₁₉ alkenyl, substituted or unsubstituted C₂-C₁₉ alkynyl, or substituted or unsubstituted C₁-C₂₀ acyl; and
R¹⁴, R¹⁵, and R¹⁶ are independently hydrogen, halogen, hydroxyl, substituted or unsubstituted C₁-C₁₈ alkyl, substituted or unsubstituted C₂-C₁₈ alkenyl, substituted or unsubstituted C₁-C₁₈ alkynyl, substituted or unsubstituted C₂-C₁₈ aryl, substituted or unsubstituted C₃-C₁₈ cycloalkyl, substituted or unsubstituted C₁-C₂₀ acyl, or NR³R⁴, or wherein, as valence permits, R¹⁴ and R¹⁵, R¹⁴ and R¹⁶, or R¹⁵ and R¹⁶, together with the atoms to which they are attached, for a 3-10 membered substituted or unsubstituted cyclic moiety optionally including from 1 to 3 heteroatoms,
with the proviso that at least two of R¹⁴, R¹⁵ and R¹⁶ are not hydrogen, halogen, or hydroxyl; and
with the proviso that when X is OH and R¹³ is a C₅ alkyl, R¹⁴, R¹⁵, and R¹⁶ are not H, methyl, and methyl.

In some examples of Formula X, the carborane cluster can include a heteroatom. In some examples of Formula X, the carborane cluster can include an isotopically labeled atom (i.e., a radio labeled atom). In some examples of Formula X, the carborane cluster can include an isotopically labeled Boron atom (e.g., ¹⁰B).

In some examples of Formula X, X is OH.

In some examples of Formula X, R¹³ is a substituted or unsubstituted C₄-C₈ alkyl. In some examples of Formula X, R¹³ is a C₄-C₈ hydroxyalkyl.

In some examples of Formula X, R¹⁴-R¹⁶ are independently hydrogen, halogen, hydroxyl, substituted or unsubstituted C₁-C₄ alkyl, with the proviso that at least two of R¹⁴, R¹⁵ and R¹⁶ are not hydrogen, halogen, or hydroxyl; and with the proviso that when X is OH and R¹³ is a C₅ alkyl, R¹⁴, R¹⁵, and R¹⁶ are not H, methyl, and methyl.

In some examples, the compounds can be selected from the group consisting of: 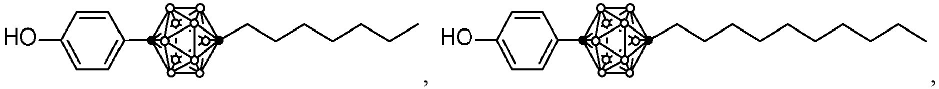 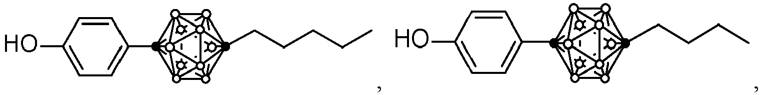 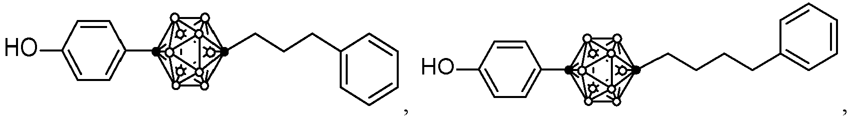 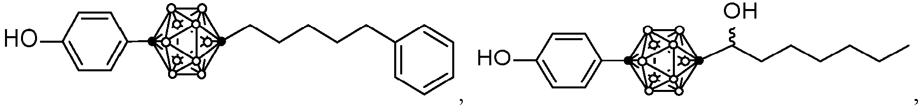 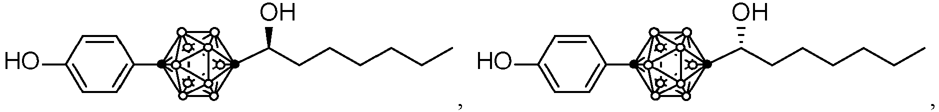 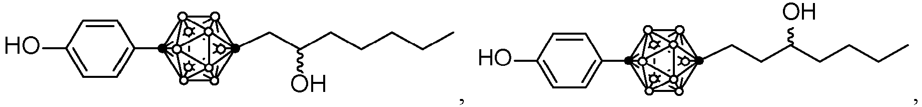 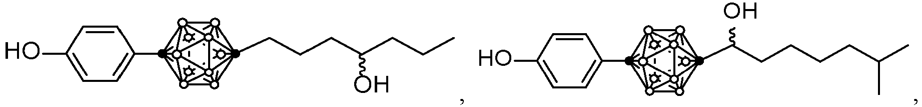 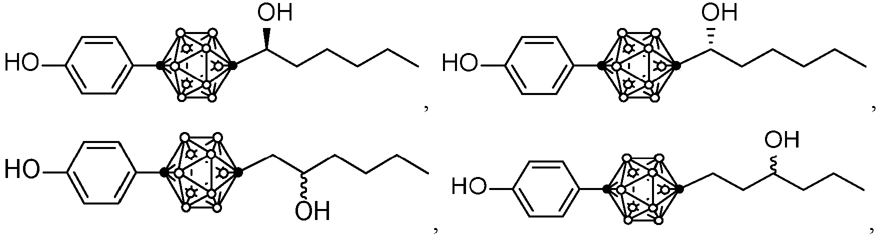 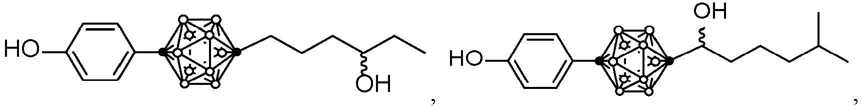 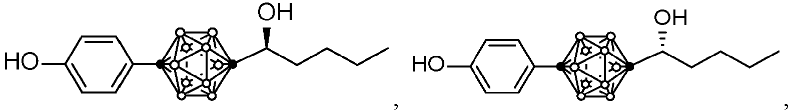 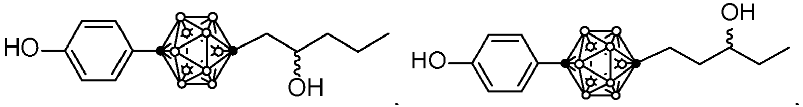 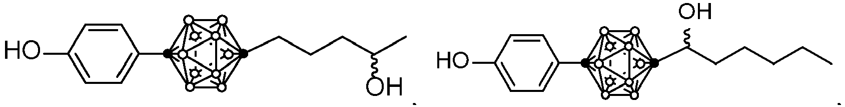 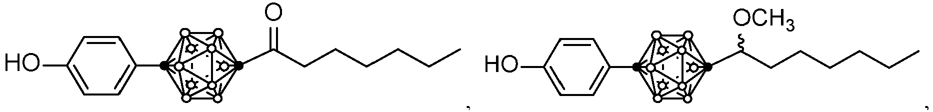 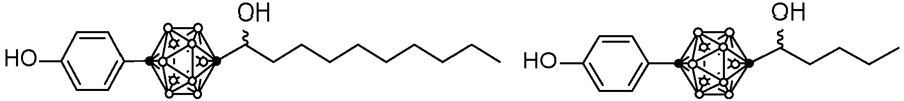 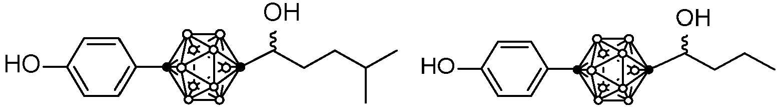 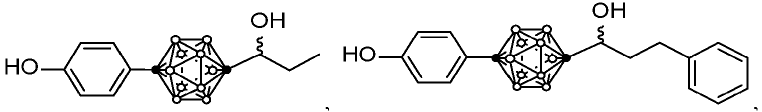 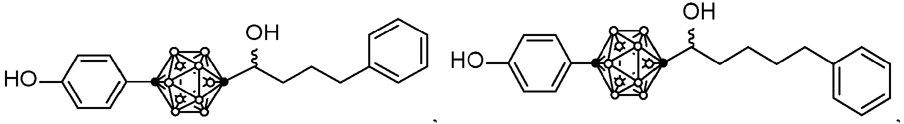 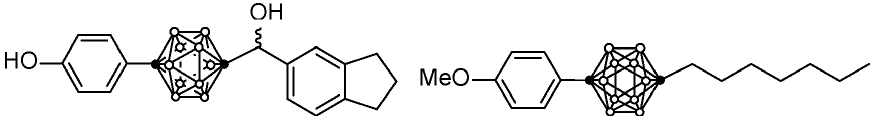 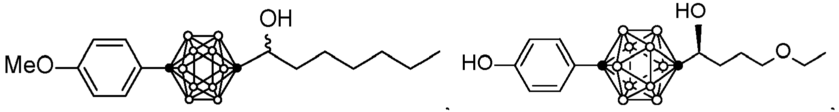 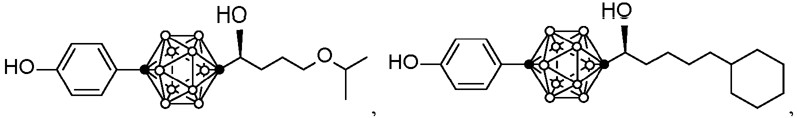 and pharmaceutically acceptable salts thereof. Note that only the carboranes recited in claim 1 form part of the present invention. In some examples, the carborane cluster can include a heteroatom.

Also disclosed herein are compounds of Formula XI, and pharmaceutically acceptable salts thereof: 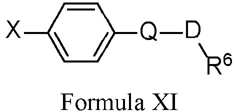 wherein
Q is a substituted or unsubstituted dicarba-closo-dodecaborane cluster;
D is -S-, -S(O)-, -S(O)(O)-, -S(O)(NH)-, -P(O)(OH)O-, -P(O)(OH)NH-, or -O-;
X is OH, NHR², SH, or S(O)(O)NHR²;
R⁶ is substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₂-C₂₀ alkylheteroaryl, substituted or unsubstituted C₄-C₂₀ alkylcycloalkyl, or substituted or unsubstituted C₄-C₂₀ alkylheterocycloalkyl; and
R² is H, OH, halogen, or substituted or unsubstituted C₁-C₄ alkyl.

In some examples of Formula XI, 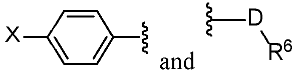 are attached to Q in a para configuration.

In some examples of Formula XI, the carborane cluster can include a heteroatom. In some examples of Formula XI, the carborane cluster can include an isotopically labeled atom (i.e., a radiolabeled atom). In some examples of Formula XI, the carborane cluster can include an isotopically labeled Boron atom (e.g., ¹⁰B).

In some examples of Formula XI, Q can be 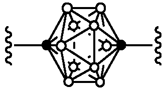 wherein
- is a carbon atom or a boron atom; and

∘ is C-H, C-halogen, C-alkyl, C-OH, C-NH₂, B-H, B-halogen, B-alkyl, B-OH, or B-NH₂.

In some examples of Formula XI, X is OH.

In some examples of Formula XI, R⁶ is a substituted or unsubstituted C₆-C₁₀ alkyl. In some examples of Formula XI, R⁶ is a substituted or unsubstituted C₂-C₁₅ alkylaryl. In some examples of Formula XI, R⁶ is a substituted or unsubstituted branched C₃-C₁₀ alkyl.

In some examples, the compounds (not claimed) can be selected from the group consisting of: 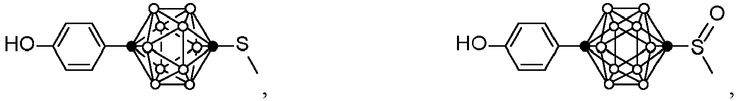 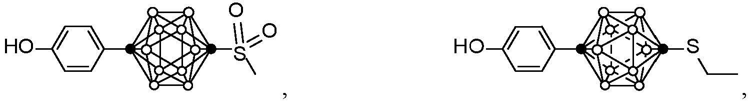 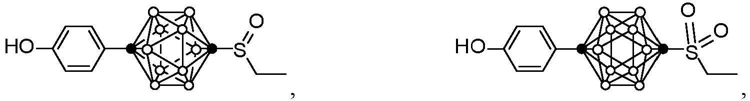 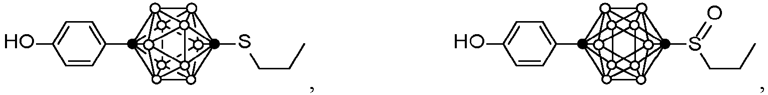 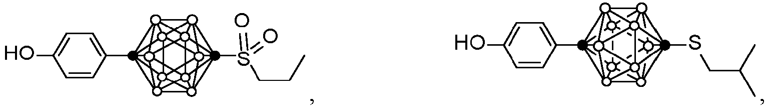 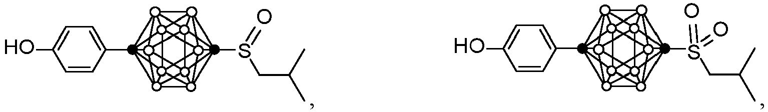 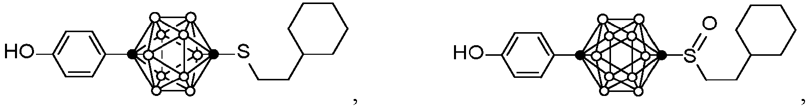 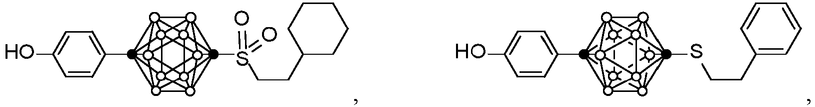 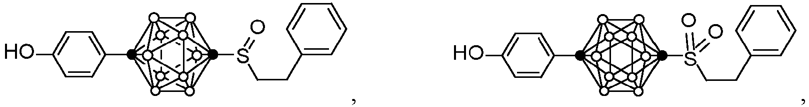 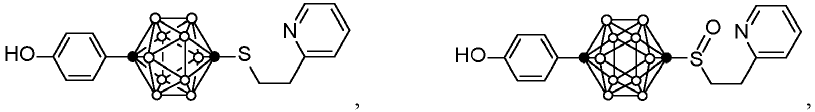 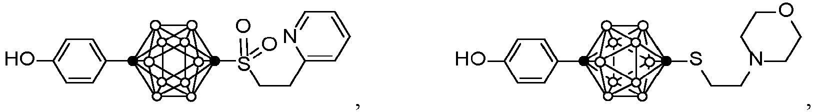 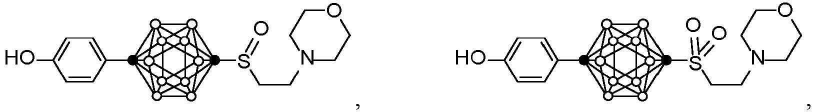 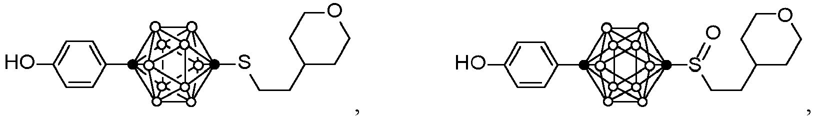 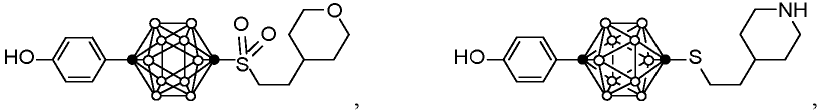 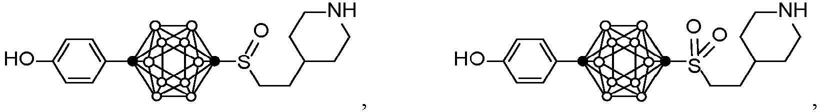 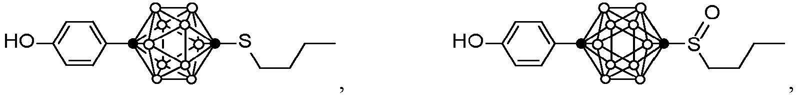 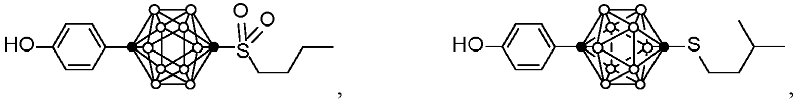 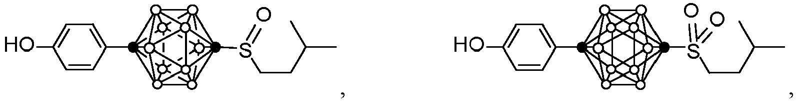 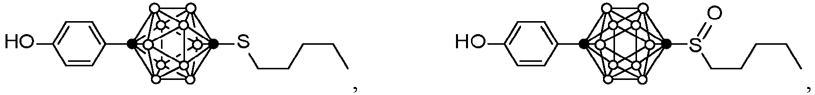 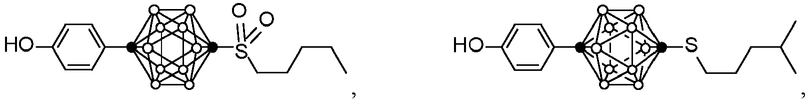 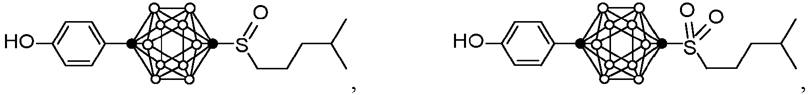 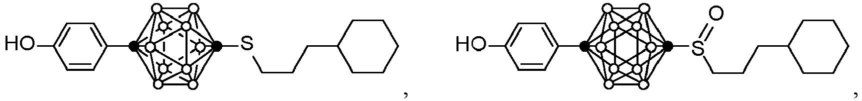 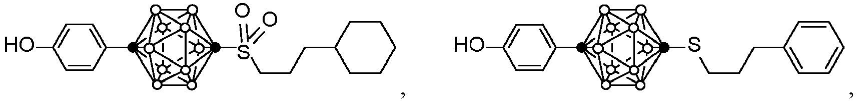 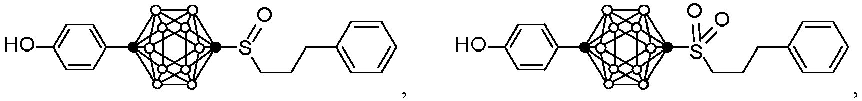 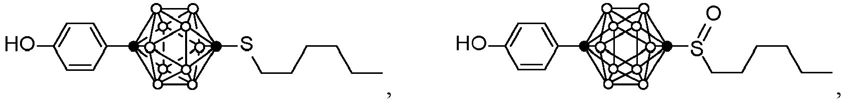 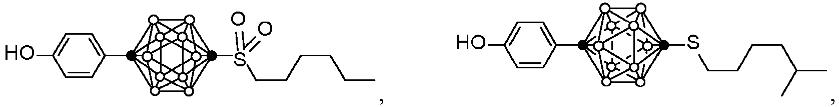 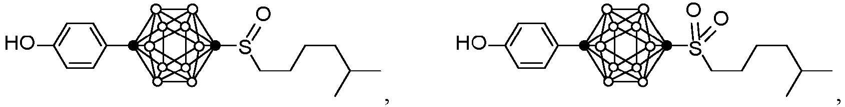 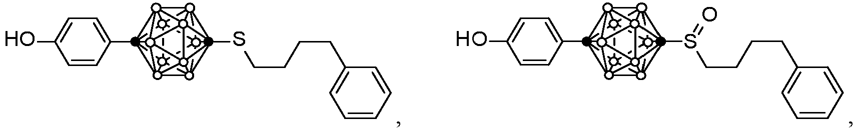 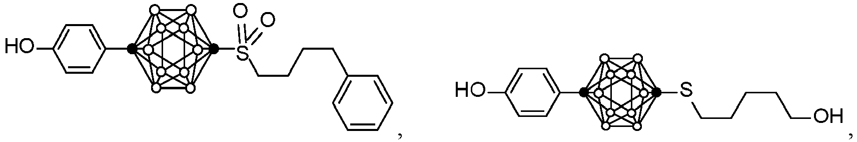 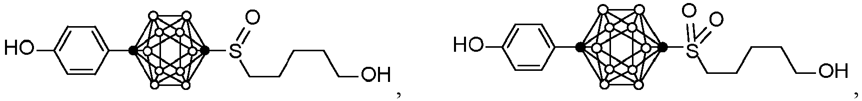 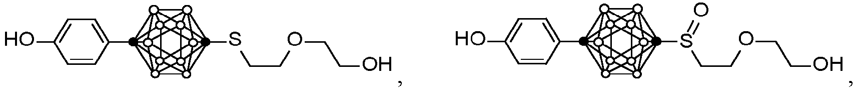 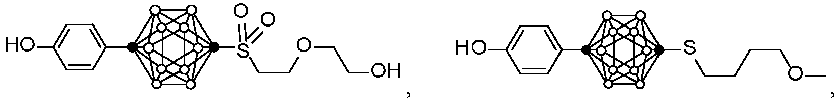 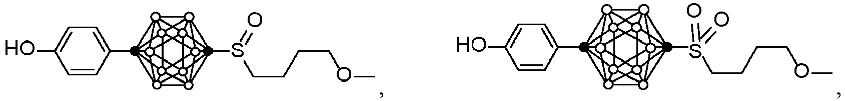 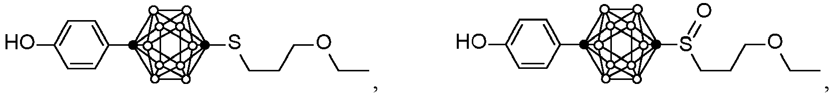 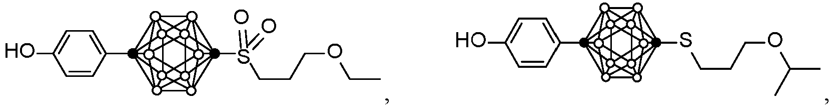 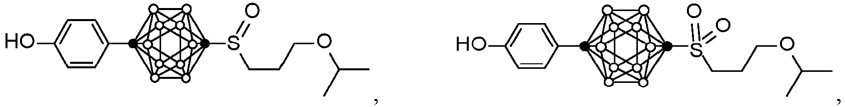 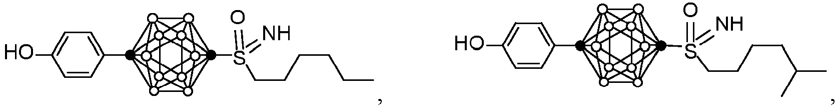 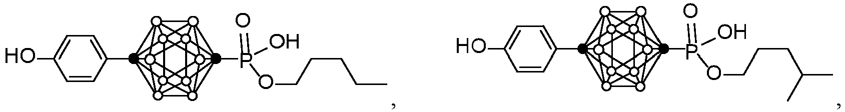 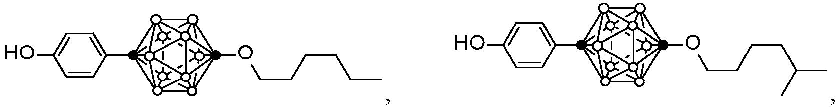 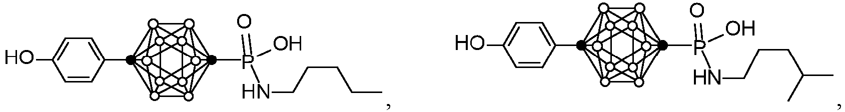 and pharmaceutically acceptable salts thereof. In some examples, the carborane cluster can include a heteroatom.

In some embodiments, the carborane can be defined by Formula XII, or a pharmaceutically acceptable salt thereof:

A-Q-R¹ Formula XII

wherein Q is a substituted or unsubstituted dicarba-closo-dodecaborane cluster, and A and R¹ are attached to Q in a para configuration; A is a substituted or unsubstituted heteroaryl ring; R¹ is substituted or unsubstituted C₂-C₂₀ alkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, C₁-C₂₀ acyl, -C(O)N R³R⁴, -S(O)-R³, -S(O₂)-R³, substituted or unsubstituted C₂-C₂₀ heteroalkyl, or NR³R⁴; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some embodiments, Q is 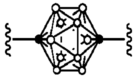 wherein **•** is a carbon atom or a boron atom; and o is C-H, C-halogen, C-alkyl, C-OH, C-NH₂, B-H, B-halogen, B-alkyl, B-OH, or B-NH₂.

In some embodiments, A can be a five-membered substituted or unsubstituted heteroaryl ring. For example, A can comprise a thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, or 1,3,4-oxadiazolyl ring. In some embodiments, A can be a six-membered substituted or unsubstituted heteroaryl ring. For example, A can comprise a pyridyl, pyrazinyl, pyrimidinyl, triazinyl, or pyridazinyl ring.

In some cases, the compound can be defined by Formula XIIA, or a pharmaceutically acceptable salt thereof: 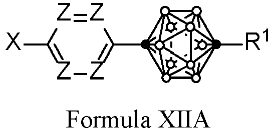 wherein **•** is a carbon atom; o is B-H, B-halogen, B-alkyl, B-OH, or B-NH₂; X is OH, NHR², SH, or S(O)(O)NHR²; Z is, individually for each occurrence, N or CH, with the proviso that at least one of Z is N; R¹ is substituted or unsubstituted C₂-C₂₀ alkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, C₁-C₂₀ acyl, -C(O)N R³R⁴, -S(O)-R³, -S(O₂)-R³, substituted or unsubstituted C₂-C₂₀ heteroalkyl, or NR³R⁴; R² is H, OH, halogen, or substituted or unsubstituted C₁-C₄ alkyl; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some cases, one of Z can be N. In some cases, two or more of Z can be N. In some cases, three of Z can be N.

In some embodiments, the compound can be defined by one of the formulae below, or a pharmaceutically acceptable salt thereof: 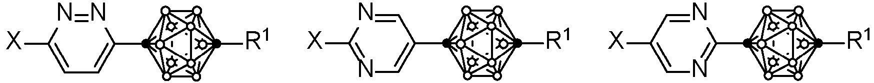 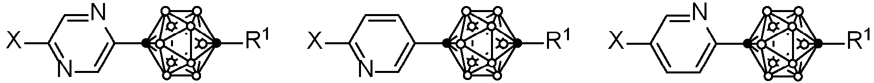 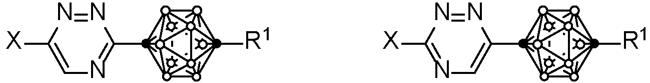 wherein **•** is a carbon atom; o is B-H, B-halogen, B-alkyl, B-OH, or B-NH₂; X is OH, NHR², SH, or S(O)(O)NHR²; R¹ is substituted or unsubstituted C₂-C₂₀ alkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, C₁-C₂₀ acyl, -C(O)N R³R⁴, -S(O)-R³, -S(O₂)-R³, substituted or unsubstituted C₂-C₂₀ heteroalkyl, or NR³R⁴; R² is H, OH, halogen, or substituted or unsubstituted C₁-C₄ alkyl; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some embodiments, the compound can be defined by one of Formula XIIB-XIIF, or a pharmaceutically acceptable salt thereof: 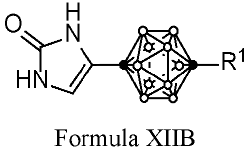 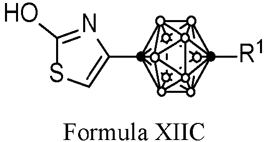 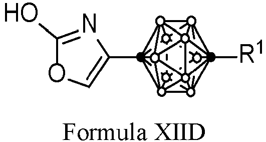 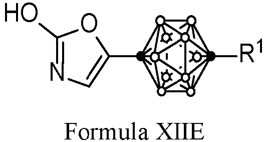 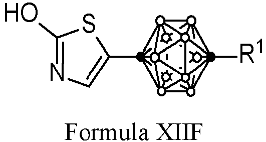 wherein • is a carbon atom; o is B-H, B-halogen, B-alkyl, B-OH, or B-NH₂; R¹ is substituted or unsubstituted C₂-C₂₀ alkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, C₁-C₂₀ acyl, -C(O)N R³R⁴, -S(O)-R³, -S(O₂)-R³, substituted or unsubstituted C₂-C₂₀ heteroalkyl, or NR³R⁴; R² is H, OH, halogen, or substituted or unsubstituted C₁-C₄ alkyl; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some of the embodiments above, X can be OH.

In some of the embodiments above, R¹ can be a substituted or unsubstituted C₆-C₁₀ alkyl (e.g., a C₆-C₁₀ hydroxyalkyl).

In some of the embodiments above, R¹ can be a substituted or unsubstituted C₃-C₁₆ alkylaryl (e.g., a C₃-C₁₆ hydroxyalkylaryl).

In some of the embodiments above, R¹ can be a substituted or unsubstituted C₈-C₂₀ alkylaryl (e.g., a C₈-C₂₀ hydroxyalkylaryl).

In some of the embodiments above, R¹ can be a substituted or unsubstituted C₅-C₁₀ acyl.

In some of the embodiments above, R¹ can be a substituted or unsubstituted branched C₄-C₁₀ alkyl (e.g., a branched C₄-C₁₀ hydroxyalkyl).

In some embodiments, the compound is defined by a formula below, or a pharmaceutically acceptable salt thereof: 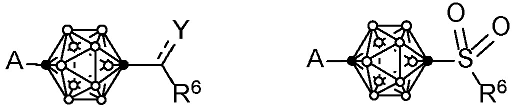 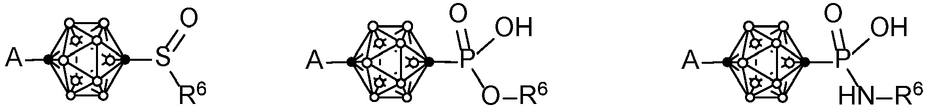 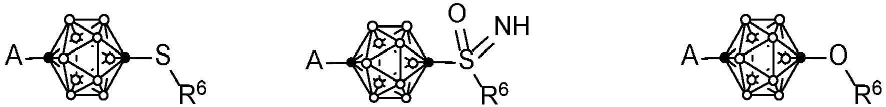 wherein • is a carbon atom; o is B-H, B-halogen, B-alkyl, B-OH, or B-NH₂; the dotted line to Y indicates that the bond can be a single bond or a double bond, as valence permits; A is a substituted or unsubstituted heteroaryl ring; Y, when present, is O, halogen, OR^{2'}, NHR², SH, or S(O)(O)NHR²; R⁶ is substituted or unsubstituted C₁-C₁₉ alkyl, substituted or unsubstituted C₂-C₁₉ alkenyl, substituted or unsubstituted C₂-C₁₉ alkynyl, substituted or unsubstituted C₂-C₁₉ alkylaryl, substituted or unsubstituted C₂-C₁₉ alkylheteroaryl, substituted or unsubstituted C₄-C₁₉ alkylcycloalkyl, substituted or unsubstituted C₄-C₁₉ alkylheterocycloalkyl, and substituted or unsubstituted C₂-C₂₀ heteroalkyl. or NR³R⁴; R² is H, OH, halogen, or substituted or unsubstituted C₁-C₄ alkyl; R^{2'} is H or substituted or unsubstituted C₁-C₄ alkyl; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some embodiments, A can be a five-membered substituted or unsubstituted heteroaryl ring. For example, A can comprise a thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, or 1,3,4-oxadiazolyl ring. In some embodiments, A can be a six-membered substituted or unsubstituted heteroaryl ring. For example, A can comprise a pyridyl, pyrazinyl, pyrimidinyl, triazinyl, or pyridazinyl ring.

In some of these embodiments, Y is OH. In some of these embodiments, Y is F. In some of these embodiments, Y is O.

In some examples, R⁶ can be a substituted or unsubstituted C₃-C₁₀ alkyl, such as a substituted or unsubstituted C₆-C₉ alkyl.

In some examples, R⁶ can be a substituted or unsubstituted C₂-C₁₅ alkylaryl.

In some examples, R⁶ can be a substituted or unsubstituted branched C₂-C₉ alkyl.

In some examples, R⁶ can be a substituted or unsubstituted C₃-C₁₀ heteroalkyl, such as a substituted or unsubstituted C₆-C₉ heteroalkyl.

Also provided are compounds defined by Formula XIII, or a pharmaceutically acceptable salt thereof:

A-Q-R¹ Formula XIII

wherein Q is a substituted or unsubstituted dicarba-closo-dodecaborane cluster, and A and R¹ are attached to Q in a para configuration; A is a substituted or unsubstituted aryl ring or a substituted or unsubstituted heteroaryl ring; R¹ is substituted or unsubstituted C₂-C₂₀ heteroalkyl, -C(O)N R³R⁴, -S(O)-R³, -S(O₂)-R³, or NR³R⁴; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₂-C₂₀ alkylheteroaryl, substituted or unsubstituted C₄-C₂₀ alkylcycloalkyl, substituted or unsubstituted C₄-C₂₀ alkylheterocycloalkyl, and substituted or unsubstituted C₂-C₂₀ heteroalkyl, with the proviso that when present, at least one of R³ and R⁴ is C₂-C₂₀ heteroalkyl.

In some embodiments, A can comprise a substituted or unsubstituted aryl ring (e.g., a substituted or unsubstituted phenyl ring). In some embodiments, A can be a five-membered substituted or unsubstituted heteroaryl ring. For example, A can comprise a thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, or 1,3,4-oxadiazolyl ring. In some embodiments, A can be a six-membered substituted or unsubstituted heteroaryl ring. For example, A can comprise a pyridyl, pyrazinyl, pyrimidinyl, triazinyl, or pyridazinyl ring.

In some embodiments, Q is 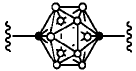 wherein • is a carbon atom or a boron atom; and o is C-H, C-halogen, C-alkyl, C-OH, C-NH₂, B-H, B-halogen, B-alkyl, B-OH, or B-NH₂.

In some embodiments, the compound can be defined by Formula XIIIA, or a pharmaceutically acceptable salt thereof: 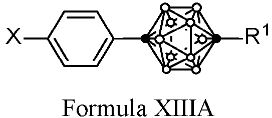 wherein • is a carbon atom; o is B-H, B-halogen, B-alkyl, B-OH, or B-NH₂; X is OH, NHR², SH, or S(O)(O)NHR²; Z is, individually for each occurrence, N or CH, with the proviso that at least one of Z is N; R¹ is substituted or unsubstituted C₂-C₂₀ heteroalkyl, -C(O)N R³R⁴, - S(O)-R³, -S(O₂)-R³, or NR³R⁴; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₂-C₂₀ alkylheteroaryl, substituted or unsubstituted C₄-C₂₀ alkylcycloalkyl, substituted or unsubstituted C₄-C₂₀ alkylheterocycloalkyl, and substituted or unsubstituted C₂-C₂₀ heteroalkyl, with the proviso that when present, at least one of R³ and R⁴ is C₂-C₂₀ heteroalkyl.

In some of these embodiments, X can be OH.

Also provided are compounds defined by any of the formula below, or a pharmaceutically acceptable salt thereof: 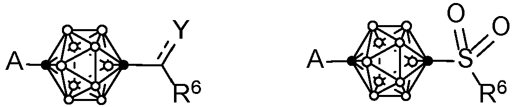 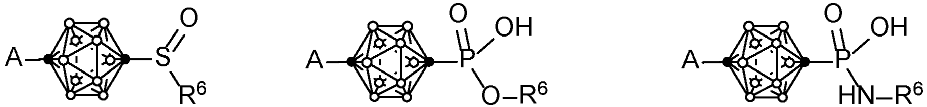 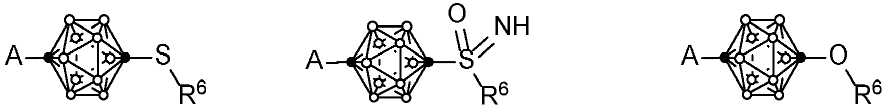 wherein • is a carbon atom; ∘ is B-H, B-halogen, B-alkyl, B-OH, or B-NH₂; the dotted line to Y indicates that the bond can be a single bond or a double bond, as valence permits; A is a substituted or unsubstituted aryl ring a substituted or unsubstituted heteroaryl ring; Y, when present, is O, halogen, OR^{2'}, NHR², SH, or S(O)(O)NHR²; R⁶ is substituted or unsubstituted C₁-C₁₉ alkyl, substituted or unsubstituted C₂-C₁₉ alkenyl, substituted or unsubstituted C₂-C₁₉ alkynyl, substituted or unsubstituted C₂-C₁₉ alkylaryl, substituted or unsubstituted C₂-C₁₉ alkylheteroaryl, substituted or unsubstituted C₄-C₁₉ alkylcycloalkyl, substituted or unsubstituted C₄-C₁₉ alkylheterocycloalkyl, and substituted or unsubstituted C₂-C₂₀ heteroalkyl. or NR³R⁴; R² is H, OH, halogen, or substituted or unsubstituted C₁-C₄ alkyl; R^{2'} is H or substituted or unsubstituted C₁-C₄ alkyl; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some embodiments, A can comprise a substituted or unsubstituted aryl ring (e.g., a substituted or unsubstituted phenyl ring). In some embodiments, A can be a five-membered substituted or unsubstituted heteroaryl ring. For example, A can comprise a thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, or 1,3,4-oxadiazolyl ring. In some embodiments, A can be a six-membered substituted or unsubstituted heteroaryl ring. For example, A can comprise a pyridyl, pyrazinyl, pyrimidinyl, triazinyl, or pyridazinyl ring.

In some of these embodiments, Y is OH. In some of these embodiments, Y is F. In some of these embodiments, Y is O.

In some examples, R⁶ can be a substituted or unsubstituted C₃-C₁₀ alkyl, such as a substituted or unsubstituted C₆-C₉ alkyl.

In some examples, R⁶ can be a substituted or unsubstituted C₂-C₁₅ alkylaryl.

In some examples, R⁶ can be a substituted or unsubstituted branched C₂-C₉ alkyl.

In some examples, R⁶ can be a substituted or unsubstituted C₃-C₁₀ heteroalkyl, such as a substituted or unsubstituted C₆-C₉ heteroalkyl.

In some examples, the carborane (not claimed) can be selected from the group consisting of: 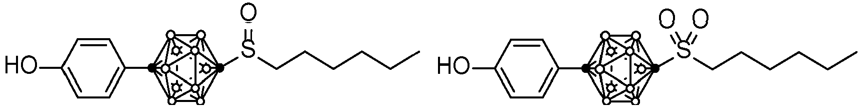 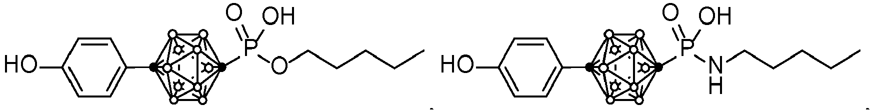 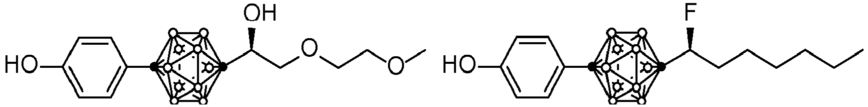 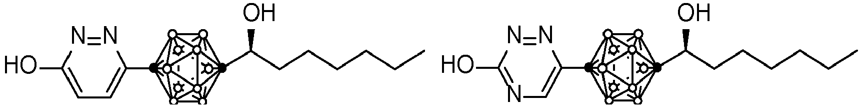 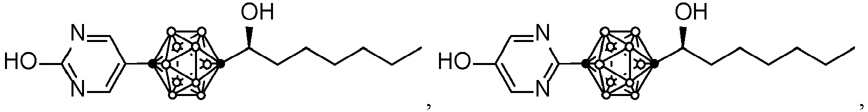 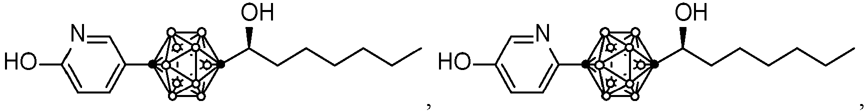 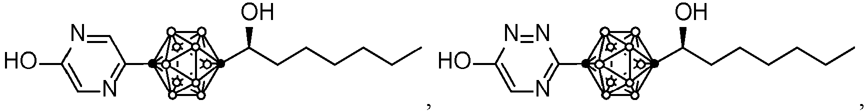 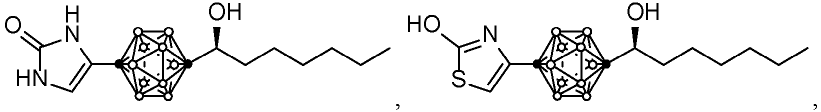 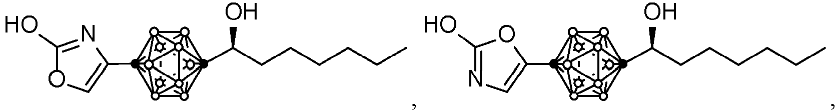 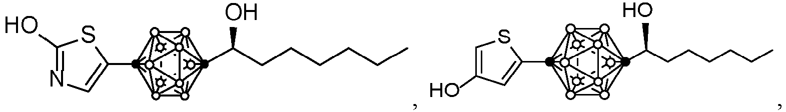 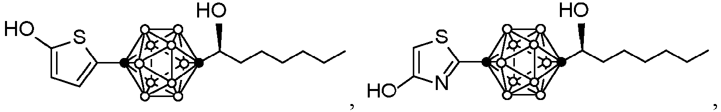 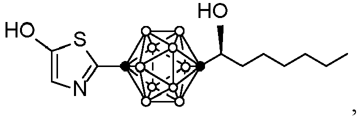 and pharmaceutically acceptable salts thereof. In some examples, the carborane cluster can include a heteroatom.

In some embodiments, the compound can be a carborane analog, such as a dicarba-closo-dodecaborane analog of, for example, the compounds described in WO 2017/049307 to Tjarks et al. The compounds include a spacer group which replaces the carborane moiety in the compounds therein. The resulting compounds can exhibit similar biological activity to the compounds described in WO 2017/049307.

For example, provided herein are compounds defined by Formula XIV, or a pharmaceutically acceptable salt thereof:

A-Q-R¹ Formula XIV

wherein A is a substituted or unsubstituted aryl ring or a substituted or unsubstituted heteroaryl ring; Q is a spacer group chosen from one of the following: 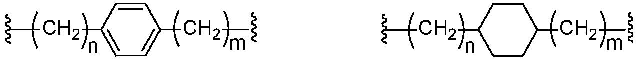 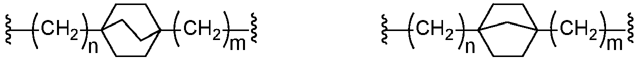 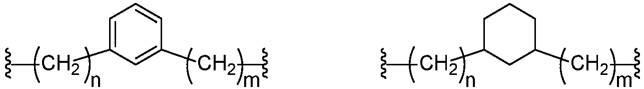 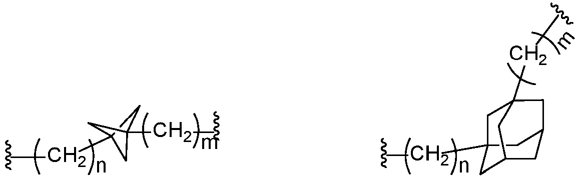 where m and n are each individually 0, 1, 2, or 3; R¹ is substituted or unsubstituted C₄-C₂₀ alkyl, substituted or unsubstituted C₄-C₂₀ heteroalkyl, substituted or unsubstituted C₂-C₂₀ alkenyl, substituted or unsubstituted C₂-C₂₀ alkynyl, substituted or unsubstituted C₃-C₂₀ alkylaryl, substituted or unsubstituted C₃-C₂₀ alkylheteroaryl, substituted or unsubstituted C₄-C₂₀ alkylcycloalkyl, substituted or unsubstituted C₄-C₂₀ alkylheterocycloalkyl, substituted or unsubstituted C₁-C₂₀ acyl, C₁-C₂₀ acyl, -C(O)N R³R⁴, or NR³R⁴; and R³ and R⁴ are independently selected from substituted or unsubstituted C₁-C₂₀ alkyl, substituted or unsubstituted C₁-C₂₀ heteroalkyl, substituted or unsubstituted C₂-C₂₀ alkenyl, substituted or unsubstituted C₂-C₂₀ alkynyl, substituted or unsubstituted C₂-C₂₀ alkylaryl, or substituted or unsubstituted C₄-C₂₀ alkylcycloalkyl.

In certain embodiments, Q can be chosen from one of the following:

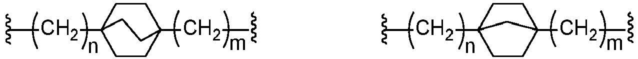

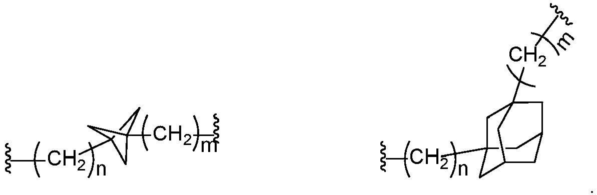

In some embodiments, A can comprise a substituted or unsubstituted aryl ring (e.g., a substituted or unsubstituted phenyl ring). In some embodiments, A can be a five-membered substituted or unsubstituted heteroaryl ring. For example, A can comprise a thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, or 1,3,4-oxadiazolyl ring. In some embodiments, A can be a six-membered substituted or unsubstituted heteroaryl ring. For example, A can comprise a pyridyl, pyrazinyl, pyrimidinyl, triazinyl, or pyridazinyl ring.

In some embodiments, A is 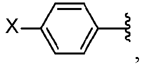 wherein X is OH, NHR², SH, or S(O)(O)NHR² and R² is H, OH, halogen, or substituted or unsubstituted C₁-C₄ alkyl. In some of these embodiments, X is OH.

In some embodiments, A is 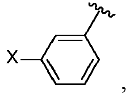 wherein X is OH, NHR², SH, or S(O)(O)NHR² and R² is H, OH, halogen, or substituted or unsubstituted C₁-C₄ alkyl. In some of these embodiments, X is OH.

In some embodiments, A is 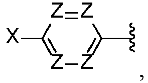 wherein Z is, individually for each occurrence, N or CH, with the proviso that at least one of Z is N; X is OH, NHR², SH, or S(O)(O)NHR²; and R² is H, OH, halogen, or substituted or unsubstituted C₁-C₄ alkyl. In some of these embodiments, A can be one of the following: 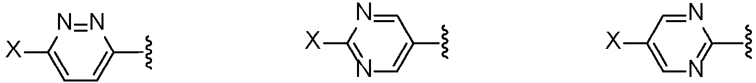 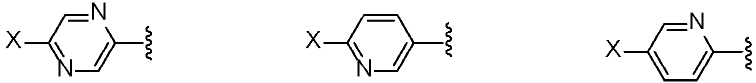 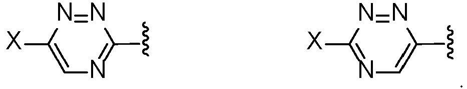 In some of these embodiments, X is OH.

In some embodiments, A is 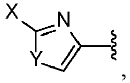 wherein Y is S or O; X is OH, NHR², SH, or S(O)(O)NHR²; and R² is H, OH, halogen, or substituted or unsubstituted C₁-C₄ alkyl. In some of these embodiments, X is OH.

In some embodiments, A is 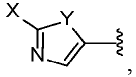 wherein Y is S or O; X is OH, NHR², SH, or S(O)(O)NHR²; and R² is H, OH, halogen, or substituted or unsubstituted C₁-C₄ alkyl. In some of these embodiments, X is OH.

In some embodiments, A is

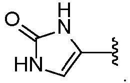

In some of the embodiments above, R¹ can be a substituted or unsubstituted C₆-C₁₀ alkyl (e.g., a C₆-C₁₀ hydroxyalkyl).

In some of the embodiments above, R¹ can be a substituted or unsubstituted C₃-C₁₆ alkylaryl (e.g., a C₃-C₁₆ hydroxyalkylaryl).

In some of the embodiments above, R¹ can be a substituted or unsubstituted C₈-C₂₀ alkylaryl (e.g., a C₈-C₂₀ hydroxyalkylaryl).

In some of the embodiments above, R¹ can be a substituted or unsubstituted C₅-C₁₀ acyl.

In some of the embodiments above, R¹ can be a substituted or unsubstituted branched C₄-C₁₀ alkyl (e.g., a branched C₄-C₁₀ hydroxyalkyl).

In some embodiments, R¹ can comprise one of the following 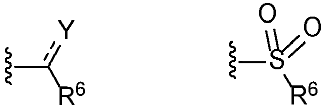 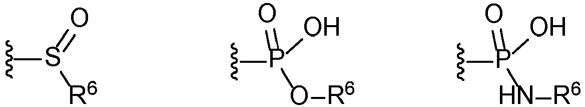 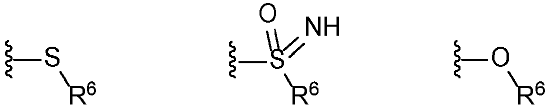 wherein the dotted line to Y indicates that the bond can be a single bond or a double bond, as valence permits; Y, when present, is O, halogen, OR^{2'}, NHR², SH, or S(O)(O)NHR²; R⁶ is substituted or unsubstituted C₁-C₁₉ alkyl, substituted or unsubstituted C₂-C₁₉ alkenyl, substituted or unsubstituted C₂-C₁₉ alkynyl, substituted or unsubstituted C₂-C₁₉ alkylaryl, substituted or unsubstituted C₂-C₁₉ alkylheteroaryl, substituted or unsubstituted C₄-C₁₉ alkylcycloalkyl, substituted or unsubstituted C₄-C₁₉ alkylheterocycloalkyl, and substituted or unsubstituted C₂-C₂₀ heteroalkyl. or NR³R⁴; R² is H, OH, halogen, or substituted or unsubstituted C₁-C₄ alkyl; R^{2'} is H or substituted or unsubstituted C₁-C₄ alkyl; and R³ and R⁴ are independently selected from substituted or unsubstituted C₁-C₂₀ alkyl, substituted or unsubstituted C₂-C₂₀ alkenyl, substituted or unsubstituted C₂-C₂₀ alkynyl, substituted or unsubstituted C₂-C₂₀ alkylaryl, substituted or unsubstituted C₄-C₂₀ alkylcycloalkyl, and substituted or unsubstituted C₂-C₂₀ heteroalkyl.

In some embodiments, A can comprise a substituted or unsubstituted aryl ring (e.g., a substituted or unsubstituted phenyl ring). In some embodiments, A can be a five-membered substituted or unsubstituted heteroaryl ring. For example, A can comprise a thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, or 1,3,4-oxadiazolyl ring. In some embodiments, A can be a six-membered substituted or unsubstituted heteroaryl ring. For example, A can comprise a pyridyl, pyrazinyl, pyrimidinyl, triazinyl, or pyridazinyl ring.

In some of these embodiments, Y is OH. In some of these embodiments, Y is F. In some of these embodiments, Y is O.

In some examples, R⁶ can be a substituted or unsubstituted C₃-C₁₀ alkyl, such as a substituted or unsubstituted C₆-C₉ alkyl.

In some examples, R⁶ can be a substituted or unsubstituted C₂-C₁₅ alkylaryl.

In some examples, R⁶ can be a substituted or unsubstituted branched C₂-C₉ alkyl.

In some examples, R⁶ can be a substituted or unsubstituted C₃-C₁₀ heteroalkyl, such as a substituted or unsubstituted C₆-C₉ heteroalkyl.

In some embodiments, the compound (not claimed) can comprise one of the following:

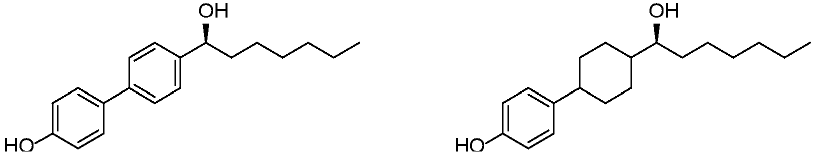

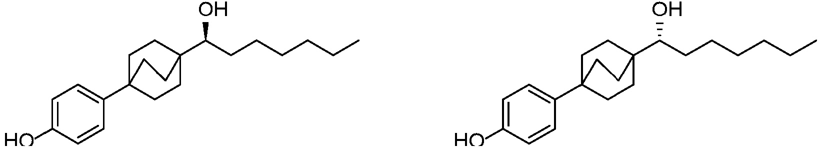

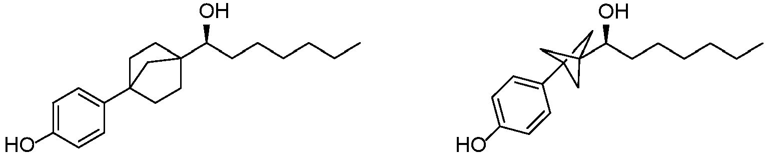

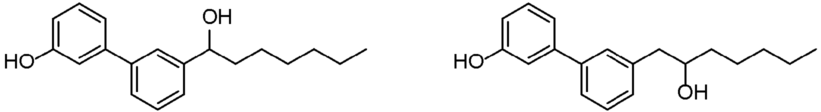

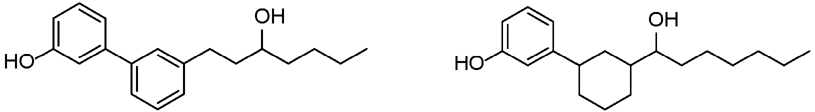

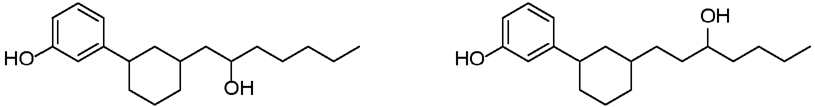

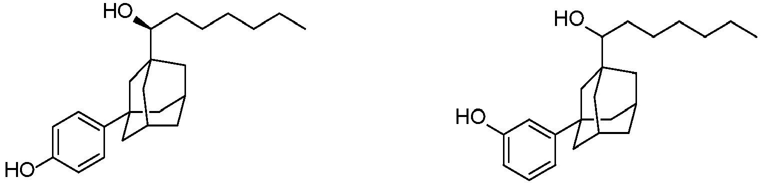

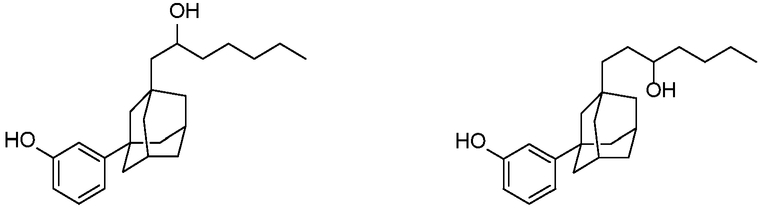

Also disclosed herein are pharmaceutically-acceptable salts and prodrugs of the carboranes and carborane analogs described herein. Pharmaceutically-acceptable salts include salts of the disclosed carboranes and carborane analogs that are prepared with acids or bases, depending on the particular substituents found on the compounds. Under conditions where the carboranes and carborane analogs disclosed herein are sufficiently basic or acidic to form stable nontoxic acid or base salts, administration of the compounds as salts can be appropriate. Examples of pharmaceutically-acceptable base addition salts include sodium, potassium, calcium, ammonium, or magnesium salt. Examples of physiologically-acceptable acid addition salts include hydrochloric, hydrobromic, nitric, phosphoric, carbonic, sulfuric, and organic acids like acetic, propionic, benzoic, succinic, fumaric, mandelic, oxalic, citric, tartaric, malonic, ascorbic, alpha-ketoglutaric, alpha-glycophosphoric, maleic, tosyl acid, methanesulfonic, and the like. Thus, disclosed herein are the hydrochloride, nitrate, phosphate, carbonate, bicarbonate, sulfate, acetate, propionate, benzoate, succinate, fumarate, mandelate, oxalate, citrate, tartarate, malonate, ascorbate, alpha-ketoglutarate, alpha-glycophosphate, maleate, tosylate, and mesylate salts. Pharmaceutically acceptable salts of a compound can be obtained using standard procedures well known in the art, for example, by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

In some examples, the carboranes and carborane analogs disclosed herein can have an EC₅₀ of 800 nM or less at estrogen receptor beta (ERβ) (e.g., 700 nM or less, 600 nM or less, 500 nM or less, 400 nM or less, 300 nM or less, 200 nM or less, 100 nM or less, 90 nM or less, 80 nM or less, 70 nM or less, 60 nM or less, 50 nM or less, 40 nM or less, 30 nM or less, 20 nM or less, 10 nM or less, 9 nM or less, 8 nM or less, 7 nM or less, 6 nM or less, 5 nM or less, 4.5 nM or less, 4 nM or less, 3.5 nM or less, 3 nM or less, 2.5 nM or less, 2 nM or less, 1.5 nM or less, 1 nM or less, 0.9 nM or less, 0.8 nM or less, 0.7 nM or less, 0.6 nM or less, 0.5 nM or less, 0.4 nM or less, 0.3 nM or less, 0.2 nM or less, or 0.1 nM or less).

In some examples, the carboranes and carborane analogs disclosed herein can have an EC₅₀ of 1 pM or more at ERβ (e.g., 0.1 nM or more, 0.2 nM or more, 0.3 nM or more, 0.4 nM or more, 0.5 nM or more, 0.6 nM or more, 0.7 nM or more, 0.8 nM or more, 0.9 nM or more, 1 nM or more, 1.5 nM or more, 2 nM or more, 2.5 nM or more, 3 nM or more, 3.5 nM or more, 4 nM or more, 4.5 nM or more, 5 nM or more, 6 nM or more, 7 nM or more, 8 nM or more, 9 nM or more, 10 nM or more, 20 nM or more, 30 nM or more, 40 nM or more, 50 nM or more, 60 nM or more, 70 nM or more, 80 nM or more, 90 nM or more, 100 nM or more, 200 nM or more, 300 nM or more, 400 nM or more, 500 nM or more, 600 nM or more, or 700 nM or more).

The EC₅₀ of the carboranes and carborane analogs at ERβ can range from any of the minimum values described above to any of the maximum values described above. For example, the carboranes and carborane analogs disclosed herein can have an EC₅₀ of from 1 pM to 800 nM at ERβ (e.g., from 1 pM to 400 nM, from 400 nM to 800 nM, from 1 pM to 300 nM, from 1 pM to 200 nM, from 1 pM to 100 nM, from 1 pM to 50 nM, from 1 pM to 20 nM, from 1 pM to 10 nM, from 1 pM to 6 nM, from 1 pM to 5 nM, from 1 pM to 2 nM, from 1 pM to 1 nM, from 1 pM to 0.7 nM, from 1 pM to 0.5 nM, from 1 pM to 0.2 pM, or from 1 pM to 0.1 nM).

In some examples, the carboranes and carborane analogs disclosed herein are selective ERβ agonist. In some examples, a selective ERβ agonist is a compound that has a lower EC₅₀ at ERβ than at estrogen receptor α (ERα). The selectivity of the compounds can, in some examples, be expressed as an ERβ-to-ERα agonist ratio, which is the EC₅₀ of the compound at ERα divided by the EC₅₀ of the compound at ERβ. In some examples, the compounds disclosed herein can have an ERβ-to-ERα agonist ratio of 8 or more (e.g., 10 or more, 20 or more, 30 or more, 40 or more, 50 or more, 60 or more, 70 or more, 80 or more, 90 or more, 100 or more, 150 or more, 200 or more, 250 or more, 300 or more, 350 or more, 400 or more, 450 or more, 500 or more, 600 or more, 700 or more, 800 or more, 900 or more, 1000 or more, 1100 or more, 1200 or more, 1300 or more, 1400 or more, 1500 or more, 2000 or more, 2500 or more).

In some examples, the carboranes and carborane analogs can have an ERβ-to-ERα agonist ratio of 3000 or less (e.g., 2500 or less, 2000 or less, 1500 or less, 1400 or less, 1300 or less, 1200 or less, 1100 or less, 1000 or less, 900 or less, 800 or less, 700 or less, 600 or less, 500 or less, 450 or less, 400 or less, 350 or less, 300 or less, 250 or less, 200 or less, 150 or less, 100 or less, 90 or less, 80 or less, 70 or less, 60 or less, 50 or less, 40 or less, 30 or less, 20 or less, or 10 or less).

The ERβ-to-ERα agonist ratio of the carboranes and carborane analogs at ERβ can range from any of the minimum values described above to any of the maximum values described above. For example, the carboranes and carborane analogs can have an ERβ-to-ERα agonist ratio of from 8 to 3000 (e.g., from 8 to 1500, from 1500 to 3000, from 400 to 3000, from 500 to 3000, from 600 to 3000, from 700 to 3000, from 800 to 3000, from 900 to 3000, from 1000 to 3000, or from 2000 to 3000).

### Methods of Making

The compounds described herein can be prepared in a variety of ways known to one skilled in the art of organic synthesis or variations thereon as appreciated by those skilled in the art. The compounds described herein can be prepared from readily available starting materials. Optimum reaction conditions can vary with the particular reactants or solvents used, but such conditions can be determined by one skilled in the art.

Variations on the compounds described herein include the addition, subtraction, or movement of the various constituents as described for each compound. Similarly, when one or more chiral centers are present in a molecule, the chirality of the molecule can be changed. Additionally, compound synthesis can involve the protection and deprotection of various chemical groups. The use of protection and deprotection, and the selection of appropriate protecting groups can be determined by one skilled in the art. The chemistry of protecting groups can be found, for example, in Wuts and Greene, Protective Groups in Organic Synthesis, 4th Ed., Wiley & Sons, 2006.

The starting materials and reagents used in preparing the disclosed compounds and compositions are either available from commercial suppliers such as Katchem (Prague, Czech Republic), Aldrich Chemical Co., (Milwaukee, WI), Acros Organics (Morris Plains, NJ), Fisher Scientific (Pittsburgh, PA), Sigma (St. Louis, MO), Pfizer (New York, NY), GlaxoSmithKline (Raleigh, NC), Merck (Whitehouse Station, NJ), Johnson & Johnson (New Brunswick, NJ), Aventis (Bridgewater, NJ), AstraZeneca (Wilmington, DE), Novartis (Basel, Switzerland), Wyeth (Madison, NJ), Bristol-Myers-Squibb (New York, NY), Roche (Basel, Switzerland), Lilly (Indianapolis, IN), Abbott (Abbott Park, IL), Schering Plough (Kenilworth, NJ), or Boehringer Ingelheim (Ingelheim, Germany), or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989). Other materials, such as the pharmaceutical excipients disclosed herein can be obtained from commercial sources.

Reactions to produce the compounds described herein can be carried out in solvents, which can be selected by one of skill in the art of organic synthesis. Solvents can be substantially nonreactive with the starting materials (reactants), the intermediates, or products under the conditions at which the reactions are carried out, *i.e.,* temperature and pressure. Reactions can be carried out in one solvent or a mixture of more than one solvent. Product or intermediate formation can be monitored according to any suitable method known in the art. For example, product formation can be monitored by spectroscopic means, such as nuclear magnetic resonance spectroscopy (*e.g.,* ¹H or ¹³C) infrared spectroscopy, spectrophotometry (*e.g.,* UV-visible), or mass spectrometry, or by chromatography such as high-performance liquid chromatography (HPLC) or thin layer chromatography.

Example methods of preparing carboranes and carborane analogs are described, for example, in U.S. Patent No. 6,838,574 to Endo, U.S. Patent Application Publication No. 2018/0264017 to Tjarks et al., and PCT/US2019/064228 to Coss et al..

### Methods of Use

The carboranes and carborane analogs described herein can inhibit the activation and proliferation of CD4+ T-cells. As such, the carboranes and carborane analogs described herein can be administered to a subject to reduce circulating CD4+ T-cell levels (e.g., relative to levels prior to administration of the carborane or carborane analog, or relative to levels in a control not treated with the carborane or carborane analog). In some embodiments, the carboranes and carborane analogs described herein can selectively reduce levels of circulating CD4+ T-cells while leaving the levels of other leukocytes substantially unchanged. For example, in some embodiments, the carboranes or carborane analogs can be administered in an effective amount to reduce circulating CD4+ T-cell levels in the subject (e.g., by at least 20%, 25%, 30%, 40%, 50%, 60%, 70%, 75%, or more) without significantly affecting circulating levels of neutrophils, monocytes, or B-cells (e.g., a change of less than 15%, less than 10%, or less than 5%). These reductions can be measured relative to levels prior to administration of the carborane or carborane analog, or relative to levels in a control not treated with the carborane or carborane analog.

CD4+ T-cells mediate wound-healing post-myocardial infarction (MI) but exacerbate left-ventricular (LV) remodeling during chronic heart failure (HF). Mechanisms that lead to this transition are unknown. However, without wishing to be bound by theory, it is believed that T-cells activate specific pathological signals to promote LV-remodeling during chronic HF. To identify such signals, limited cell RNA-sequencing of CD4+ T-cells sorted from the failing hearts (8 weeks post-MI) of male mice was performed and, surprisingly, activation of estrogen receptor (ER)-α signaling was observed. As ERα effects are antagonized by ERβ, ERβ agonists (including the carboranes and carborane analogs described herein) can be administered to modulate T-cell activity and LV remodeling.

As detailed in the Examples below, *in vitro* assays showed that an example carborane (Compound 1) dose dependently inhibited the activation and proliferation of T-cells sorted from male mice (IC₅₀ 3.4 µM). *In vivo* assays (60 mg/kg/day; oral) showed no overt toxicity and a significant reduction in circulating T cells without affecting neutrophils, monocytes, or B-cells indicating specificity against T-cells. Furthermore, this effect was specific to TCR-mediated T-cell activation and the drug did not affect T-cells stimulated with PMA/Ionomycin suggesting preferential inhibition of antigenically-activated T-cells.

To test therapeutic efficacy, male 10-12 week old mice underwent coronary ligation or sham operation and at 4 weeks post-MI randomized according to their cardiac function to receive either the vehicle or Compound 1 (60 mg/kg/day, oral) for the next 4 weeks. Consistently, at 8 weeks a significant reduction in T-cells in the circulation and the spleens of drug-treated mice was observed when compared with the vehicle treatment. Further, vehicle treated HF mice showed progressive LV dilatation with significantly increased end-diastolic and end-systolic volumes (EDV and ESV, respectively) from 4-8 weeks post-MI. Importantly, treatment with Compound 1 significantly inhibited these changes and blunted LV remodeling from 4-8 weeks post-MI. Significant reduction in tibia-normalized heart weights supported these results. These examples suggest that the carborane and carborane analogs described herein can selectively inhibit T-cell activation and blunt pathological LV-remodeling during chronic HF.

Accordingly, provided herein are methods for treating or preventing chronic heart failure in a subject following myocardial infarction. These methods can comprise administering to the subject a carborane or carborane analog during a maladaptive remodeling phase following the myocardial infarction. Importantly, in some embodiments, the carborane or carborane analog is not administered to the subject during a healing phase or a repair phase preceding the maladaptive remodeling phase (i.e., administration of the carborane or carborane analog does not commence until the acute phase following MI is complete and the chronic phase has commenced).

In some embodiments, administration of the carborane or carborane analog commences at least 10 days following the myocardial infarction, such as at least 14 days following the myocardial infarction, at least 21 days following the myocardial infarction, at least 28 days following the myocardial infarction, at least 35 days following myocardial infarction, at least 42 days following myocardial infarction, at least 49 days following myocardial infarction, or at least 56 days following myocardial infarction.

These methods can further comprise assessing the subject to determine whether the subject has entered the maladaptive remodeling phase. This can be done via any suitable method. For example, in some embodiments, assessing the subject to determine whether the subject has entered the maladaptive remodeling phase can comprise measuring circulating CD4+ T-cell levels in the subject to determine when the subject has entered the maladaptive remodeling phase. In some embodiments, assessing the subject to determine whether the subject has entered the maladaptive remodeling phase can comprise detecting one or more biomarkers in the subject to determine when the subject has entered the maladaptive remodeling phase. Such biomarkers are known in the art, and include, for example, the relative levels of myosin heavy chain isoforms, GLUT-1 expression levels, alpha-actin expression levels, natriuretic peptide expression levels, galectin expression levels, caveolin expression levels, neuronal nitric oxide synthase expression levels, angiotensin-converting enzyme expression levels, GLUT-4 expression levels, SERCA2a expression levels, and a shift from glucose to fatty acid oxidation. In some embodiments, assessing the assessing the subject to determine whether the subject has entered the maladaptive remodeling phase can comprise echocardiography, ventriculography, nuclear magnetic resonance, or any combination thereof. Imaging techniques such as echocardiography and/or MRI can also be used to measure left-ventricular dilatation (increased end-diastolic and end-systolic volumes) as an indicator of LV remodeling.

In some embodiments, the carborane or carborane analog can be administered in an effective amount to inhibit activation and proliferation of CD4+ T-cells in the subject. As such, the carboranes and carborane analogs described herein can be administered to a subject to reduce circulating CD4+ T-cell levels (e.g., relative to levels prior to administration of the carborane or carborane analog, or relative to levels in a control not treated with the carborane or carborane analog). In some embodiments, the carboranes and carborane analogs described herein can selectively reduce levels of circulating CD4+ T-cells while leaving the levels of other leukocytes substantially unchanged. For example, in some embodiments, the carboranes or carborane analogs can be administered in an effective amount to reduce circulating CD4+ T-cell levels in the subject (e.g., by at least 20%, 25%, 30%, 40%, 50%, 60%, 70%, 75%, or more) without significantly affecting circulating levels of neutrophils, monocytes, or B-cells (e.g., a change of less than 15%, less than 10%, or less than 5%). These reductions can be measured relative to levels prior to administration of the carborane or carborane analog, or relative to levels in a control not treated with the carborane or carborane analog.

In some embodiments, the carborane or carborane analog can be administered in an effective amount to alter morphological changes associated with CHF following MI. For example, in some embodiments, the carborane or carborane analog can be administered in an effective amount to decrease left ventricular (LV) remodeling in the subject. In some embodiments, the carborane or carborane analog can be administered in an effective amount to reduce changes is cardiac function associated with CHF following MI. For example, in some embodiments, the carborane or carborane analog can be administered in an effective amount to inhibit an increase in left ventricular end-diastolic volume in the subject, inhibit an increase in left ventricular end-systolic volume in the subject, or any combination thereof.

Also provided and not claimed are methods of treating and preventing autoimmune disorders in which the carboranes and carboranes analogs are administered to modulate T-cell activity (e.g., selectively modulate T-cell activity) in a subject. For example, provided herein are methods of treating or preventing graft-versus-host disease, multiple sclerosis (MS), and/or experimental autoimmune encephalomyelitis (EAE) in a subject that comprise administering to the subject a carborane or carborane analog.

When practicing these methods, the carborane or carborane analog can administered in an effective amount to inhibit activation and proliferation of CD4+ T-cells in the subject. For example, the carborane or carborane analog can be administered in an effective amount to reduce reducing circulating CD4+ T-cell levels in the subject. In certain embodiments, the carborane or carborane analog can be administered in an effective amount to reduce circulating CD4+ T-cell levels in the subject without significantly affecting circulating levels of neutrophils, monocytes, or B-cells.

The methods and compounds as described herein are useful for both prophylactic and therapeutic treatment. As used herein the term treating or treatment includes prevention; delay in onset; diminution, eradication, or delay in exacerbation of signs or symptoms after onset; and prevention of relapse. For prophylactic use, a therapeutically effective amount of the compounds and compositions or pharmaceutically acceptable salts thereof as described herein are administered to a subject prior to onset (*e*.*g*., before obvious signs of the disease or disorder), during early onset (*e.g*., upon initial signs and symptoms of the disease or disorder), or after an established development of the disease or disorder. Prophylactic administration can occur for several days to years prior to the manifestation of symptoms of a disease or disorder. Therapeutic treatment involves administering to a subject a therapeutically effective amount of the compounds and compositions or pharmaceutically acceptable salts thereof as described herein after the disease or disorder is diagnosed.

### Compositions, Formulations and Methods of Administration

*In vivo* application of the disclosed compounds, and compositions containing them, can be accomplished by any suitable method and technique presently or prospectively known to those skilled in the art. For example, the disclosed compounds can be formulated in a physiologically- or pharmaceutically-acceptable form and administered by any suitable route known in the art including, for example, oral, nasal, rectal, topical, and parenteral routes of administration. As used herein, the term parenteral includes subcutaneous, intradermal, intravenous, intramuscular, intraperitoneal, and intrasternal administration, such as by injection. Administration of the disclosed compounds or compositions can be a single administration, or at continuous or distinct intervals as can be readily determined by a person skilled in the art.

The compounds disclosed herein, and compositions comprising them, can also be administered utilizing liposome technology, slow release capsules, implantable pumps, and biodegradable containers. These delivery methods can, advantageously, provide a uniform dosage over an extended period of time. The compounds can also be administered in their salt derivative forms or crystalline forms.

The compounds disclosed herein can be formulated according to known methods for preparing pharmaceutically acceptable compositions. Formulations are described in detail in a number of sources which are well known and readily available to those skilled in the art. For example, Remington's Pharmaceutical Science by E.W. Martin (1995) describes formulations that can be used in connection with the disclosed methods. In general, the compounds disclosed herein can be formulated such that a therapeutically effective amount of the compound is combined with a suitable excipient in order to facilitate effective administration of the compound. The compositions used can also be in a variety of forms. These include, for example, solid, semi-solid, and liquid dosage forms, such as tablets, pills, powders, liquid solutions or suspension, suppositories, injectable and infusible solutions, and sprays. The preferred form depends on the intended mode of administration and therapeutic application. The compositions also preferably include conventional pharmaceutically-acceptable carriers and diluents which are known to those skilled in the art. Examples of carriers or diluents for use with the compounds include ethanol, dimethyl sulfoxide, glycerol, alumina, starch, saline, and equivalent carriers and diluents. To provide for the administration of such dosages for the desired therapeutic treatment, compositions disclosed herein can advantageously comprise between about 0.1% and 100% by weight of the total of one or more of the subject compounds based on the weight of the total composition including carrier or diluent.

Formulations suitable for administration include, for example, aqueous sterile injection solutions, which can contain antioxidants, buffers, bacteriostats, and solutes that render the formulation isotonic with the blood of the intended recipient; and aqueous and nonaqueous sterile suspensions, which can include suspending agents and thickening agents. The formulations can be presented in unit-dose or multi-dose containers, for example sealed ampoules and vials, and can be stored in a freeze dried (lyophilized) condition requiring only the condition of the sterile liquid carrier, for example, water for injections, prior to use. Extemporaneous injection solutions and suspensions can be prepared from sterile powder, granules, tablets, *etc.* It should be understood that in addition to the excipients particularly mentioned above, the compositions disclosed herein can include other agents conventional in the art having regard to the type of formulation in question.

Compounds disclosed herein, and compositions comprising them, can be delivered to a cell either through direct contact with the cell or via a carrier means. Carrier means for delivering compounds and compositions to cells are known in the art and include, for example, encapsulating the composition in a liposome moiety. Another means for delivery of compounds and compositions disclosed herein to a cell comprises attaching the compounds to a protein or nucleic acid that is targeted for delivery to the target cell. U.S. Patent No. 6,960,648 and U.S. Application Publication Nos. 20030032594 and 20020120100 disclose amino acid sequences that can be coupled to another composition and that allows the composition to be translocated across biological membranes. U.S. Application Publication No. 20020035243 also describes compositions for transporting biological moieties across cell membranes for intracellular delivery. Compounds can also be incorporated into polymers, examples of which include poly (D-L lactide-co-glycolide) polymer for intracranial tumors; poly[bis(p-carboxyphenoxy) propane:sebacic acid] in a 20:80 molar ratio (as used in GLIADEL); chondroitin; chitin; and chitosan.

In certain examples, compounds and compositions disclosed herein can be locally administered at one or more anatomical sites, such as sites of unwanted cell growth optionally in combination with a pharmaceutically acceptable carrier such as an inert diluent. Compounds and compositions disclosed herein can be systemically administered, such as intravenously or orally, optionally in combination with a pharmaceutically acceptable carrier such as an inert diluent, or an assimilable edible carrier for oral delivery. They can be enclosed in hard or soft shell gelatin capsules, can be compressed into tablets, or can be incorporated directly with the food of the patient's diet. For oral therapeutic administration, the active compound can be combined with one or more excipients and used in the form of ingestible tablets, buccal tablets, troches, capsules, elixirs, suspensions, syrups, wafers, aerosol sprays, and the like.

The tablets, troches, pills, capsules, and the like can also contain the following: binders such as gum tragacanth, acacia, corn starch or gelatin; diluents such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring can be added. When the unit dosage form is a capsule, it can contain, in addition to materials of the above type, a liquid carrier, such as a vegetable oil or a polyethylene glycol. Various other materials can be present as coatings or to otherwise modify the physical form of the solid unit dosage form. For instance, tablets, pills, or capsules can be coated with gelatin, wax, shellac, or sugar and the like. A syrup or elixir can contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound can be incorporated into sustained-release preparations and devices.

Compounds and compositions disclosed herein, including pharmaceutically acceptable salts or prodrugs thereof, can be administered intravenously, intramuscularly, or intraperitoneally by infusion or injection. Solutions of the active agent or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations can contain a preservative to prevent the growth of microorganisms.

The pharmaceutical dosage forms suitable for injection or infusion can include sterile aqueous solutions or dispersions or sterile powders comprising the active ingredient, which are adapted for the extemporaneous preparation of sterile injectable or infusible solutions or dispersions, optionally encapsulated in liposomes. The ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. Optionally, the prevention of the action of microorganisms can be brought about by various other antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the inclusion of agents that delay absorption, for example, aluminum monostearate and gelatin.

Sterile injectable solutions are prepared by incorporating a compound and/or agent disclosed herein in the required amount in the appropriate solvent with various other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, compounds and agents disclosed herein can be applied in as a liquid or solid.

Useful solid carriers include finely divided solids such as talc, clay, microcrystalline cellulose, silica, alumina and the like. Useful liquid carriers include water, alcohols or glycols or water-alcohol/glycol blends, in which the compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers, for example.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the compounds and agents and pharmaceutical compositions disclosed herein can be determined by comparing their *in vitro* activity, and *in vivo* activity in animal models. Methods for the extrapolation of effective dosages in mice, and other animals, to humans are known to the art.

The dosage ranges for the administration of the compositions are those large enough to produce the desired effect in which the symptoms or disorder are affected. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counterindications. Dosage can vary, and can be administered in one or more dose administrations daily, for one or several days.

Also disclosed are pharmaceutical compositions that comprise a compound disclosed herein in combination with a pharmaceutically acceptable excipient. Pharmaceutical compositions adapted for oral, topical or parenteral administration, comprising an amount of a compound constitute a preferred aspect. The dose administered to a patient, particularly a human, should be sufficient to achieve a therapeutic response in the patient over a reasonable time frame, without lethal toxicity, and preferably causing no more than an acceptable level of side effects or morbidity. One skilled in the art will recognize that dosage will depend upon a variety of factors including the condition (health) of the subject, the body weight of the subject, kind of concurrent treatment, if any, frequency of treatment, therapeutic ratio, as well as the severity and stage of the pathological condition.

Also disclosed are kits that comprise a compound disclosed herein in one or more containers. The disclosed kits can optionally include pharmaceutically acceptable carriers and/or diluents. In one embodiment, a kit includes one or more other components, adjuncts, or adjuvants as described herein. In one embodiment, a kit includes instructions or packaging materials that describe how to administer a compound or composition of the kit. Containers of the kit can be of any suitable material, *e.g.,* glass, plastic, metal, *etc.,* and of any suitable size, shape, or configuration. In one embodiment, a compound and/or agent disclosed herein is provided in the kit as a solid, such as a tablet, pill, or powder form. In another embodiment, a compound and/or agent disclosed herein is provided in the kit as a liquid or solution. In one embodiment, the kit comprises an ampoule or syringe containing a compound and/or agent disclosed herein in liquid or solution form.

### EXAMPLES

The following examples are set forth to illustrate the methods and results according to the disclosed subject matter. These examples are not intended to be inclusive of all aspects of the subject matter disclosed herein, but rather to illustrate representative methods and results.

### Example 1. Evaluation of Example Carborane for the Treatment of Heart Failure

Immune and inflammatory responses contribute to left ventricular (LV) remodeling after myocardial infarction (MI). Heightened levels of inflammatory cytokines promote cardiac remodeling and disease progression in heart failure (HF); however, clinical trials of TNF neutralization failed to show benefit and even revealed harm at high doses. These paradoxical results suggest a more complex role for inflammatory activation in heart failure than gleaned from cytokine levels alone. Activation of innate and adaptive immune cells underlies inflammatory responses in many chronic diseases. Monocytes, macrophages, and dendritic cells (DCs) mediate innate immune responses, whereas CD3+CD4+ helper and CD3+CD8+ cytotoxic T-cells arbitrate adaptive immunity. While innate immune cells comprise the first line of defense against acute injury, chronic inflammation often implies activation and clonal expansion of specialized effector T-cells following antigen presentation.

Previously published studies demonstrated that chronic ischemic heart failure can be characterized by global expansion of CD4+ T-cells in blood, spleen and the heart. Moreover, depletion of CD4+ T-cells (using anti-CD4 antibody) in heart failure mice prevented the deterioration of cardiac function and progression of left ventricular remodeling. These studies suggested that T-lymphocyte activation during chronic heart failure can play an important role in mediating pathological left ventricular (LV) remodeling associated with progressive cardiac dysfunction (increased end-diastolic and end-systolic volumes and ejection fraction), and heightened myocyte hypertrophy and fibrosis.

The gene expression for estrogen receptors (ERs) α and β (ERα and ERβ) in the ovaries (positive control), hearts from both males and females (M+F), and male spleens is shown in Figure 1. β-Actin was used as an internal control and fold changes with respect to the heart (left and middle panel) or ERα (right panel) are shown in Figure 1. Figure 1 shows that ERα expression is similar in the hearts and spleens and is almost negligible as compared to the ovaries. In contrast, ERβ gene expression in spleens is almost 1/3^{rd} of that in ovaries and is much higher as compared to the hearts (middle panel, Figure 1). Moreover, the ratio of ERβ to ERα is much higher in spleens as compared to ovaries or the hearts (right panel, Figure 1) suggesting preferential expression of ERβ as compared to ERα in splenocytes.

Representative flow cytometric histograms for ERα (top panels) and ERβ (bottom panels) expression in different circulating (left panels) and splenic (right panels) immune cells in a male mouse are shown in Figure 2. Circulating and splenic immune cells do not express ERα, whereas several immune cell subsets were found to express significant levels of ERβ protein (Figure 2). ERβ expression is highest in CD11b+ myeloid cells (Ly6G+ neutrophils and Ly6C+ monocytes/macrophages) followed by CD4+ helper T-cells, and is lowest in CD19+ B cells (Figure 2).

Representative flow cytometric histograms for cell trace violet labeled (CTV; a cell proliferation dye) CD4+ T-cells either unstimulated or CD3/CD28 TCR stimulated and treated with either Estradiol (5 and 50 nM) or Compound 1 (5 µM, structure shown below) or both are shown in Figure 3. Peak patterns from high to low fluorescence intensity in stimulated cells represent halving of dye concentration in the cell membranes of the daughter cells with every successive cell division. Group quantitation for cell proliferation (%) measured as dye dilution with every successive cell division for stimulated and non-stimulated groups are shown in Figure 4 and Figure 5, respectively.

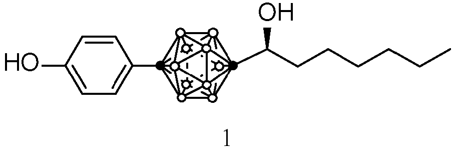

Cell survival of CD3/CD28 mediated *in-vitro* TCR stimulation with and without Compound 1 treatment are shown in Figure 6. The results indicate that treatment with Compound 1 does not affect cell survival of T-cells stimulated with CD3/CD28 antibodies (Figure 6).

Representative flow cytometric histograms for TNFα expression in CD4+ T-cells either unstimulated or CD3/CD28 TCR stimulated and treated with either Estradiol (5 and 50 nM) or Compound 1 (5 µM) or both are shown in Figure 7. Group quantitations for stimulated groups are shown in Figure 8 and unstimulated control groups are shown in Figure 9. The results indicate that treatment with Compound 1 suppressed the number of TNFα+ helper T-cells (CD4+).

Representative flow cytometric histograms for IFNγ expression in CD4+ T-cells either unstimulated or CD3/CD28 TCR stimulated and treated with either Estradiol (5 and 50 nM) or Compound 1 (5 µM) or both are shown in Figure 10. Group quantitations for stimulated groups are shown in Figure 11 and unstimulated control groups are shown in Figure 12. The results indicate that treatment with Compound 1 did not significantly affect the number of IFNγ+ helper T-cells (CD4+).

The results of CD3/CD28 TCR mediated *in-vitro* T-cell proliferation assays using T-cells from Female mice with and without Compound 1 are shown in Figure 13-Figure 16. The results show group quantitation for cell-survival (Figure 13), proliferation (Figure 14), TNFα+ helper T-cells (CD4+) (Figure 15), and IFNγ+ helper T-cells (CD4+) (Figure 16) either unstimulated or CD3/CD28 TCR stimulated and treated with either Estradiol (5 and 50 nM) or Compound 1 (5 µM) or both. The results suggest that treatment with Compound 1 suppresses cell proliferation, the number of TNFα+ helper T-cells (CD4+), and the number of IFNγ+ helper T-cells, without negatively affecting overall cell-survival.

The results of non-specific PMA/ionomycin mediated *in-vitro* T-cell activation with and without Compound 1 are shown in Figure 17-Figure 20.

The results show group quantitation for cell-survival (Figure 17), TNFα+ helper T-cells (CD4+) (Figure 18), IFNγ+ helper T-cells (CD4+) (Figure 19), and CD69+ helper T-cells (CD4+) (Figure 20) either unstimulated or stimulated with PMA/Ionomycin and treated with Compound 1 (5 µM). The results indicate that Compound 1 treatment did not affect the levels of TNFα+, IFNγ+, or CD69+ T-cells post-PMA/Ionomycin stimulation, suggesting Compound 1 specifically affects CD3/CD28 TCR mediated T-cell activation pathways.

Next, *in vivo* treatment was tested using mice. First, treatment was started at 7 days post-infarction to inhibit immune activation in the acute phase (Figure 27). The body weight of sham-operated and myocardial infarction (MI) mice treated with either vehicle control or Compound 1 is shown in Figure 21 (day 0 corresponds to the day treatment started, e.g., 7 days post myocardial infarction). A Kaplan-Meier curve shows mortality rate in myocardial infarction and sham-operated mice treated with vehicle or drug in Figure 22 (day 0 corresponds to the day treatment started, e.g., 7 days post myocardial infarction). As can be seen in Figure 22, treatment with Compound 1 during the acute phase of immune activation increased mortality (decreased survival), indicating the immune response during the acute phase is important for adequate healing.

Accordingly, subsequent tests examined the effect of treatment at 28 days post-infarction to inhibit immune activation in the chronic (e.g., maladaptive remodeling) phase (Figure 27). The body weight and tibia normalized heart weights of sham-operated and myocardial infarction (MI) mice treated with either vehicle control or Compound 1 28 days post-myocardial infarction are shown in Figure 23 and Figure 24, respectively. The results indicate that treatment with Compound 1 28 days post-myocardial infarction stopped the increase in heart weight (Figure 24). Further, there was no significant increase in mortality associated with Compound 1 treatment 28 days post-myocardial infarction.

The levels of circulating CD4+ Helper T-cells (per µL blood) and its subsets viz CD4+Foxp3+ (Tregs), CD4+TNFα+ cells, CD4+IFNγ+ (Th1), CD4+IL-4+ (Th2) and CD4+IL-17+ (Th17) T-cells at 8 weeks post-surgery in mice treated with either vehicle or Compound 1 from 4 to at 8 weeks post-surgery are shown in Figure 25. The results indicate that treatment with Compound 1 lead to a decrease in various types of CD4+ Helper T-cells.

Quantitative group data for changes in left ventricular end-diastolic and end-systolic volumes (EDV and ESV) and ejection fraction (EF) in ligated mice before (4 weeks post-myocardial infarction) and after (8 weeks post-myocardial infarction) treatment with either vehicle or Compound 1 are shown in Figure 26. The results indicate that the change in the end-systolic volume, end-diastolic volume, and ejection fraction were significantly less for ligated mice treated with Compound 1 than for the control.

Herein, a drug molecule Compound 1 was tested for its ability to inhibit T-lyphocyte activation and proliferation during chronic heart failure. It was found that Compound 1 inhibited CD3/CD28 TCR mediated T-cell activation under *in-vitro* T-cell proliferation assays. Administration of Compound 1 to heart failure mice (from 4 weeks postmyocardial infarction) reduced CD4+ T-lymphocyte levels in the circulation and blunted progressive left ventricular remodeling measured at 8 weeks post-ligation.

### Example 2

Sustained and inappropriate inflammatory activation, as manifested by elevated levels of inflammatory cytokines such as tumor necrosis factor-α (TNF), contributes to disease progression in chronic heart failure (HF) (Ismahil et al. Circ Res. 2014; 114(2); 266-82). However, clinical trials of antibody-based anti-TNF therapies in heart failure failed to show clinical benefit (Anker et al. Int J Cardiol. 2002;86:123-30). This suggests that inflammatory mechanisms in heart failure, and, by analogy, the approaches to therapeutic immunomodulation, are more complex and nuanced than gradations of cytokine responses in failing myocardium. As plasma cytokine levels are often a combined result of the interplay between activated immune cells, they may be less sensitive indicators of the underlying tissue events that are specifically mediated by inflammatory and immune cells. Indeed, levels of circulating and cardiac inflammatory cells (monocytes (Nahrendorf et al. J Exp Med. 2007;204:3037-47), macrophages (Ismahil et al. Circ Res. 2014; 114(2); 266-82), and T-lymphocytes (Bansal et al. Circ Heart Fail. 2017;10:e003688)) are increased during both the acute and chronic phases of heart failure, necessitating spatio-temporal dissection and identification of specific molecular signatures that can be targeted to restrain activation of pathological immune cells to achieve therapeutic immune-modulation.

Recent studies have established complex interrelationships between innate (e.g., monocytes/macrophages and dendritic cells) and adaptive immune cells (e.g., T-lymphocytes) in the regulation of tissue remodeling (Ismahil et al. Circ Res. 2014; 114(2); 266-82). Both the acute and chronic phases of left ventricular (LV) remodeling are influenced by cardiac infiltration of dendritic cells (Ismahil et al. Circ Res. 2014; 114(2); 266-82), monocytes (Nahrendorf et al. J Exp Med. 2007;204:3037-47), macrophages (Ismahil et al. Circ Res. 2014; 114(2); 266-82 ), and CD4⁺ helper T-lymphocytes (Bansal et al. Circ Heart Fail. 2017;10:e003688). Importantly, in contrast to early (7 days) cardiac remodeling after infarction, increased levels of pro-inflammatory iNOS⁺ and TNF⁺ innate (Kingery et al. Basic Res Cardiol. 2017;112:19) and adaptive immune (Bansal et al. Circ Heart Fail. 2017;10:e003688) cells in chronic heart failure is indicative of complex pathological modulation of the global immune cell networks. Furthermore, adoptive transfer of splenocytes (Ismahil et al. Circ Res. 2014; 114(2); 266-82) or splenic CD4⁺ T-cells (Bansal et al. Circ Heart Fail. 2017;10:e003688) from heart failure mice induced significant left ventricular remodeling and cardiac dysfunction in naive mice, whereas splenectomy (Ismahil et al. Circ Res. 2014; 114(2); 266-82) or antibody-mediated depletion of CD4⁺ T-cells (Bansal et al. Circ Heart Fail. 2017;10:e003688) inhibited left ventricular remodeling and improved cardiac function in heart failure mice. These studies indicate that chronic ischemic heart failure is a state of global immune cell activation and expansion in the heart, blood, spleen, and lymph nodes (LNs). Several studies also show that estrogen receptors (ER) α and β are expressed on myeloid (monocytes/macrophages/dendritic cells) and lymphoid (B cells and T-cells) immune cells (Kovats S. Cell Immunol. 2015;294:63-9). Furthermore, ERβ activation inhibits TNFα mediated NF-kB translocation (Xing et al . PLoS One. 2012;7:e36890), blunts IFNα and iNOS expression (Kovats S. Cell Immunol. 2015;294:63-9), and suppresses T-cell mediated auto-immunity-critical regulators of left ventricular remodeling and heart failure (Aggelakopoulou et al. J Immunol. 2016; 196:4947-56). This heretofore-unappreciated role of ERβ activation on expansion and pro-inflammatory switching of immune cells raises the possibility of an approach to immunomodulation that specifically reverses this pathological and tissue-injurious phenotypic switching of immune cells during the progression of chronic heart failure.

Epidemiologic studies have shown that pre-menopausal women are more protected against cardiovascular disease (CVD) as compared to post-menopausal women (Hayward et al. Cardiovasc Res. 2000;46:28-49). Moreover, the incidence is much lower in women, in general, and is delayed by 10 years when compared with age-matched males (Wake et al. Recent Pat Cardiovasc Drug Discov. 2009;4:234-40). Males, as compared to females, have also been shown to have significantly more pathological cardiac remodeling with accentuated activation of fibrotic and inflammatory genes (Wake et al. Recent Pat Cardiovasc Drug Discov. 2009;4:234-40), suggesting a role for estrogen receptors (ERs) in mediating cardiovascular disease related inflammation.

Whereas, ERα levels are associated with maladaptive cardiac remodeling in human heart failure patients (Mahmoodzadeh et al. Faseb J. 2006;20:926-34), ERβ overexpression improves heart function and survival by reducing cardiac fibrosis (Pedram et al. Mol Cell Endocrinol. 2016;434:57-68). ERα mediated signaling regulates the production of type I IFNs in macrophages and dendritic cells and ERα^{-/-} immune cells produce significantly lower amounts of pro-inflammatory cytokines such as IL-6, IL-23, IL-12 and IL-1β (Kovats S. Cell Immunol. 2015;294:63-9). Notably, all of these cytokines have been shown to be increased in heart failure patients (Dubnika et al. Cytokine Growth Factor Rev. 2018). Activation of ERβ, on the other hand, has been shown to inhibit TGFβ synthesis and myofibroblastic transition from the fibroblasts and blunts the fibrotic events induced by Angiotensin II and endothelin-1 (Pedram et al. Mol Cell Endocrinol. 2016;434:57-68 ). Estradiol, via ERβ stimulation, also inhibits TNFα mediated activation of NF-kB (Xing et al . PLoS One. 2012;7:e36890) and inhibits iNOS production in peritoneal macrophages (Xiu-li et al. Mol Immunol. 2009;46:2413-8). A recent study has also shown that ERβ mediated signaling in CD4+ T-cells can be important for the suppression of autoimmune reactions in multiple sclerosis (Aggelakopoulou et al. J Immunol. 2016; 196:4947-56). Although, these studies provide preliminary evidence for the role of ERβ in regulating immune responses, whether and to what extent this happens in immune activation during heart failure is not known. In several published studies (Ismahil et al. Circ Res. 2014; 114(2); 266-82; Nahrendorf et al. J Exp Med. 2007;204:3037-47; Bansal et al. Circ Heart Fail. 2017;10:e003688; Kingery et al. Basic Res Cardiol. 2017;112:19; Yang et al. Circulation. 2006;114:2056-64), it has been shown that immune activation acutely after myocardial infarction (1-10 days) is protective in nature and mediates tissue repair. It has also been shown that chronic heart failure (4 to 8 weeks post-myocardial infarction) is associated with a 2nd wave of immune activation (Ismahil et al. Circ Res. 2014; 114(2); 266-82; Bansal et al. Circ Heart Fail. 2017;10:e003688; Kingery et al. Basic Res Cardiol. 2017;112:19), during which they undergo a phenotypic pro-inflammatory switch associated with increased TNFα and iNOS production and this phase coincides with the highest rates of fibrosis, hypertrophy, and maladaptive left ventricular remodeling. However, the molecular mechanisms that aid in this transition and the conditions that trigger immune cells to become pathological are not clearly defined.

Hence, it is hypothesized that selective ERβ activation can: inhibit TNFα mediated NF-kB translocation, ameliorate left ventricular remodeling, and improve cardiac function by deterring the activation of pathological immune cells. It is further suggested that ERβ stimulation can represent a cellular target for therapeutic immunomodulation in heart failure.

Proposed studies will identify therapeutic indications for Compound 1 as a selective immune-modulator to curb sustained immune activation associated with chronic heart failure. ERβ activation on immune cells can potentially reverse the proinflammatory phenotype of immune cells which in turn can prevent pathological left ventricular remodeling and progressive cardiac dysfunction in heart failure patients. Despite widespread understanding that heart failure is a state of chronic inflammation; no large-scale safe immunomodulatory therapies for heart failure have yet been successfully translated to clinical practice. To date, attempts at therapeutic immunomodulation in heart failure have primarily focused on protein mediators such as inflammatory cytokines. Proposed herein is a paradigm for immune-modulation in which leukocytes primed against the heart are targeted to reverse their proinflammatory phenotype using a selective ERβ agonist rather than cytokine mediators.

Aim 1: To delineate spatio-temporal changes in ERα and ERβ expression on immune cells during ischemic heart failure.

Rationale. Recent studies have defined the sequence of events that lead to activation, and infiltration of systemic and tissue-resident innate and adaptive immune cells during the early (Nahrendorf et al. J Exp Med. 2007;204:3037-47) and late stages of post-myocardial infarction left ventricular remodeling (Ismahil et al. Circ Res. 2014; 114(2); 266-82; Epelman et al. Immunity. 2014;40:91-104; Heidt et al. Circ Res. 2014;115:284-95; Lavine et al. Proc Natl Acad Sci U S A. 2014;111:16029-34). Several studies have also shown that ERs are constitutively expressed on the immune cells of myeloid (monocytes/macrophages) and lymphoid origin (B and T lymphocytes) (Kovats S. Cell Immunol. 2015;294:63-9; Aggelakopoulou et al. J Immunol. 2016;196:4947-56). However, detailed spatio-temporal alterations of ERs on the immune cell populations during ischemic heart failure has not been performed. Hence, the working hypothesis herein is that immune cells progressively shift the expression of ERs by reducing ERβ levels to effect persistent global inflammatory activation (e.g., heightened systemic TNF elaboration) during the evolution of chronic heart failure. As the spatiotemporal kinetics of this switch are unknown, in Aim 1 the activation, proliferation, and pro-inflammatory phenotype of immune cells (monocytes/macrophages and T-cells) in PB, spleen, mediastinal LNs and the heart at 1, 2, 4 and 8 weeks post-myocardial infarction will be characterized (Figure 28). More specifically, ERα and ERβ expression will be profiled on monocytes/macrophages and CD4⁺ T-cells in the heart, blood, spleen, and mediastinal LNs by flow cytometry and/or immunohistochemistry at 1, 2, 4, and 8 weeks after coronary ligation in mice as compared with sham operated controls. TNFα and nuclear factor (NF)-κB p65 will be used to index pro-inflammatory signaling, and will also index immune cell proliferation. Using human heart failure samples, the expression of ERs on circulating immune cells in ambulatory patients with systolic heart failure versus matched non-failing controls will be profiled.

Aim 2: To establish the protective role of immune cell specific ERβ on left ventricular remodeling and chronic heart failure

Rationale. ERβ plays an obligatory role in the biology of immune cell by regulating their activation, and the expression of pro-inflammatory cytokines such as TNFα (Xing et al . PLoS One. 2012;7:e36890) and iNOS (Xiu-li et al. Mol Immunol. 2009;46:2413-8). Furthermore, ERβ activation has also been shown to suppress immune activation in auto-immune diseases (Aggelakopoulou et al. J Immunol. 2016;196:4947-56). Since heart failure is also characterized by autoimmune reactions, treatment with a selective ERβ agonist can provide therapeutic benefits by inhibiting pathological left ventricular remodeling mediated by auto-immune reactions. Therefore, Compound 1 will be injected (i.p.) daily in the heart failure mice from 4 to 8 weeks post-myocardial infarction (Figure 29). This time-point was chosen as previous studies have shown that immune cells undergo a pathological phenotypic switch at around 4 weeks post-myocardial infarction (Bansal et al. Circ Heart Fail. 2017;10:e003688). Cardiac function will be measured before (4 weeks post-myocardial infarction) and after the treatment (8 weeks post-myocardial infarction) using echocardiography and monocytes/macrophages and t-cells will be profiled using flow cytometry. The cardiac function will be evaluated before (4 weeks post-myocardial infarction) and after the treatment (8 weeks post-myocardial infarction), changes in systemic/cardiac immune cell profiles will be evaluated, and left ventricular remodeling (hypertrophy, apoptosis, fibrosis, and capillary rarefaction) during chronic heart failure will be evaluated. Several parameters of left ventricular remodeling, such as myocyte hypertrophy (wheat-germ agglutinin staining), fibrosis (masson trichrome staining), myocyte apoptosis (TUNEL staining) and capillary rarefaction (isolectin staining), and ERβ signaling in isolated cardiac and splenic immune cells, will also be evaluated.

Since ERβ is also expressed on myocytes, these studies will be repeated in bone-marrow (BM) chimera mice. Wild type mice will be lethally irradiated and reconstituted with the BM from the ERP^{-/-} mice to specifically deplete ERβ from the immune cells. It is expected that the cardio-protective effects of ERβ activation will not be observed in these mice, which can definitively prove an obligatory role of ERβ receptor signaling in immune cell activation during chronic heart failure.

These studies investigating the importance of ERβ on immune cells in the pathogenesis of left ventricular remodeling and chronic ischemic heart failure will thereby further the understanding of the cellular basis for inflammation in this disease. Moreover, by providing direct evidence for the protective role of ERβ in inhibiting cardiac specific immune cell activation, therapeutic indications for ERβ agonist for immunomodulation in heart failure can be identified.

### Example 3

**Methods.** Animal studies were approved by the Institutional Animal Care and Use Committee at the Ohio State University and were done in accordance with the NIH Guide for the care and Use of Laboratory Animals (DHHS publication No. 85-23, revised 1996). All mice had free access to food and water *ad-libitum* and a total of 138 mice were used for all the studies.

**Mouse Model, Surgical Protocol and Drug Treatment.** Male, 10 to 12-week-old C57BL/6 mice (Jackson Laboratories, stock# 000664) underwent left thoracotomy followed by permanent left coronary artery ligation to induce MI and ischemic HF (n=80) or sham surgery (n=28), as described previously. At 8 weeks post MI, peripheral blood was collected from the facial vein and the mice were euthanized by cervical dislocation. Hearts and spleens were collected, weighed, and processed either for mononuclear cell isolation or histological analysis. Tibia length was measured to normalize all gravimetric data.

A 20-fold stock solution of compound 1 was prepared in DMSO and stored at -20 °C. At the time of dosing, the drug was diluted using an equal volume of tween 20 and saline maintaining a ratio of 5:5:90 (DMSO:Tween 20:saline). All mice were weighed every day and the drug was administered at 60 mg/kg dose (200 µL per 25 g body weight) *via* gavage.

**Echocardiography.** Echocardiography was conducted under 1-1.5% isoflurane anesthesia using a VisualSonics Vevo 3100 and body temperature was maintained using an adjustable heated rail system (Vevo Imaging Station) and RMV707B scanhead as previously described.

**Immune Cell Isolation and Fixation.** Immune cells were isolated from the spleens, blood, and hearts, as described previously. Briefly, spleens were triturated using the plunger of a 3-mL syringe in sterile PBS supplemented with 2% BSA and 2 mM EDTA to release all splenocytes and were filtered through 40 µm cell strainers to remove connective tissue. Peripheral blood (100 µL) was collected from cheek veins in BD microtainer tubes containing EDTA, and RBCs were lysed using lysis buffer (eBioScience). Hearts were finely minced, digested using collagenase II (1 mg/mL) to obtain single cell suspensions, and filtered through 40 µm cell strainers. Cells from the tissues were pelleted by centrifugation at 500 g, re-suspended in 100 µL PBS supplemented with 2% BSA and 2 mM EDTA, fixed using 100 µL of 1% w/v PFA, and stored at 4 °C for flow cytometric staining.

**T-cell Proliferation Assays.** Splenic CD4+ T cells were magnetically purified using automated RoboSep^{™} cell separation system from Stemcell technologies and MojoSort Mouse CD4⁺ T-cell isolation kit (BioLegend), as per manufacturer's instructions. Purified live cells were counted using trypan blue exclusion assay, reconstituted at a concentration of 1x10⁶ cells/mL in sterile PBS and were incubated at 37 °C in the dark with an equal volume of 4 µM Tag-it Violet^{™} (BioLegend) for 10 min. Excess dye was quenched by adding complete RPMI media (supplemented with 10% charcoal stripped FBS to endogenous hormones and 1% P/S/G) (Gibco) at five times the volume of the dye solution used for staining, and incubating for 5 min on ice. Labeled cells were pelleted by centrifugation at 500 g for 5 min and re-suspended in complete RPMI media at a concentration of 1x10⁶ cells/mL for all assays.

Flat bottom 96-well plates (FisherSci) were incubated with 50 µL of a 4.5 mg/mL solution of Anti-Hamster IgG (MilliporeSigma) at room temperature. After 1 h, wells were washed with sterile PBS to remove excess antibody, and were coated with 50 µL of rat anti-mouse CD3 antibody (BioLegend) at a concentration of 2 µg/mL for 1 h at room temperature. Non-stimulated control wells were incubated only with sterile PBS. All the wells were washed with sterile PBS to remove excess antibody followed by plating of 1x10⁵ Tag-it Violet^{™} dye labeled CD4⁺ T-cells in each well. Co-stimulation was effected by adding 100 µL of complete RPMI media containing 4 µg/mL of Rat anti-mouse CD28 antibody (BioLegend) while 100 µL of complete RPMI media (without anti-CD28 antibody) was added to non-stimulated wells. Cells were incubated for 72 h at 37 °C and 5% CO₂ and dye dilution with each successive cell division was measured either by Becton Dickinson LSRFortessa or NL3000 Northern Lights (Cytek^{®}) flow cytometer.

**Flow Cytometric Staining.** Detailed protocol for cell staining has been described previously. Briefly, cell pellets were re-suspended, aliquoted into flow tubes and incubated on ice with a cocktail of extracellular rat anti-mouse antibodies for 45 min. Cells were washed with PBS supplemented with 2% BSA and 2 mM EDTA and fixed using 1% PFA. For intracellular staining, cells were permeabilized using 0.5% v/v tween-20 and incubated with a cocktail of antibodies (on ice) for 45 min. Anti-mouse CD4 SB600/PE-Cy7, Foxp3-APC, IFNγ eFluor 450, Ly6C eFluor 450 and ERβ antibodies were from ThermoFisher Scientific; CD8 APC-Cy7, CD11b APC-Cy7, and CD19 PerCP-Cy5.5 antibodies were from Tonbo Biosciences; IL-17 AF700, TNFα FITC, CD11b-APC, CD69 PerCP, Ly6G PE and CD69 AF700 antibodies were from Biolegend and ERα PE antibody was from Abcam.

**Hypertrophy (WGA) Staining.** Formalin-fixed, paraffin-embedded hearts were sectioned (5 µm thickness), deparaffinized, rehydrated, and stained as described previously. Masson's trichrome staining was used to quantify tissue fibrosis while Alexa Fluor 488-conjugated wheat germ agglutinin (ThermoFisher Scientific) was used to label cellular membranes. Myocyte area was quantified in the remote zone of failing hearts from 3 to 4 high-power fields per section.

**Statistical Analysis.** All data are shown as mean±SD. Unpaired student's 'T' test with equal or unequal variance was used to compare 2-groups while 1-way or 2-way Anova with Tukey's post-hoc test was used for comparing more than 2 groups. In some cases, 1-way Anova with correction for multiple comparisons by controlling the false discovery rate was used. GraphPad Prism version 9.0 was used for all statistical analyses and a P value of <0.05 was considered significant.

### ERα Signaling is Upregulated in CD4⁺ T-cells during Ischemic HF

In previous studies, it was shown that, in contrast to their wound-healing properties during myocardial infarction (MI), CD4+ T-cells undergo a pathological phenotypic shift during chronic heart failure (HF), and accentuate left ventricular (LV) remodeling and cardiac dysfunction. Therefore, to identify potential phenotypic switches, CD4+ T-cells (150-300) were sorted from the failing hearts & the mediastinal lymph nodes of the male mice at 8 wks post-MI and limited cell RNA-sequencing was conducted (Figure 30). IPA analysis of the differentially expressed genes showed that downstream of SIRT1, ESR1 (ERα) signaling was significantly upregulated in cardiac CD4⁺ T-cells as compared to the mediastinal lymph nodes of HF mice and several genes downstream of ESR1 signaling were altered (Figure 31). This was highly interesting as these T cells were sorted from male failing hearts. To further validate these findings, the gene expression of ERα and ERβ in the remote zone-LV and splenic mononuclear cells from the HF mice was measured. As shown in Figure 32, while both ERα and ERβ were increased in the hearts, only ERα was increased in the splenic immune cells.

Several studies in other autoimmune diseases, such as multiple sclerosis (MS) and experimental autoimmune encephalomyelitis (EAE), have shown the role of estrogen receptors (ERs) in modulating T-cell activity. However, these studies are mostly done using ovariectomized female mice and very limited work has been done in male mice. Therefore, steady-state expression of ERα and ERβ was measured in the ovaries (as a positive control), hearts of male and female mice, and spleens of naive male mice. As shown in Figure 33, while ERα expression in the hearts and spleens was comparable; ERβ expression was ~11-fold higher in the spleen as compared to the heart. Splenic CD4⁺ T-cells from the naive male mice were then magnetically sorted and ER expression in them was measured. Interestingly, ERβ expression in splenic CD4+ T-cells was 10-fold lower as compared to ERα (Figure 34), suggesting that ERα signaling is predominant in CD4+ T-cells and that other splenic immune cells express significantly higher levels of ERβ as compared to CD4+ T-cells. This was consistent with other studies showing highest expression of ERα in T-cells isolated from the human PBMCs. Further analysis of ERβ expression in different circulating and splenic immune cells showed this to be the case, as cells of the myeloid origin (such as Ly6G+ neutrophils & Ly6C+ monocytes) expressed much higher ERβ as compared to CD4+ T-cells, while CD19+ B-lymphocytes had comparable expression (Figure 35 and Figure 36).

### ERα is Temporally Modulated while ERβ Expression is Spatially Altered in CD4⁺ T-cells during Ischemic HF.

Epidemiological (in premenopausal women) and preclinical studies indicate protective effects of ER signaling in cardiovascular diseases. However, given the pathological role of T-cells identified in previous studies, it is not known what role, if any, ER signaling plays in modulating CD4⁺ T-cell polarization/activation in the context of MI and HF. Since 3 days (d) post-MI marks the peak inflammatory response and 8 weeks (w) post-MI exhibits significant LV remodeling with pathological transitioning of CD4⁺ T-cells, ERα and ERβ expression in CD4⁺ T-cells was measured at these time-points. As shown in Figure 37 and Figure 38, ERα expression was significantly decreased at 3 d post-MI, but was significantly increased at 8 w, which was consistent with 1c-RNA seq data showing the activation of ERα pathway at this time-point. ERβ expression, on the other hand, did not change at either of the time-points when compared with the sham mice (Figure 39). Upon further analysis of spatial changes in ERβ expression, it was found that, despite low ERβ expression in spleen (Figure 40 and Figure 41), CD4+ T-cells infiltrating into the failing hearts had higher expression of ERβ as compared to other cells of myeloid, such as Ly6G+ neutrophils or Ly6C⁺ monocytes/macrophages, or lymphoid origin, such as CD19⁺ B-cells (Figure 42). Moreover, at 3d post-MI, ERβ expression in cardiac CD4⁺ T-cells was much higher as compared to circulating or splenic T-cells, and was significantly lower at 8 weeks post-MI (Figure 43 and Figure 44). These changes were not due to differences in tissue background or differential non-specific tissue binding between splenic or cardiac single cell preparations, as B-lymphocytes (comparable in size and ERβ expression in the naive mice (Figure 36)) did not exhibit this change in ERβ expression even at 3d post-MI (Figure 45), the peak of acute inflammatory response post-MI. These data show that: 1) ERα expression is temporally regulated and it's high levels coincide with pathological transitioning of T-cells, 2) ERβ expression is significantly and sustainably increased in cardiac CD4⁺ T-cells when compared with other immune cells post-ischemic injury, 3) ERβ is spatially modulated, and its expression in CD4⁺ T-cells intensifies upon infiltration into the injured hearts at 3d post-MI but declines at 8 wks post-MI when compared with other tissues, and, last but not the least, 4) ERβ activation to constrain ERα signaling could be a potential target to blunt pathological transitioning of CD4+ T-cells.

### ERβ agonist Dose Dependently Inhibits Anti-CD3/CD28 Mediated T-cell Proliferation

ERα signaling is antagonized by ERβ agonism. Thus, an ERβ agonist, Compound 1, a lipophilic and orally bioavailable compound with ~200-fold selectivity for ERβ as compared to ERα was identified. This compound was tested for its ability to inhibit TCR (anti-CD3/CD28) mediated proliferation of CD4⁺ T-cells isolated from the spleens of naive male mice. As shown in Figure 46, compound 1 dose-dependently inhibited TCR mediated T-cell proliferation with an IC₅₀ of 3.4 µM. Importantly, while the drug (5 µM) did not affect non-stimulated CD4⁺ T cells (Figure 47), it significantly inhibited the proliferation of TCR activated CD4⁺ T-cells even in the presence of low and high estradiol concentrations (Figure 48 and Figure 49). When compared with non-stimulated T-cells, there was an increasing trend in the frequency of live cells upon TCR stimulation, presumably due to proliferation of live cells (Figure 50). However, in the TCR stimulated T-cells treated with the 5 µM drug (Figure 50 and Figure 54), no change was seen in the frequency of live cells as compared to the non-stimulated cells, suggesting that the reduction in T-cell proliferation was not due to increased cell-death. Using this concentration, the effects of ERβ agonism on the expression of pro-inflammatory cytokines such as TNFα and IFN-γ was also tested. As compared to non-stimulated T-cells, TCR stimulation significantly increased TNFα (Figure 51 and Figure 52) and IFNγ (Figure 53) expression both in the absence as well as in the presence of estradiol, which was significantly inhibited by the drug. There was no change in either the TNFα or IFNγ expression in the non-stimulated cells treated with the drug in the presence or absence of estradiol (Figure 54 and Figure 55), suggesting that the inhibition of cytokine expression was specific to T-cell activation and does not affect homeostatic expression of these cytokines. A significant reduction in the frequency of CD69 expressing CD4⁺ helper T-cells upon treatment with 5 µM compound 1 was also observed (Figure 56), suggesting blunting of T-cell activation as well. Similar effects were observed with CD4+ T-cells isolated from the female mice (Figure 57 - Figure 60), suggesting that, although the drug was specific for ERβ, the effects were not gender-specific, and T cells from both male and female mice were equally inhibited. This also indicates that the role of estradiol and ERs in T-cell activation is ubiquitous and TCR signaling through this pathway is not dependent upon the gender.

To determine if the inhibitory effects of compound 1 were against a specific activation mechanism or is non-specific in nature, the drug was also tested in the presence of PMA & Ionomycin (Figure 61 - Figure 64). These agents activate T-cells by increasing intracellular Ca²⁺ and PKC activity, and bypasses TCR activation. PMA/Ionomycin treatment significantly increased the expression of pro-inflammatory cytokines, TNFα and IFNγ, and activation marker CD69 in T-cells (Figure 61 - Figure 64). Interestingly, none of these pro-inflammatory and activation markers were inhibited by compound 1. These results suggest that compound 1 selectively inhibits TCR mediated T-cell proliferation while sparing other activation mechanisms.

### Compound 1 Activates ERβ but Inhibits ERα Signaling

To further investigate the specificity of the drug, RNA was isolated from anti-CD3/CD28 stimulated CD4⁺ T-cells cultured with and without 5 µM drug. Principal component analysis (Figure 65) showed that the RNA transcriptome of TCR activated CD4⁺ T-cells was significantly altered in the presence of 5 µM compound 1 as compared to the stimulated cells treated with the vehicle. Several genes were identified that were either downregulated or upregulated (>2-fold and p<0.01) in the presence of drug (Figure 66). Several genes involved in ERβ pathway were upregulated while some were downregulated (Figure 67 and Figure 68), leading to an overall activation of this pathway with concomitant inhibition of ERα signaling. Downregulation of several genes involved in TCR activation was also observed, further supporting TCR specificity of compound 1. The effects of ERβ agonism on other pathways required for immune activation were further assessed. Analysis of upregulated and downregulated gene transcripts showed that several pathways involved in inflammation, immune activation, and metabolism were either inhibited or negatively-affected by the compound 1 (Figure 69 and Figure 70).

### Compound 1 Treatment Specifically during Chronic HF Ameliorates Cardiac Remodeling

Considering that CD4+ T-cells are critical for would healing during initial 10-14 days post-MI but are pathological and dispensable during chronic HF, the efficacy of the drug at both phases was tested. For acute-MI, using echocardiography, akinetic area at 7 d post-MI was measured and mice were randomized to either receive vehicle or 60 mg/kg compound 1 (summarized in Figure 71). Daily administration of the drug *via* gavage did not affect the body weight of infarcted or sham mice (Figure 72), suggesting that the drug did not have any overt side-effects on rodent physiology. However, a significantly increased mortality was observed with drug treatment, as several mice (~60%) died during the first week of treatment when compared with the vehicle control (15-20%) (Figure 73). This, consistent with other studies, underscores the vital and protective role of CD4⁺ T-cells for adequate wound-healing post-MI.

For chronic HF, cardiac function was measured at 4 w post-MI and all animals were randomized according to the degree of cardiac dysfunction (Figure 74), reflected by end-systolic and end-diastolic volumes (ESV and EDV), and ejection fraction (EF), to either receive vehicle or 60 mg/kg drug, daily, *via* gavage, for 4 weeks. No drug related mortality nor any changes in the body weight were observed (Figure 75) during this time. Importantly, echocardiography data at 8 w post-MI (4 w post-treatment) showed that the cardiac dysfunction in vehicle-treated HF mice progressed during this time with increased end-systolic and end-diastolic volumes (ESV and EDV), and reduced ejection fraction (Figure 76 and Figure 77). In contrast, the cardiac function of compound 1 treated mice did not worsen, and the ESV, EDV, and EF did not change over 4 weeks of drug treatment. The cardiac function in drug treated mice was significantly better than the vehicle treated mice at 8 w post-MI. These differences in cardiac function were not due to heart rate, as the mean heart rate was more than 480 BPM at both time-points and was comparable between both groups (Figure 78).

### Compound 1 Treatment Ameliorates Cardiac Hypertrophy

Gravimetric analysis of heart and LV weights showed that, while vehicle treated HF mice exhibited a significant increase in tibia normalized heart and LV weights, drug treatment from 4 to 8 w inhibited cardiac hypertrophy as reflected by significantly reduced heart (Figure 79) and LV (Figure 80) weights when compared with the vehicle treated HF mice. To further validate these results, WGA staining was conducted and cardiomyocyte area in LVs was measured. A shown in Figure 81 and Figure 82, drug treatment significantly decreased cardiomyocyte area and hypertrophy when compared with the vehicle treated HF mice. A significant decrease in the gene expression of several hypertrophy markers (such as Gja1, Gja5, MyH7, and MyH6) was also observed, while some others (such as BNP and RyR2) showed a decreasing trend (Figure 83 and Figure 84). This was also supported by RNA seq data obtained from drug treated CD4⁺ T-cells, as upon retrospective analysis it was observed that several signaling pathways in T-cells that mediate cardiac hypertrophy were downregulated with compound 1 treatment.

### Compound 1 Treatment Specifically Depletes CD4+ Helper T-cells in HF mice

Using flow cytometry, CD4⁺ T-cells were measured in the circulation, failing hearts, and spleens of vehicle- and drug-treated mice at 8 w post-MI (4 w post-treatment). As shown in Figure 85, daily treatments with the compound 1 significantly decreased circulating CD4⁺ T-cells at 8 w. Reduction in T-cell numbers was not limited to a particular helper T-cell subset, as numbers for both the pro- and anti-inflammatory cells, viz CD4⁺TNFα⁺, CD4⁺FoxP3⁺ Tregs, CD4⁺IFNγ⁺ Th1 T-cells, CD4⁺IL-4⁺ Th2 T-cells, and CD4⁺IL-17⁺ Th17 T-cells were decreased significantly. Similarly, a significant decrease in cardiac CD4⁺ T-cells in compound 1 treated HF mice was also observed when compared with the vehicle-treated mice (Figure 86 and Figure 87). This decrease was reflected in the significant decline of both TNFα⁺ and IFNγ⁺ cells, suggesting inhibition of cytokine production in CD4⁺ T-cells, consistent with *in-vitro* T-cell inhibition assays. A similar decrease in CD4⁺ T-cell numbers (and frequency) was also observed in splenic CD4⁺ T-cells (Figure 88 and Figure 89), including diminished counts of FoxP3⁺ Tregs (Figure 90 and Figure 91). Interestingly, the overall frequency of Foxp3+ Tregs was increased in CD4+ T-cells (Figure 92), suggesting that the other Th subsets were decreased more than the FoxP3+ Tregs. Since Foxp3 expression has been found to directly correlate with the immune-suppressive capacity of Tregs, its expression was also measured in vehicle and drug-treated mice. As shown in Figure 93, drug treatment significantly increased FoxP3 expression (reflected as MFI) in Tregs, suggesting that the FoxP3+ Tregs remaining after the drug treatment were significantly potent and immune-suppressive. This was an interesting finding, as it was previously shown that Tregs undergo a pro-inflammatory (and pathological) phenotypic switch with loss of their immune-suppressive potential during chronic HF. Increased FoxP3 expression with compound 1 treatment, thus, indicates improved competence of Tregs in suppressing pathological immune activation during chronic HF.

ERβ expression is stabilized/increased in splenic T-cells with compound 1 treatment (Figure 94).

To determine the effects of compound 1 on other immune cells, also known to play a key role in LV remodeling during chronic HF, circulating, splenic, and cardiac levels of other immune cells of myeloid and lymphoid origin were measured. As shown in Figure 95 - Figure97, compound 1 did not alter the frequency of either the myeloid cells (CD11b⁺) such as Ly6G⁺ neutrophils or Ly6C⁺ monocytes/macrophages, or any other lymphocyte populations, such as CD19⁺ B-cells or CD8⁺ cytotoxic T-cells in the hearts (Figure 95), blood (Figure 96) or spleens (Figure 97) of HF mice, suggesting highly specific effects of compound 1 on the CD4⁺ Helper T-cells.

The effect of compound 1 on thymic cellularity and T-cell development was also investigated ( Figure 98 - Figure 100).

### Discussion

Several findings were demonstrated in this study. First, ERα signaling is significantly upregulated in CD4⁺ T-cells during chronic HF. Second, while ERα expression is temporally regulated to significantly decrease during MI and increase during chronic HF, ERβ expression is spatially regulated and is increased specifically in T-cells infiltrated into the ischemic and failing hearts as compared to other cardiac immune cells. Third, ERβ agonists dose dependently inhibit CD4⁺ T-cell activation, proliferation, and the expression of pro-inflammatory cytokines both in males as well females to a similar extent. More importantly, this inhibition is specific to antigenic TCR-activation pathway. Fourth, ERβ agonists, if given early after MI, result in increased morbidity and mortality but improve cardiac function and reduce cellular hypertrophy if given during chronic HF. This underscores the critical and protective role of CD4⁺ T-cells in mediating wound-healing and scar formation post-MI but a pathological role during chronic HF, and points to significant immunological differences between the two, at least from the perspective of adaptive immunity. Fifth, ERβ agonists can selectively blunt CD4⁺ T-cell levels in the failing hearts, lymphoid tissues, and circulation without affecting other immune cells, suggesting that they can be developed as highly specific therapeutics for temporal modulation of pathological CD4⁺ T-cells without affecting other protective immune responses.

Canonical ER signaling is mediated either by binding of liganded ERs on EREs of target genes or indirectly by binding of (un)liganded ERs with other transcription factors such as AP-1 and NF-kB, to regulate activation, polarization, and proliferation of T-cells. ERα signaling is essential for: i) CD4+ T-cell activation against exogenous antigens, ii) polarization into IFNγ producing Th1 T-cells, and iii) trafficking and migration of activated CD4+ T-cells into tissues by regulating the expression of chemokine receptors such as CCR1, CCR2, CCR3, CCR4 and CCR5. In a limited cohort of human patients, it has been shown that systemic estradiol levels are increased during MI (first 3 days of hospitalization), are positively correlated with serum creatine phosphokinase levels, and very high estradiol levels are associated with increased mortality in the MI patients. Along the same lines, Th1/Th2 T-cell ratios have also been shown to increase in STEMI patients and are associated with increased adverse events. Together, these studies would suggest a pathological role of excessive estradiol mediated ERα signaling and Th1 T-cell polarization. However, preclinical studies have also shown that CD4+ T-cell activation during MI is protective and necessary for adequate healing and scar formation. The findings herein are consistent with these seemingly contrasting studies and underscore the importance of decreased ERα signaling in CD4⁺ T-cells at 3d post-MI to diminish non-antigenic T-cell activation and initiate regulated Th1 polarization. Nonetheless, these protective responses are considerably compromised during chronic HF. ERα levels and its downstream signaling is significantly upregulated to effect their antigen-dependent activation and pathological transitioning of CD4⁺ T-cells at 8 weeks post-MI, as has been previously shown. The data herein also shows that both during acute-MI and HF, CD4⁺ T-cells infiltrated into the ischemic and failing hearts had higher ERβ expression as compared to other immune cells of myeloid and lymphoid origin. This is highly intriguing considering the fact that HIF1α activation is known to increase ERβ signaling which, in turn, attenuates transcriptional activity of HIF1α. ERβ is also known to enhance integrin α1 and β1 expression in tumor cells to augment vinculin mediated adhesive potential, cellular motility and transmigration. Increased ERβ levels selectively in cardiac T-cells would, therefore, indicate its involvement either in i) regulating T-cell activation by antagonizing ERα, ii) amplifying integrin-mediated T-cell transmigration into the hearts or waning their exit, or iii) regulating HIF1α activation in T-cells to control ischemic damage.

Preclinical studies in pressure-overload and ischemic HF models have shown that T-cell activation during HF is antigen-dependent and is mediated by the TCR activation. It is, therefore, important that immuno-modulatory strategies targeting CD4+ T-cells specifically diminish TCR mediated T-cell activation to avoid general immune-suppression and infections by opportunistic pathogens. ER pathway is, thus, of significant therapeutic potential as ERα gene depletion from CD4+ T-cells result in defective TCR mediated T-cell activation by decreasing NFAT1, Zap70, and STATS levels. This defective TCR signaling could either be a direct consequence of lost ERα signaling or it could also be due to amplified ERβ signaling in the absence of ERα. The studies herein show latter to be the case, as the ERβ agonist dose-dependently inhibited the proliferation of TCR activated CD4⁺ T-cells in a gender-independent manner. ERα also regulates the gene expression of several T- cell specific cytokines (such as IFNγ, TNFα, IL-4, and IL-17), chemokine receptors (such as CCR1, CCR2, CCR3, CCR4 and CCR5), and other transcription factors (such as NF-kB and NFAT), all of which have been implicated in auto-immune diseases, including HF. Although, in the RNA sequencing data, no significant changes were observed in the expression of chemokines, compound 1 effectively inhibited expression of pro-inflammatory cytokines induced by TCR activation but failed to do so when PMA/Ionomycin was used as the stimulus. These findings suggest that ERs are not merely the accessory pathways to regulate the transcription and expression of these pro-inflammatory cytokines/activation markers, but are direct downstream regulators of TCR specific signaling.

ER signaling is a key pathway in promoting CD4+ T cell activation in autoimmune diseases such as MS, EAE, and Asthma. While high estradiol concentrations and ERα mediated Th1 polarization exacerbates MS; Th2 T cells promote EAE, suggesting that excessive polarization of T cells either to pro- or anti-inflammatory subsets disrupts the steady-state and can be pathological. It has also been shown that hearts form ERα^{-/-} mice are more prone to ventricular fibrillations and tachycardia, and exhibit increased cell death and reduced contractility. Alternatively, ERα agonists, but not ERβ, if given before I/R reduce infarct-size in female rabbits. This, in conjunction with the fact that whole body ERP^{-/-} female mice exhibit increased mortality post-MI, suggest that ERα activation at the time of ischemic injury is cardio-protective and loss of ERβ is disrupts cardiac healing. Although, the cellular responders of protective ERα and ERβ signaling were not identified in these studies, others have shown that cardiac ERα or ERβ expression is not required for estradiol mediated cardio-protection. Along the same lines, it was previously shown that chronic HF is associated with a 2^{nd} wave of CD4⁺ T-cell activation, reflected as global increase in both pro- and anti-inflammatory helper T-cell subsets, in the circulation, spleen, mediastinal lymph nodes, and the failing hearts. Moreover, these T-cells exhibit a pathological phenotype and promote LV remodeling and progressive cardiac dysfunction as their depletion specifically from 4 to 8 weeks post-MI blunts progressive increases in ESV and EDV, and improves cardiac function. In contrast, studies by others have shown that CD4^{-/-} mice exhibit accentuated chamber dilatation and LV remodeling post-MI, suggesting their protective roles in mediating wound-healing, neovascularization, and fibrotic scar formation. These contradictory findings suggest that the CD4⁺ T-cells activated immediately after ischemic injury, as in MI, are immunologically and phenotypically different than those activated during chronic HF. However, the signaling mechanisms that mediate this transition from being protective during MI to pathological during HF are unknown. In these studies, it was observed that compound 1 mediated ERβ activation and CD4+ T-cell depletion at 7d post-MI resulted in significantly increased mortality but blunted LV remodeling and improved cardiac function upon administration from 4 to 8 weeks post-MI. These studies, for the first time, show that ER signaling could be one of important pathological phenotypic switch in CD4+ T-cells and selective ERβ agonists could provide a therapeutic immunomodulatory tool to inhibit ERα and blunt CD4⁺ T-cell activation and proliferation during HF. These results suggest that a balance of ERα and ERβ signaling in immune cells is critical during ischemic injury, further underscoring the key role of immune activation early after MI, and importance of identifying time-dependent changes in immune cells to determine optimal therapeutic window for temporal immune-modulation.

Expression of ERs is not restricted only to CD4+ T-cells and other immune cells, such as macrophages, neutrophils, dendritic cells, B-cells, and CD8+ T-cells, also express significant levels of these transcription factors. In monocytes and macrophages, estradiol exerts receptor dependent effects. While it promotes phagocytosis and pro-resolution *via* ERβ, it induces iNOS and, decreases Arg1 and IL-10 expression *via* ERα. In neutrophils, estradiol delays cell apoptosis and promotes netosis. In B-cells, estradiol mediated ERα signaling magnifies activation and inhibits apoptosis. Despite this widespread expression, compound 1 selectively inhibited CD4+ T-cells and did not affect other immune cells either in the circulation, spleen, or failing hearts. This specificity could be due to several factors. First, it could be due to differences in steady-state vs injury mediated activation of ERβ signaling. During steady-state, ERβ levels in CD4+ T-cells are significantly lower than the myeloid cells. However, during ischemic injury, significant amplification of ERβ expression selectively in cardiac CD4+ T-cells was observed, which was even higher than the myeloid cells, suggesting an important role of this pathway in mediating T-cells dependent protective responses. Second, differences in disease etiologies may activate disparate pathways in different immune cells. While myeloid cells activate ER signaling during trauma/hemorrhage; CD4+ T-cells probably activate this pathway specifically during autoimmune (ischemic/non-ischemic) injury. Specificity of the ERβ agonist only against the TCR mediated T-cell activation further supports this. Third, while ER signaling alters both activation/function and proliferation of CD4+ T-cells; it only regulates functional competency of other immune cells such as phagocytosis in monocyte/macrophages and netosis in neutrophils without altering their cell numbers.

## Claims

1. A carborane for use in a method for treating or preventing chronic heart failure in a subject following myocardial infarction, the method comprising administering to the subject the carborane during a maladaptive remodeling phase following the myocardial infarction, and wherein the carborane is selected from the group consisting of: 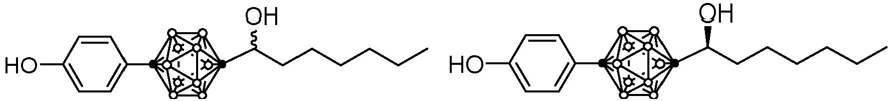 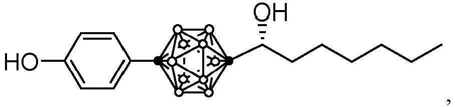 and pharmaceutically acceptable salts thereof.

2. The carborane for use of claim 1, wherein the method comprises treating or preventing chronic heart failure in a subject following myocardial infarction and the carborane is not administered to the subject during a healing phase or a repair phase preceding the maladaptive remodeling phase.

3. The carborane for use of any of claims 1-2, wherein the method comprises treating or preventing chronic heart failure in a subject following myocardial infarction and administration of the carborane commences at least 10 days following the myocardial infarction, such as at least 14 days following the myocardial infarction, at least 21 days following the myocardial infarction, at least 28 days following the myocardial infarction, at least 35 days following myocardial infarction, at least 42 days following myocardial infarction, at least 49 days following myocardial infarction, or at least 56 days following myocardial infarction.

4. The carborane for use of any of claims 1-3, wherein the method comprises treating or preventing chronic heart failure in a subject following myocardial infarction and the method further comprises assessing the subject to determine whether the subject has entered the maladaptive remodeling phase.

5. The carborane for use of claim 4, wherein assessing the subject to determine whether the subject has entered the maladaptive remodeling phase comprises:
measuring circulating CD4+ T-cell levels in the subject to determine when the subject has entered the maladaptive remodeling phase;
detecting one or more biomarkers in the subject to determine when the subject has entered the maladaptive remodeling phase, preferably wherein the one or more biomarkers are chosen from relative levels of myosin heavy chain isoforms, GLUT-1 expression level, alpha-actin expression level, natriuretic peptide expression level, galectin expression level, caveolin expression level, neuronal nitric oxide synthase expression level, angiotensin-converting enzyme expression level, GLUT-4 expression level, SERCA2a expression level, and a shift from glucose to fatty acid oxidation; or
echocardiography, ventriculography, nuclear magnetic resonance, or any combination thereof.

6. The carborane for use of any of claims 1-5, wherein the carborane is administered in an effective amount to:
inhibit activation and proliferation of CD4+ T-cells in the subject;
reduce circulating CD4+ T-cell levels in the subject, preferably without significantly affecting circulating levels of neutrophils, monocytes, or B-cells;
decrease left ventricular (LV) remodeling in the subject;
inhibit an increase in left ventricular end-diastolic volume in the subject;
inhibit an increase in left ventricular end-systolic volume in the subject;
or a combination thereof.

7. The carborane for use of any of claims 1-6, wherein the carborane comprises 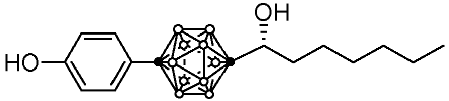 or a pharmaceutically acceptable salt thereof.

8. The carborane for use of any of claims 1-7, wherein the carborane is a selective ERβ agonist; the carborane has an EC₅₀ of 800 nM or less, such as an EC₅₀ of 6 nM or less, at estrogen receptor beta (ERβ); the carborane has an ERβ-to-ERα agonist ratio of 8 or more, such as an ERβ-to-ERα agonist ratio of 400 or more; or a combination thereof.

## Patentansprüche

1. Carboran zur Verwendung in einem Verfahren zur Behandlung oder Vorbeugung von chronischer Herzinsuffizienz bei einem Subjekt nach einem Myokardinfarkt, wobei das Verfahren die Verabreichung des Carborans an das Subjekt während einer maladaptiven Remodellierungsphase nach dem Myokardinfarkt umfasst und wobei das Carboran aus der Gruppe ausgewählt ist, bestehend aus: 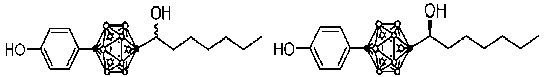 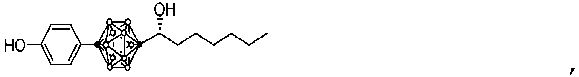 und pharmazeutisch verträgliche Salze davon.

2. Carboran zur Verwendung nach Anspruch 1, wobei das Verfahren die Behandlung oder Vorbeugung einer chronischen Herzinsuffizienz bei einem Subjekt nach einem Myokardinfarkt umfasst und das Carboran dem Subjekt nicht während einer Heilungsphase oder einer Reparaturphase, die der maladaptiven Remodelingphase vorsteht, verabreicht wird.

3. Carboran zur Verwendung nach einem der Ansprüche 1 bis 2, wobei das Verfahren die Behandlung oder Vorbeugung einer chronischen Herzinsuffizienz bei einem Subjekt nach einem Myokardinfarkt umfasst und die Verabreichung des Carborans mindestens 10 Tage nach dem Myokardinfarkt, wie beispielsweise mindestens 14 Tage nach dem Myokardinfarkt, beginnt, mindestens 21 Tage nach dem Myokardinfarkt, mindestens 28 Tage nach dem Myokardinfarkt, mindestens 35 Tage nach dem Myokardinfarkt, mindestens 42 Tage nach dem Myokardinfarkt, mindestens 49 Tage nach dem Myokardinfarkt oder mindestens 56 Tage nach dem Myokardinfarkt.

4. Carboran zur Verwendung nach einem der Ansprüche 1 bis 3, wobei das Verfahren die Behandlung oder Vorbeugung einer chronischen Herzinsuffizienz bei einem Subjekt nach einem Myokardinfarkt umfasst und das Verfahren ferner das Beurteilen des Subjekts umfasst, um zu bestimmen, ob das Subjekt in die maladaptive Remodellierungsphase eingetreten ist.

5. Carboran zur Verwendung nach Anspruch 4, wobei die Beurteilung des Subjekts, um zu bestimmen, ob das Subjekt in die maladaptive Remodellierungsphase eingetreten ist, Folgendes umfasst:
Messen der zirkulierenden CD4+-T-Zellen in dem Subjekt, um zu bestimmen, wann das Subjekt in die maladaptive Remodellierungsphase eingetreten ist;
Nachweisen eines oder mehrerer Biomarker in dem Subjekt, um zu bestimmen, wann das Subjekt in die maladaptive Remodellierungsphase eingetreten ist, wobei der eine oder die mehreren Biomarker vorzugsweise aus den relativen Spiegeln der Isoformen der schweren Myosinkette, dem GLUT-1-Expressionsniveau, dem Alpha-Actin-Expressionsniveau ausgewählt sind, natriuretischem Peptid-Expressionsniveau, Galectin-Expressionsniveau, Caveolin-Expressionsniveau, neuronalem Stickoxid-Synthase-Expressionsniveau, Angiotensin-umwandelndem Enzym-Expressionsniveau, GLUT-4-Expressionsniveau, SERCA2a-Expressionsniveau und einer Verschiebung von Glucose- zu Fettsäureoxidation ausgewählt sind; oder
Echokardiographie, Ventrikulographie, Kernspintomographie oder einer Kombination davon.

6. Carboran zur Verwendung nach einem der Ansprüche 1 bis 5, wobei das Karboran in einer wirksamen Menge verabreicht wird zum:
Hemmen der Aktivierung und Proliferation von CD4+-T-Zellen in dem Subjekt;
Reduzieren des zirkulierenden CD4+-T-Zellen-Spiegels im Subjekt, vorzugsweise ohne signifikante Beeinträchtigung des zirkulierenden Spiegels von Neutrophilen, Monozyten oder B-Zellen;
Verringern des linksventrikulären (LV) Remodelings im Subjekt;
Hemmen eines Anstiegs des enddiastolischen Volumens der linken Herzkammer bei dem Subjekt;
Hemmen eines Anstiegs des linksventrikulären endsystolischen Volumens bei dem Subjekt; oder eine Kombination davon.

7. Carboran zur Verwendung nach einem der Ansprüche 1 bis 6, wobei das Carboran Folgendes umfasst 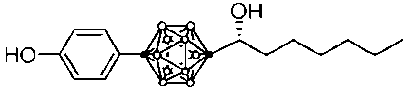 oder ein pharmazeutisch zulässiges Salz davon.

8. Carboran zur Verwendung nach einem der Ansprüche 1 bis 7, wobei das Carboran ein selektiver ERβ-Agonist ist; das Carboran eine EC₅₀ von 800 nM oder weniger, wie eine EC₅₀ von 6 nM oder weniger, am Östrogenrezeptor beta (ERβ) aufweist; das Carboran ein ERβ-zu-ERα-Agonisten-Verhältnis von 8 oder mehr, wie ein ERβ-zu-ERα-Agonisten-Verhältnis von 400 oder mehr, aufweist; oder eine Kombination davon.

## Revendications

1. Carborane pour une utilisation dans un procédé de traitement ou de prévention de l'insuffisance cardiaque chronique chez un sujet à la suite d'un infarctus du myocarde, le procédé comprenant l'administration au sujet du carborane pendant une phase de remodelage inadapté à la suite de l'infarctus du myocarde, et dans lequel le carborane est choisi dans le groupe constitué de : 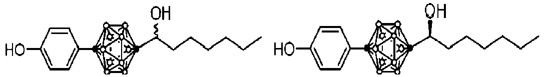 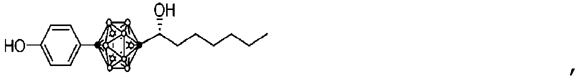 et leurs sels pharmaceutiquement acceptables.

2. Carborane pour une utilisation selon la revendication 1, dans lequel le procédé comprend le traitement ou la prévention de l'insuffisance cardiaque chronique chez un sujet à la suite d'un infarctus du myocarde et le carborane n'est pas administré au sujet pendant une phase de guérison ou une phase de réparation précédant la phase de remodelage inadapté.

3. Carborane pour une utilisation selon l'une quelconque des revendications 1 à 2, dans lequel le procédé comprend le traitement ou la prévention de l'insuffisance cardiaque chronique chez un sujet à la suite d'un infarctus du myocarde et l'administration du carborane commence au moins 10 jours suivant l'infarctus du myocarde, par exemple au moins 14 jours suivant l'infarctus du myocarde, au moins 21 jours suivant l'infarctus du myocarde, au moins 28 jours suivant l'infarctus du myocarde, au moins 35 jours suivant l'infarctus du myocarde, au moins 42 jours suivant l'infarctus du myocarde, au moins 49 jours suivant l'infarctus du myocarde ou au moins 56 jours suivant l'infarctus du myocarde.

4. Carborane pour une utilisation selon l'une quelconque des revendications 1 à 3, dans lequel le procédé comprend le traitement ou la prévention de l'insuffisance cardiaque chronique chez un sujet à la suite d'un infarctus du myocarde et le procédé comprend en outre l'évaluation du sujet pour déterminer si le sujet est entré dans la phase de remodelage inadapté.

5. Carborane pour une utilisation selon la revendication 4, dans lequel l'évaluation du sujet pour déterminer si le sujet est entré dans la phase de remodelage inadapté comprend :
la mesure de niveaux de lymphocytes T CD4+ circulants chez le sujet pour déterminer quand le sujet est entré dans la phase de remodelage inadapté ;
la détection d'un ou de plusieurs biomarqueurs chez le sujet pour déterminer quand le sujet est entré dans la phase de remodelage inadapté, de préférence dans lequel les un ou plusieurs biomarqueurs sont choisis parmi les niveaux relatifs des isoformes de la chaîne lourde de la myosine, le niveau d'expression de GLUT-1, le niveau d'expression de l'alpha-actine, le niveau d'expression du peptide natriurétique, le niveau d'expression de la galectine, le niveau d'expression de la cavéoline, le niveau d'expression de l'oxyde nitrique synthase neuronale, le niveau d'expression de l'enzyme de conversion de l'angiotensine, le niveau d'expression de GLUT-4, le niveau d'expression de SERCA2a et un passage de l'oxydation du glucose à l'oxydation des acides gras ;
ou
l'échocardiographie, la ventriculographie, la résonance magnétique nucléaire ou toute combinaison de celles-ci.

6. Carborane pour une utilisation selon l'une quelconque des revendications 1 à 5, dans lequel le carborane est administré en une quantité efficace pour :
inhiber l'activation et la prolifération des lymphocytes T CD4+ chez le sujet ;
réduire les niveaux de lymphocytes T CD4+ circulants chez le sujet, de préférence sans affecter de manière significative les niveaux de neutrophiles, de monocytes ou de lymphocytes B circulants ;
diminuer le remodelage ventriculaire gauche (VG) chez le sujet ;
inhiber une augmentation du volume télédiastolique ventriculaire gauche chez le sujet ;
inhiber une augmentation du volume télésystolique ventriculaire gauche chez le sujet ; ou une combinaison de ceux-ci.

7. Carborane pour une utilisation selon l'une quelconque des revendications 1 à 6, dans lequel le carborane comprend 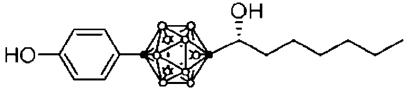 ou sel pharmaceutiquement acceptable de celui-ci.

8. Carborane pour une utilisation selon l'une quelconque des revendications 1 à 7, dans lequel le carborane est un agoniste sélectif de ERβ ; le carborane présente une EC₅₀ de 800 nM ou moins, telle qu'une EC₅₀ de 6 nM ou moins, au niveau du récepteur bêta des œstrogènes (ERβ) ; le carborane présente un rapport agoniste ERβ-ERα de 8 ou plus, tel qu'un rapport agoniste ERβ-ERα de 400 ou plus ; ou une combinaison de ceux-ci.
